# NEUE EUROPÄISCHE PATENTSCHRIFT

(11) **EP 3 177 659 B2**
(45) Veröffentlichungstag und Bekanntmachung des Hinweises auf die Entscheidung über den Einspruch: **19.02.2025**
(45) Hinweis auf die Patenterteilung: 09.12.2020
(21) Anmeldenummer: 15739623.5
(22) Anmeldetag: 23.07.2015
(51) Int. Cl.: C08G 18/20, C08G 18/48, C08G 18/76, C08G 18/16

(54) **STICKSTOFFHALTIGE VERBINDUNGEN, GEEIGNET ZUR VERWENDUNG BEI DER HERSTELLUNG VON POLYURETHANEN**
NITROGEN-CONTAINING COMPOUNDS SUITABLE FOR USE IN THE PRODUCTION OF POLYURETHANES
COMPOSÉS AZOTÉS ADAPTÉS À UNE UTILISATION DANS LA FABRICATION DE POLYURÉTHANES

(30) Priorität: 05.08.2014 DE 102014215382
(43) Veröffentlichungstag der Anmeldung: 14.06.2017
(73) Patentinhaber: Evonik Operations GmbH, 45128 Essen (DE)
(72) Erfinder: GÜNTHER, Thomas, 41464 Neuss (DE); FIEDEL, Michael, 45131 Essen (DE); FIEDEL, Olga, 45131 Essen (DE); GLOS, Martin, 46325 Borken (DE); HUBEL, Roland, 45136 Essen (DE)
(74) Vertreter: Evonik Patent Association
(86) Internationale Anmeldenummer: PCT/EP2015/066827
(87) Internationale Veröffentlichungsnummer: WO 2016/020199

(56) Entgegenhaltungen:
- EP-A1- 0 054 219
- EP-A1- 0 495 249
- WO-A1-2013/019462
- WO-A1-2015/200408
- CN-A- 102 503 909
- GB-B- 896 437
- US-A- 4 450 246
- Jeffol Brochure
- WONG WING-LEUNG ET AL: "A green catalysis of CO2fixation to aliphatic cyclic carbonates by a new ionic liquid system", APPLIED CATALYSIS A: GENERAL, ELSEVIER SCIENCE, AMSTERDAM, NL, vol. 472, 28 December 2013 (2013-12-28), pages 160 - 166, XP028667441, ISSN: 0926-860X, DOI: 10.1016/J.APCATA.2013.12.027
- WONG WING-LEUNG ET AL: "Controlling the selectivity of the manganese/bicarbonate/hydrogen peroxide catalytic system by a biphasic pyrrolidinium ionic liquid/n-heptane medium", APPLIED CATALYSIS A: GENERAL, ELSEVIER SCIENCE, AMSTERDAM, NL, vol. 453, 4 January 2013 (2013-01-04), pages 244 - 249, XP028976047, ISSN: 0926-860X, DOI: 10.1016/J.APCATA.2012.12.026

## Beschreibung

Die vorliegende Erfindung liegt auf dem Feld der stickstoffhaltigen Verbindungen, insbesondere der Amine, und der Polyisocyanat-Polyadditionsprodukte, insbesondere der Polyurethane. Sie betrifft insbesondere die Verwendung von stickstoffhaltigen Verbindungen der Formel (VI), 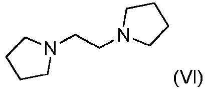 entsprechend quaternisierter und/oder protonierter Verbindungen, zur Herstellung von Polyurethanen, insbesondere von Polyurethanschaumstoffen, Zusammensetzungen, die diese Verbindungen enthalten sowie Polyurethansysteme, die unter Verwendung der Verbindungen erhalten wurden.

Die Verwendung von tertiären Aminen bei der Herstellung von Polyurethanen ist bekannt. Dabei wird eine Vielzahl von strukturell unterschiedlichen Aminen als Katalysatoren eingesetzt.

Als Polyurethane werden hier alle Reaktionsprodukte ausgehend von Isocyanaten, insbesondere von Polyisocyanaten, und entsprechend isocyanat-reaktiven Molekülen verstanden. Dies umfasst unter anderem auch Polyisocyanurate, Polyharnstoffe sowie Allophanat-, Biuret-, Uretdion-, Uretonimin- oder Carbodiimid-enthaltende Isocyanat- oder Polyisocyanat-Reaktionsprodukte. Bevorzugt ist die Verwendung der tertiären Amine bei der Herstellung von Polyisocyanat-Polyadditionsprodukten.

Polyurethansysteme sind z. B. Polyurethanbeschichtungen, Polyurethanadhäsive, Polyurethandichtmittel, Polyurethanelastomere oder Polyurethanschaumstoffe, auch bezeichnet als Polyurethanschäume oder PU-Schäume.

Besonders bei der Herstellung von Polyurethanschaumstoffen spielen tertiäre Amine eine wichtige Rolle, da hier die sogenannte Treibreaktion - Wasser reagiert mit Isocyanat unter Bildung von Kohlendioxid als Treibgas - und die Gelreaktion - Polyole reagieren mit Isocyanaten zu Urethanen, was zu einer Steigerung der Molmasse und entsprechender Vergelung führt - genau aufeinander abgestimmt sein müssen, damit ein qualitativ hochwertiger Schaum entstehen kann.

Polyurethanschäume sind zelluläre und/oder mikrozelluläre Polyurethanmaterialen und können grob in geschlossenzellige oder teilweise geschlossenzellige Polyurethanhartschaumstoffe und offenzellige oder teilweise offenzellige Polyurethanweichschaumstoffe unterteilt werden. Polyurethanhartschaumstoffe werden vorwiegend als Dämmmaterialien zum Beispiel in Kühlschranksystemen oder bei der Wärmeisolation von Gebäuden eingesetzt. Polyurethanweichschaumstoffe werden in einer Vielzahl von technischen Anwendungen in Industrie und privatem Bereich eingesetzt, beispielsweise zur Geräuschdämmung, zur Herstellung von Matratzen oder zur Polsterung von Möbeln. Einen besonders wichtigen Markt für verschiedene Typen von PU-Schäumen, wie konventionelle Weichschaumstoffe auf Ether- oder Esterpolyolbasis, Kaltweichschaumstoffe, im Folgenden auch als Kaltschäume (häufig auch als "High Resilience" (HR) Schäume) bezeichnet, und Hartschäume, sowie Schäume deren Eigenschaften zwischen diesen Klassifizierungen liegen, stellt die Automobilindustrie dar. Es können hier zum Beispiel Hartschäume als Dachhimmel, Esterschäume zur Innenverkleidung der Türen sowie für ausgestanzte Sonnenblenden, Kalt- und Weichschäume für Sitzsysteme und Matratzen verwendet werden.

Mit Blick auf Weichschaumstoffe kann auch zwischen Kaltweichschaumstoffen und Heißweichschaumstoffen unterschieden werden, wie z.B. in EP 2042534 A1 beschrieben, worauf hier im vollen Umfang Bezug genommen wird.

Es besteht nach wie vor der Bedarf an weiteren alternativen Katalysatoren, vorzugsweise von stickstoffhaltigen Katalysatoren, insbesondere von alternativen Aminen, die zur Herstellung von Polyurethanen und Polyurethanschaumstoffen geeignet sind, vorzugsweise geeignet zur Herstellung von geruchsarmen, alterungsbeständigen Polyurethansystemen mit geringen Amin- oder anderen Emissionen, wie zum Beispiel Formaldehyd und/oder Dimethylformamid (DMF).

WO 2015/200408 A1 beschreibt Katalysatoren auf Pyrrolidinbasis zur Verwendung in einer Polyurethanformulierung. EP 0 495 249 A1 offenbart die Verwendung von N-(3-Aminopropyl)-Pyrrolidin oder N-(3-(Methylamino)-propyl)-pyrrolidin als Katalysatoren bei der Herstellung von Polyurethanen. EP 0 054 219 A1 offenbart einen Katalysator, enthaltend eine Morpholin- und eine Pyrrolidingruppe, sowie Zusammensetzungen, die einen trifunktionellen Polyether und einen solchen Katalysator enthalten. Ebenfalls werden in EP 0 054 219 A1 Schaumstoffe erwähnt. In CN 102 503 909 A werden Katalysatoren für die PU-Schaumstoff-Herstellung beschrieben, welche zwei Pyrrolidingruppen aufweisen, die über eine Alkylharnstoffgruppe miteinander verbunden sind. WONG WING-LEUNG ET AL, "A green catalysis of CO2fixation to aliphatic cyclic carbonates by a new ionic liquid system", APPLIED CATALYSIS A: GENERAL, ELSEVIER SCIENCE, AMSTERDAM, NL, (20131228), vol. 472, doi:10.1016/J.APCATA.2013.12.027, ISSN 0926-860X, pages 160 - 166, XP028667441, sowie WONG WING-LEUNG ET AL, "Controlling the selectivity of the manganese/bicarbonate/hydrogen peroxide catalytic system by a biphasic pyrrolidinium ionic liquid/n-heptane medium", APPLIED CATALYSIS A: GENERAL, ELSEVIER SCIENCE, AMSTERDAM, NL, (20130104), vol. 453, doi:10.1016/J.APCATA.2012.12.026, ISSN 0926-860X, pages 244 - 249, XP028976047
sowie WO 2013/019462 A1 offenbaren Verbindungen auf Pyrrolidinbasis im allgemeinen technischen Feld der Katalyse, allerdings ohne einen Bezug zur Katalyse von Polyurethanreaktionen.

Konkrete Aufgabe der vorliegenden Erfindung war deshalb die Bereitstellung eines alternativen Katalysators zur Herstellung von Polyisocyanat-Reaktionsprodukten, vorzugsweise Polyurethanen, insbesondere von Polyurethanschaumstoffen, die vorzugsweise geruchsarm, alterungsbeständig und/oder frei von Emissionen oder allenfalls mit geringen Amin- oder anderen Emissionen, wie zum Beispiel Formaldehyd und/oder Dimethylformamid (DMF), behaftet sind.

Überraschenderweise wurde gefunden, dass Verbindungen der Formel (VI), die entsprechenden quaternisierten und/oder protonierten Verbindungen diese Aufgabe lösen, d.h. sowohl der Einsatz der Verbindungen der Formel (VI), als auch der Einsatz der entsprechenden protonierten Verbindungen als auch der Einsatz der entsprechenden quaternisierten Verbindungen als auch der Einsatz von entsprechenden Mischungen löst jeweils die genannte Aufgabe.

Gegenstand der vorliegenden Erfindung ist somit die Verwendung zumindest einer stickstoffhaltigen Verbindung, einer entsprechenden quaternisierten und/oder protonierten Verbindung bei der Herstellung von Polyisocyanat-Polyadditionsprodukten, vorzugsweise von Polyurethanen, insbesondere von Polyurethanschaumstoffen, wobei diese stickstoffhaltige Verbindung der Formel (VI) genügt,

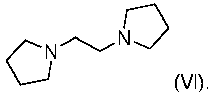

Der Ausdruck "Verwendung zumindest einer stickstoffhaltigen Verbindung, einer entsprechenden quaternisierten und/oder protonierten Verbindung" umfasst im Sinne dieser Erfindung hier und im weiteren den Einsatz der betreffenden stickstoffhaltigen Verbindung als auch den Einsatz der entsprechenden protonierten Verbindungen als auch den Einsatz der entsprechenden quaternisierten Verbindungen als auch den Einsatz von entsprechenden Mischungen.

Die erfindungsgemäß verwendeten stickstoffhaltigen Verbindungen nach der Formel (VI) sowie entsprechend quaternisierte und/oder protonierte Verbindungen, sowie Mischungen der vorgenannten, sind als Katalysatoren zur Herstellung von Polyisocyanat-Reaktionsprodukten, vorzugsweise von Polyurethanen, insbesondere von Polyurethanschaumstoffen geeignet und können sowohl die Gel- als auch die Treib-Reaktion bei der Verschäumung, sowie vorteilhafterweise weitere Isocyanat-Reaktionen, wie im Folgenden beschrieben, katalysieren.

Vorteilhafterweise ermöglicht die vorliegende Erfindung zudem eine Verminderung oder Vermeidung der katalysebedingten Emissionen bei der Herstellung von Polyurethansystemen, insbesondere von Polyurethanschäumen.

Insbesondere besteht ein zusätzlicher Vorteil der Erfindung darin, dass die erfindungsgemäß verwendeten stickstoffhaltigen Verbindungen nach der Formel (VI), entsprechend quaternisierte und/oder protonierte Verbindungen, sowie Mischungen der vorgenannten, vorteilhafterweise emissionsarm, bevorzugt frei hinsichtlich typischer unerwünschter Emissionen der resultierenden Polyurethansysteme, insbesondere Polyurethanschäume, besonders bevorzugt Polyurethanweichschaumstoffe sind, nämlich vorteilhafterweise emissionsarm hinsichtlich Emissionen stickstoffhaltiger Verbindungen, im Folgenden auch Amin-Emissionen genannt, vorteilhafterweise emissionsarm hinsichtlich Emissionen von Dimethylformamid (DMF), sowie vorteilhafterweise emissionsarm hinsichtlich Aldehydemissionen, insbesondere hinsichtlich von Formaldehydemissionen.

"Emissionsarm" hinsichtlich von Aminen umfasst im Sinne der vorliegenden Erfindung insbesondere, dass das Polyurethansystem, bevorzugt der Polyurethanschaumstoff, weiter bevorzugt der Polyurethanweichschaum, besonders bevorzugt der Polyurethanheißweichschaum, vorzugsweise zur Herstellung von Matratzen und/oder Polstermöbeln, eine Amin-Emission von ≥ 0 µg/m³ und ≤ 40 µg/m³, vorzugsweise ≤ 10 µg/m³, besonders bevorzugt ≤ 5 µg/m³, aufweist, entsprechend ermittelt nach dem Prüfkammerverfahren in Anlehnung an die DIN-Norm DIN EN ISO 16000-9:2008-04, 24 Stunden nach Prüfkammerbeladung, und/oder dass das Polyurethansystem, bevorzugt der Polyurethanschaumstoff, insbesondere der Polyurethanweichschaum, besonders bevorzugt der Polyurethankaltweichschaum, vorzugsweise zur Herstellung von Polyurethanen für die Anwendung in der Automobilindustrie, insbesondere in Automobilinnenräumen, beispielsweise als Dachhimmel, Innenverkleidung von Türen, ausgestanzte Sonnenblenden, Lenkräder und/oder Sitzsysteme, eine Amin-Emission, im Folgenden auch als VOC-Emission oder VOC-Wert nach VDA 278 bezeichnet (VOC = Volatile Organic Compounds), von ≥ 0 µg/g und ≤ 40 µg/g, vorzugsweise ≤10 µg/g, besonders bevorzugt ≤ 5 µg/g, aufweist, entsprechend des VDA 278 Analyseverfahrens in der Version vom Oktober 2011, "Thermodesorptionsanalyse organischer Emissionen zur Charakterisierung nichtmetallischer KFZ-Werkstoffe" (30 Minuten bei 90 °C), und/oder das das Polyurethansystem, insbesondere der Polyurethanweichschaum, besonders bevorzugt der Polyurethankaltweichschaum, vorzugsweise zur Herstellung von Polyurethanen für die Anwendung in der Automobilindustrie, insbesondere in Automobilinnenräumen, beispielsweise als Dachhimmel, Innenverkleidung von Türen, ausgestanzte Sonnenblenden, Lenkräder und/oder Sitzsysteme, eine Amin-Emission, im Folgenden auch Fog-Emission oder Fog-Wert nach VDA 278 (Fog: schwer flüchtige Substanzen, die bei Raumtemperatur leicht kondensieren und zum Foggingbeschlag an der Windschutzscheibe beitragen) genannt, von ≥ 0 µg/g und ≤ 40 µg/g, vorzugsweise ≤ 10 µg/g, besonders bevorzugt ≤ 5 µg/g, aufweist, entsprechend des VDA 278 Analyseverfaherens in der Version vom Oktober 2011 (60 Minuten bei 120 °C). VDA ist der Verband der Automobilindustrie (www.vda.de). Je nach Anwendungsgebiet der Polyurethansysteme, insbesondere der Polyurethanschaumstoffe, beispielsweise bei der Anwendung in der Automobil-Industrie, kann es je nach Fahrzeughersteller-Spezifikation Grenzen für die Gesamtemissionen flüchtiger organischer Verbindungen (VOC_{ges}, und/oder Fog_{ges}) geben, beispielsweise VOC_{ges} ≤ 100 µg/g und/oder Fog_{ges} ≤ 250 µg/g. Umso wichtiger ist es, dass der Beitrag der Amine zur Gesamtemission (VOC_{Amin} und/oder Fog_{Amin}) möglichst klein ist. Die im Sinne der vorliegenden Erfindung gewählten Bestimmungsmethoden in Anlehnung an die DIN-Norm DIN EN ISO 16000-9:2008-04 und die VDA 278 sind im Beispielteil detailliert erläutert.

"Emissionsarm" hinsichtlich Emissionen von Dimethylformamid (DMF) bedeutet im Sinne der vorliegenden Erfindung insbesondere, dass die erfindungsgemäßen stickstoffhaltigen Verbindungen nach der Formel (VI) und/oder entsprechende Polyurethansysteme, bevorzugt Polyurethanschaumstoffe, insbesondere Polyurethanweichschaumstoffe, besonders bevorzugt Polyurethanheißweichschaumstoffe, hergestellt unter Verwendung der vorgenannten Verbindungen, eine DMF-Emission von ≥ 0 ppm und ≤ 5 ppm, vorzugsweise ≤ 1 ppm, besonders bevorzugt ≤ 0,1 ppm, aufweist. Vorteilhafterweise ermöglicht die vorliegende Erfindung somit insbesondere die Bereitstellung von Polyurethanweichschäumen, und zwar ganz besonders Polyurethanheißweichschäumen, der hinsichtlich Emissionen von Dimethylformamid besonders emissionsarm sind. Die "DMF-Emission" ist im Sinne der vorliegenden Erfindung keine Untermenge der "Amin-Emission".

"Emissionsarm" hinsichtlich Emissionen von Aldehyden, insbesondere von Formaldehyd, bedeutet im Sinne der vorliegenden Erfindung insbesondere, dass das Polyurethansystem, bevorzugt der Polyurethanschaumstoff, insbesondere der Polyurethanweichschaum, die von den Schaumherstellern und der Möbelindustrie in Europa und der USA im Rahmen des freiwillig auferlegten Programms "CertiPUR" festgelegten Grenzen für Aldehydemissionen, insbesondere für Formaldehydemissionen erfüllt und/oder dass der Austausch der konventionellen Katalysatoren, insbesondere von Aminen, besonders von tertiären Aminen, enthaltend eine oder mehrere N-Methyl- oder N,N-Dimethyl-Gruppen, nach dem Stand der Technik durch erfindungsgemäß einzusetzende stickstoffhaltige Verbindungen in der Formulierung eines entsprechenden Polyurethansystems zu einer Verbesserung der Aldehydinsbesondere Formaldehyd-bedingten Emissionen führt. Die Grenze für Formaldehydemissionen liegt laut "CertiPUR" zum Beispiel für Matratzen bei 0,1 mg/m³ gemessen gemäß ASTM Methode D5116-97 "Small Chamber Test" bei Konditionierung über 16 Stunden. Dem Fachmann sind unterschiedliche analytische Methoden zur Bestimmung von Aldehydemissionen bekannt. Beispielhaft seien hier VDA 275, VDA 277 oder auch VDA 278 genannt, ebenso sei auf diverse Kammertestmethoden hingewiesen. VDA ist der Verband der Automobilindustrie (www.vda.de). "VDA 275", nach der Version vom Juli 1994, liefert ein Messverfahren zur Bestimmung der Aldehyd-, insbesondere der Formaldehydabgabe nach der modifizierten Flaschen-Methode, wobei als Derivatisierungsreagenz von Aldehyden neben dem üblicherweise verwendeten Acetylaceton (über photometrische Detektion) auch 2,4-Dinitrophenylhydrazin (2,4-DNP) (Detektion über HPLC nach externer Kalibrierung) eingesetzt werden kann um neben Formaldehyd auch Acetaldehyd und Propionaldehyd besser bestimmen zu können. Im Rahmen dieser Erfindung wird auf beide Verfahrensauslegungen dieser VDA 275 als bevorzugte Verfahren zur Bestimmung von Aldehyd-, insbesondere Formaldehydemissionen, Bezug genommen.

Vorteilhafterweise ermöglicht die vorliegende Erfindung somit die Bereitstellung von Polyurethansystemen, vorzugsweise von Polyurethanschaumstoffen, insbesondere von Polyurethanweichschaumstoffen, die hinsichtlich Emissionen von stickstoffhaltigen Verbindungen, im Folgenden auch Amin-Emissionen genannt, auch bei unterschiedlichen Anforderungen besonders emissionsarm, bevorzugt frei von solchen Emissionen ist.

Vorteilhafterweise ermöglicht die vorliegende Erfindung somit die Bereitstellung von Polyurethansystemen, vorzugsweise von Polyurethanschaumstoffen, insbesondere von Polyurethanweichschaumstoffen, hergestellt unter Verwendung der vorgenannten stickstoffhaltigen Verbindungen, die hinsichtlich Emissionen von Dimethylformamid (DMF) auch bei unterschiedlichen Anforderungen besonders emissionsarm, bevorzugt frei von solchen Emissionen sind.

Vorteilhafterweise trägt die vorliegende Erfindung bei zur Bereitstellung Polyurethansystemen, vorzugsweise Polyurethanschaumstoffen, insbesondere von Polyurethanweichschaumstoffen, hergestellt unter Verwendung der vorgenannten stickstoffhaltigen Verbindungen, die hinsichtlich Emissionen von Aldehyden, insbesondere von Formaldehyd, auch bei unterschiedlichen Anforderungen, emissionsärmer sind als entsprechende stickstoffhaltige Katalysatoren oder entsprechenden Polyurethansysteme, bei denen an Stelle der erfindungsgemäßen stickstoffhaltigen Verbindungen konventionelle Katalysatoren, insbesondere tertiären Amine, enthaltend eine oder mehrere N-Methyl- oder N,N-Dimethyl-Gruppen, nach dem Stand der Technik, eingesetzt werden. Denn handelsübliche Amine oder PU-Systeme, enthaltend handelsübliche Amine, können ansonsten zum Beispiel aufgrund ihrer industriellen Herstellung Formaldehyd als Verunreinigung enthalten, beispielsweise in dem bei der Amin-Herstellung Formaldehyd oder Methanol als Alkylierungsmittel verwendet wurden.

Vorteilhafterweise trägt die vorliegende Erfindung außerdem zur Bereitstellung von geruchsarmen Polyurethansystemen, vorzugsweise Polyurethanschaumstoffen, insbesondere von Polyurethanweichschaumstoffen bei. Geruchsarm bedeutet hier, dass das resultierende Polyurethansystem einen möglichst geringen Produktgeruch aufweist, insbesondere bei Verwendung der erfindungsgemäßen stickstoffhaltigen Verbindungen als alternative Katalysatoren zu Katalysatoren nach dem Stand der Technik, was insbesondere durch olfaktorische Überprüfung durch ein Panel von geruchlich geschulten Personen überprüfbar ist.

Vorteilhafterweise trägt die vorliegende Erfindung außerdem zur Verbesserung des Alterungsverhaltens, insbesondere zur Hitzebeständigkeit und/oder Alterungsbeständigkeit bei Temperierung (Wärmealterung), von Polyurethansystemen, vorzugsweise Polyurethanschaumstoffen, insbesondere von Polyurethanweichschaumstoffen bei. Solche Alterungsphänomene sind oft eng mit der Wahl des Katalysatorsystems zur Herstellung der Polyurethansysteme verknüpft und führen in der Regel zu einer Materialermüdung. Mit den erfindungsgemäßen stickstoffhaltigen Verbindungen kann die Hitzebständigkeit und/oder Haltbarkeit der entsprechenden Polyurethansysteme gegenüber Polyurethansystemen, die mit konventionellen Katalysatoren nach dem Stand der Technik hergestellt wurden, hier vorteilhafterweise verbessert werden. Vorteilhafterweise kann dieser Effekt insbesondere bei Polyurethanschaumstoffen, bevorzugt Weichblockschaumstoffen, insbesondere im Sinne einer trockenen Wärmealterung nach der DIN-Norm DIN EN ISO 2440/A1:2009-01 beobachtet werden, insbesondere bei einer Temperatur von 70, 100, 120, 125 und/oder 140 °C und einer Alterungsdauer von 2, 4, 16, 22, 24, 48, 72 und/oder 168 Stunden bevorzugt bei 2, 24 und/oder 168 Stunden, wenn bei der Verschäumung erfindungsgemäße stickstoffhaltige Verbindungen nach Formel (VI) als Alternativen zu strukturverwandten Katalysatoren nach dem Stand der Technik eingesetzt werden.

Vorteilhafterweise ermöglicht die vorliegende Erfindung außerdem die Bereitstellung von vorzugsweise verfärbungsminimierten Polyurethansystemen, insbesondere von Polyurethanschaumstoffen, vorzugsweise von Polyurethanen für die Anwendung in der Automobilindustrie, insbesondere in Automobilinnenräumen, beispielsweise als Dachhimmel, Innenverkleidungen von Türen, ausgestanzte Sonnenblenden, Lenkräder und/oder Sitzsysteme, wobei die unter Verwendung von erfindungsgemäßen stickstoffhaltigen Katalysatoren bereitgestellten Polyurethansysteme insbesondere zu geringeren Verfärbungen von Kunststoffen, insbesondere Kunststoffbezügen, in Automobilinnenräumen führen als solche Polyurethansysteme, die unter Einsatz konventioneller Katalysatoren nach dem Stand der Technik, insbesondere von nicht erfindungsgemäßen Aminen, hergestellt werden können. Dies kann insbesondere an Hand eines PVC-Verfärbungs-Tests gemäß der Volkswagen-Prüfvorschrift VW PV 3937, Aminemissionen nach der Indikatormethode, gezeigt werden.

Vorteilhafterweise ermöglicht die vorliegende Erfindung ein breiteres Verarbeitungsspiel bei der Herstellung von Polyurethansystemen, insbesondere von halbharten Polyurethanschäumen (offenzelligen Hartschäumen, beispielsweise zur Anwendung als Dachhimmel in Automobilinnenräumen). Das heißt, dass vorteilhafterweise eine größere Variationsbreite der Einsatzkonzentration der erfindungsgemäßen stickstoffhaltigen Verbindungen ohne negative Beeinflussung der gewünschten Materialeigenschaften, beispielsweise der Offenzelligkeit des Schaums oder der Raumgewichtsverteilung über den Schaumblock, möglich ist gegenüber vergleichbaren oder für solche Anwendungen üblicherweise eingesetzten Amin-Katalysatoren nach dem Stand der Technik. Dies bedeutet eine enorme Arbeitserleichterung für den Anwender.

Zur möglichen Quaternisierung der Verbindungen der Formel (VI) können alle als Quaternisierungsreagenz bekannten Reagenzien eingesetzt werden. Vorzugsweise werden als Quaternisierungsmittel Alkylierungsmittel, wie z. B. Dimethylsulfat, Methylchlorid oder Benzylchlorid, bevorzugt Methylierungsmittel wie insbesondere Dimethylsulfat verwendet. Ebenso kann mit Alkylenoxiden wie zum Beispiel Ethylenoxid, Propylenoxid oder Butylenoxid, vorzugsweise mit anschließender Neutralisation mit anorganischen oder organischen Säuren, quaternisiert werden.

Die Verbindungen der Formel (VI) können, sofern quaternisiert, einfach oder mehrfach quaternisiert sein. Vorzugsweise sind die Verbindungen der Formel (VI) nur einfach quaternisiert. Bei einfacher Quaternisierung sind die Verbindungen der Formel (VI) vorzugsweise an einem Stickstoffatom quaternisiert, welches Teil eines Ringes, bevorzugt eines Pyrrolidin-Ringes, ist.

Die Verbindungen der Formel (VI) können durch Umsetzung mit organischen oder anorganischen Säuren in die entsprechenden protonierten Verbindungen überführt werden. Diese protonierten Verbindungen können z.B. bevorzugt sein, wenn z.B. eine verlangsamte Polyurethan-Reaktion erzielt werden soll oder wenn das Reaktionsgemisch bei der Anwendung ein verbessertes Fließverhalten haben soll.

Als organische Säuren können zum Beispiel alle nachfolgend genannten organischen Säuren, beispielsweise Carbonsäuren mit 1 bis 36 Kohlenstoffatomen (aromatisch oder aliphatisch, linear oder verzweigt), beispielsweise Ameisensäure, Milchsäure, 2-Ethylhexansäure, Salicylsäure und Neodecansäure, oder auch polymere Säuren wie z.B. Polyacryl- oder Polymethacrylsäuren eingesetzt werden. Als anorganische Säuren können z.B. Phosphorbasierende Säuren, Schwefel-basierende Säuren oder Bor-basierende Säuren eingesetzt werden.

Der Einsatz von Verbindungen der Formel (VI), die nicht quaternisiert oder protoniert sind, ist im Sinne dieser Erfindung allerdings besonders bevorzugt.

Die erfindungsgemäßen Gegenstände werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt, insbesondere in Bezug auf den Sachverhalt, in dessen Zusammenhang das Dokument zitiert wurde, vollständig zum Offenbarungsgehalt der vorliegenden Erfindung gehören. Bei Prozentangaben handelt es sich, wenn nicht anders angegeben, um Angaben in Gewichtsprozent. Werden nachfolgend Mittelwerte angegeben, so handelt es sich, wenn nicht anderes angegeben, um Gewichtsmittel. Werden nachfolgend Parameter angegeben, die durch Messung bestimmt wurden, so wurden die Messungen, wenn nicht anders angegeben, bei einer Temperatur von 25 °C und einem Druck von 101.325 Pa durchgeführt.

Unter Polyurethan (PU) wird im Rahmen der vorliegenden Erfindung insbesondere ein Produkt erhältlich durch Reaktion von Polyisocyanaten und Polyolen bzw. Verbindungen mit Isocyanat-reaktiven Gruppen verstanden. Es können hierbei neben dem Polyurethan auch weitere funktionelle Gruppen gebildet werden, wie z.B. Uretdione, Carbodiimide, Isocyanurate, Allophanate, Biurete, Harnstoffe und/oder Uretonimine. Daher werden unter PU im Sinne der vorliegenden Erfindung sowohl Polyurethan als auch Polyisocyanurat, Polyharnstoffe und Uretdion-, Carbodiimid-, Allophanat-, Biuret- und Uretonimin-Gruppen enthaltende Polyisocyanat-Reaktionsprodukte verstanden. Unter Polyurethanschaum (PU-Schaum) wird im Rahmen der vorliegenden Erfindung Schaum verstanden, der als Reaktionsprodukt basierend auf Polyisocyanaten und Polyolen bzw. Verbindungen mit Isocyanat-reaktiven Gruppen erhalten wird. Es können hierbei neben dem Namen gebenden, Polyurethan auch weitere funktionelle Gruppen gebildet werden, wie z.B. Allophanate, Biurete, Harnstoffe, Carbodiimide, Uretdione, Isocyanurate oder Uretonimine. Daher werden unter PU-Schäumen im Sinne der vorliegenden Erfindung sowohl Polyurethanschäume (PUR-Schäume) als auch Polyisocyanurat-Schäume (PIR-Schäume) verstanden. Bevorzugte Polyurethanschäume sind Polyurethanweichschaumstoffe, Polyurethanhartschaumstoffe und Polyurethanintegralschaumstoffe. Hierin besonders bevorzugt sind konventionelle Polyurethanweichschäume auf Ether- oder Esterpolyolbasis, hochelastische Polyurethankaltschaumstoffe (häufig auch als "High Resilience" (HR) Schäume bezeichnet), viskoelastische Polyurethanschäume, halbharte Polyurethanschäume und Polyurethanhartschaumstoffe, sowie Schäume deren Eigenschaften zwischen diesen Klassifizierungen liegen und in der Automobilindustrie eingesetzt werden.

Gemäß Anspruch 1 der Erfindung gelangt zumindest eine stickstoffhaltige Verbindung zur Anwendung, die der Formel (VI) genügt.

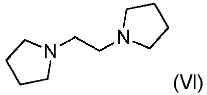

Wenn im Rahmen dieser Erfindung von zumindest einer stickstoffhaltigen Verbindung nach der Formel (VI) die Rede ist, dann umfasst das den Einsatz von einer stickstoffhaltigen Verbindung der Formel (VI), sowie den Einsatz der entsprechenden protonierten Verbindung als auch den Einsatz der entsprechenden quaternisierten Verbindung als auch den Einsatz von entsprechenden Mischungen der vorgenannten stickstoffhaltigen Verbindung der Formel (VI), entsprechender protonierter und/oder quaternisierter Verbindungen.

Die zuvor beschriebene erfindungsgemäße stickstoffhaltige Verbindung nach der Formel (VI) ist grundsätzlich zugänglich über gängige Verfahren zur Amin-Herstellung. Eine gute Übersicht zur Herstellung und Derivatisierung von Aminen, beispielsweise mit Ethylenoxid und Propylenoxid (Alkoxylierung), zur Herstellung von Harnstoffen, insbesondere auch zur Synthese von Pyrrolidin, ist in dem Artikel "Amines, Aliphatic" in Ullmann's Encylopedia of Industrial Chemistry, Wiley-VCH, Weinheim, 2012, Vol. 2, S. 647-698 (DOI: 10.1002/14356007.a02_001) und den darin enthaltenen Literaturstellen beschrieben.

Bevorzugte Synthese-Bausteine, im Sinne der vorliegenden Erfindung, sind insbesondere Polyole, vorzugsweise Diole, beispielsweise Glycole, Amine, Polyamine, insbesondere Diamine, oder Aminoalkohole, sowie Aminogruppen-tragende Alkylchloride, insbesondere solche vorgenannten Synthese-Bausteine, die bereits über eine Pyrrolidin-Funktion verfügen. Bevorzugt verwendete Polyole sind beispielsweise Monoethylenglycol (MEG), 1,2-Propylenglycol (PG), 1,4-Butandiol oder 1,6-Hexandiol. Bevorzugt verwendete Polyamine sind beispielweise lineare oder verzweigte, aliphatische Diamine mit jeweils zwei terminalen primären Amino-Gruppen wie zum Beispiel 1,4-Butandiamin oder 1,6-Hexandiamin, vorzugsweise Ethylendiamin (EDA), Trimethylendiamin, 1-(2-Aminoethyl)pyrrolidin oder 1-(3-Aminopropyl)pyrrolidin, welches beispielsweise in Anlehnung an das in WO 2012072441 beschriebene Verfahren oder wie nach Krupka und Jiri et al. in Czechoslovak Chemical Communications, 65 (11), 1805-1819; 2000 beschrieben, durch eine Cyanoethylierung und Reduktion zum Amin oder wie in SU1421738 (A1) oder von Katritzky et al. in Journal of Organic Chemistry (1994), 59, 5206-5214, beschrieben, erhalten werden kann. Die Synthese von 1-(3-Aminopropyl)pyrrolidin ist darüber hinaus im Beispielteil beschrieben. Bevorzugt verwendete Aminoalkohole können zum Beispiel durch Umsetzung von Ammoniak oder Aminen mit Epoxiden (Alkoxylierung), vorzugsweise mit Ethylenoxid (EO) und/oder Propylenoxid (PO), durch Umsetzung von Alkoholen oder Polyolen, vorzugsweise von Diolen, insbesondere von Glycolen, mit Acrylnitril (Michael-Reaktion) und anschließende Hydrogenierung, wie beispielsweise in Catalysis Today, 1998, 44, 277-283 beschrieben, und/oder durch Aminierung von Alkoholen oder Polyolen, vorzugsweise von Diolen, insbesondere von Glycolen mit Ammoniak oder Aminen nach bekannten Verfahren, wie beispielsweise von Beller et al. in Chem. Asian J. 2007, 2, 403 - 410, von Milstein et al. in Angew. Chem. 2008, 120, 8789 -8792, von Watson und Williams in Science 2010, Vol. 329, S. 635-636 oder von Börner et al. in ChemCatChem 2010, 2, 640 - 643 beschrieben, erhalten werden. Beispiele hierfür sind Monoethanolamin (MEA), 3-Amino-1-propanol, 1-(2-Hydroxyethyl)pyrrolidin und 1-(3-Hydroxypropyl)pyrrolidin. Die hier exakt beschriebenen Polyamine, Polyole und Aminoalkohole sind alle kommerziell verfügbar. Aminogruppen-tragende Alkylchloride können zum Beispiel durch Umsetzung von Bischloro-Alkyl-Verbindungen, beispielsweise 1,2-Dichloroethan, 1,4-Dichlorobutan oder 1,6-Dichlorohexan, mit einem Äquivalent eines Amins, insbesondere mit Pyrrolidin (nukleophile Substitution), erhalten werden. Erfindungsgemäße Harnstoffe und Guanidine können zum Beispiel durch Umsetzung der entsprechenden primären Amine mit Harnstoff, Methylharnstoff oder Guanidin Hydrochlorid erhalten werden. Triazine lassen sich durch Umsetzung primärer Amine mit Formaldehyd, beispielsweise beschrieben in EP 0872505 oder DE 2439278, herstellen.

Die in allen Verbindungen enthaltenen Pyrrolidin-Gruppen können hierbei entweder am Anfang oder am Ende, je nach gewünschter Syntheseroute, eingebracht werden. Hierzu kann es bevorzugt sein Pyrrolidin selbst zu verwenden, zum Beispiel bei Umsetzung von Pyrrolidin mit Alkylhalogeniden, insbesondere mit Alkylchloriden (nukleophile Substitution), wie beispielsweise von Aitken et al. in Tetrahedron, 2002, Vol. 58, 29, S. 5933-5940 bzw. von Tijskens et al. in Journal of Organic Chemistry, 1995, Vol. 60, 26, S. 8371-8374 be-schrieben, und/oder durch Umsetzung von Pyrrolidin mit Bischloroalkylethern, und/oder durch Umsetzung von Pyrrolidin mit Epoxiden (Alkoxylierung) oder Epoxid-tragenden Verbindungen, insbesondere mit Ethylenoxid (EO) und/oder Propylenoxid°(PO), wie zum Beispiel von Reppe et al. in Justus Liebigs Annalen der Chemie, 1955, Vol. 596, S. 1149 oder von Moffett et al. in Journal of Organic Chemistry, 1949, Vol. 14, S. 862-866 und in Org. Synth. Coll., 1963, Vol. IV, S. 834ff, und/oder durch Umsetzung von Pyrrolidin mit Alkoholen und/oder Polyolen, vorzugsweise Diolen, insbesondere Glycolen, beispielsweise in einer übergangsmetallkatalysierten Ausführung wie von Jenner et al. in Journal of Organometallic Chemistry, 373 (1989), 343-352 beschrieben. Des Weiteren kann die Pyrrolidin-Funktion auch durch Umsetzung von 1,4-Butandiol mit primären Aminen, wie zum Beispiel wiederum von Jenner et al. in Journal of Organometallic Chemistry, 373 (1989), 343-352 beschrieben, und/oder durch Umsetzung von 1,4-Butandiol mit Ammoniak und Hydroxyl-Gruppen tragenden organischen Verbindungen, vorzugsweise von Polyolen, insbesondere Diolen, bevorzugt von Glycolen wie Monoethylenglycol (MEG) oder Diethylenglycol (DEG) und/oder Aminoalkoholen, insbesondere enthaltend eine primäre Hydroxyl-Funktion, wie beispielsweise in DE 701825C beschrieben, eingebracht werden. Zur Illustration ist die Herstellung und anwendungstechnische Ausprüfung ausgewählter erfindungsgemäßer Verbindungen nach Formel (VI) im Beispielteil genau beschrieben.

Je nach Herstellungsprozess kann eine stickstoffhaltige Verbindung nach der Formel (VI), je nach Güte des gewählten Prozesses und Anzahl der entsprechenden Aufreinigungsschritte in technischer Qualität, im Folgenden auch als technische Produktmischungen bezeichnet, vorliegen, also beispielsweise Zwischen- und/oder Nebenprodukte als Nebenbestandteile und/oder weitere Verunreinigungen aufweisen. Auch der Einsatz technischer Produktmischungen entspricht einer bevorzugten Ausführungsform im Sinne dieser Erfindung.

Insbesondere die erfindungsgemäße Verwendung, wobei zumindest eine stickstoffhaltige Verbindung der Formel (VI) als technische Produktmischung eingesetzt wird, insbesondere enthaltend Verunreinigungen, Zwischen- und/oder Nebenprodukte als weitere Inhaltsstoffe, insbesondere umfassend Pyrrolidin, 1-(3-Aminopropyl)pyrrolidin, 1-(2-Aminoethyl)pyrrolidin, 1-(2-Hydroxyethyl)pyrrolidin, 1-(3-Hydroxypropyl)pyrrolidin, Trimethylendiamin, Ethylendiamin (EDA), 1,4-Butandiol, Monoethylenglycol (MEG), Diethylenglycol (DEG), und/oder Monoethanolamin (MEA), und zwar in einer Gesamtmenge von bis zu 95 Gew.-%, vorzugsweise ≤ 70 Gew.-%, insbesondere ≤ 30 Gew.-%, bevorzugt ≤ 10 Gew.-%, besonders bevorzugt ≤ 5 Gew.-%, stellt eine bevorzugte Ausführungsform der Erfindung dar. Eine Untergrenze kann z.B. bei ≥ 0 Gew.-% liegen, oder z.B. bei 0,1 Gew.-%.

Insbesondere die erfindungsgemäße Verwendung einer technischen Produktmischung, wobei die technische Produktmischung enthält
(a) zumindest eine stickstoffhaltige Verbindung der Formel (VI), vorteilhafterweise in einer Gesamtmenge von ≥ 5 Gew.-%, bevorzugt 20-95 Gew.-%, insbesondere 30-70 Gew.-%,
(b) optional 1-(3-Aminopropyl)pyrrolidin, vorteilhafterweise in einer Menge ≥ 5 Gew.-%, bevorzugt 20-95 Gew.-%, insbesondere 30-70 Gew.-%,
(c) optional 1-(2-Aminoethyl)pyrrolidin, vorteilhafterweise in einer Menge ≥ 5 Gew.-%, bevorzugt 20-95 Gew.-%, insbesondere 30-70 Gew.-%,
(d) optional 1-(2-Hydroxyethyl)pyrrolidin, vorteilhafterweise in einer Menge ≥ 5 Gew.-%, bevorzugt 20-95 Gew.-%, insbesondere 30-70 Gew.-%,
(e) optional 1-(3-Hydroxypropyl)pyrrolidin, vorteilhafterweise in einer Menge ≥ 5 Gew.-%, bevorzugt 20-95 Gew.-%, insbesondere 30-70 Gew.-%,
(f) optional Trimethylendiamin, vorteilhafterweise in einer Menge ≥ 5 Gew.-%, insbesondere 20-95 Gew.-%, bevorzugt 30-70 Gew.-%,
(g) optional Ethylendiamin (EDA), vorteilhafterweise in einer Menge ≤ 95 Gew.-%, insbesondere 20-90 Gew.-%, bevorzugt 30-80 Gew.-%,
(h) optional 1,4-Butandiol, vorteilhafterweise in einer Menge ≤ 95 Gew.-%, insbesondere 20-90 Gew.-%, bevorzugt 30-80 Gew.-%,
(i) optional Monoethylenglycol (MEG), vorteilhafterweise in einer Menge ≤ 95 Gew.-%, insbesondere 20-90 Gew.-%, bevorzugt 30-80 Gew.-%,
(j) optional Diethylenglycol (DEG) vorteilhafterweise in einer Menge ≤ 95 Gew.-%, insbesondere 20-90 Gew.-%, bevorzugt 30-80 Gew.-%, und/oder
(k) optional Monoethanolamin (MEA) vorteilhafterweise in einer Menge ≤ 95 Gew.-%, insbesondere 20-90 Gew.-%, bevorzugt 30-80 Gew.-%,
entspricht einer bevorzugten Ausführungsform der Erfindung.

Im Sinne der vorgenannten bevorzugten Ausführungsform sind besonders bevorzugte technische Produktmischungen im Rahmen der vorliegenden Erfindung solche Zusammensetzungen, in denen zumindest eine stickstoffhaltige Verbindung nach Formel (VI) und/oder eine entsprechende quaternisierte und/oder protonierte Verbindung verwendet wird in Kombination
mit b), mit c), mit d), mit e), mit f), mit g), mit h), mit i), mit j), mit k), mit b) und h), mit c) und h), mit d) und h), mit e) und h), mit b) und i), mit c) und i), mit d) und i), mit e) und i), mit b) und j), mit c) und j), mit d) und j), mit e) und j), mit b) und d), mit b), d) und h), mit b), d) und i) oder mit b), d) und j).

Vorzugsweise werden die zuvor beschriebenen Verbindungen der Formel (VI) und/oder entsprechende quaternisierte und/oder protonierte Verbindungen bei der erfindungsgemäßen Herstellung von Polyurethansystemen, vorzugsweise zur Herstellung von Polyurethanbeschichtungen, Polyurethanadhäsiven, Polyurethandichtmitteln, Polyurethanelastomeren oder insbesondere zur Herstellung von Polyurethanschaumstoffen als Katalysatoren verwendet. Die Verbindungen der Formel (VI) und/oder die entsprechenden quaternisierten und/oder protonierten Verbindungen können dabei ergänzend zu üblichen Katalysatoren oder als Ersatz für übliche Katalysatoren verwendet werden. Insbesondere können die erfindungsgemäßen Verbindungen als Ersatz für andere stickstoffhaltige Katalysatoren, im Folgenden auch als Amin-Katalysatoren oder Amine bezeichnet, und je nach Anwendung als teilweiser oder vollständiger Ersatz für übliche metallhaltige Katalysatoren nach dem Stand der Technik verwendet werden.

Es entspricht daher einer bevorzugten Ausführungsform der Erfindung, wenn zumindest eine stickstoffhaltige Verbindung nach Formel (VI), eine entsprechend quaternisierte und/oder protonierte Verbindung, oder Mischungen der stickstoffhaltigen Verbindung nach Formel (VI) mit entsprechenden quaternisierten und/oder protonierten Verbindungen als Katalysator bei der Herstellung von Polyurethansystemen, insbesondere Polyurethanschaumstoffen, eingesetzt wird. Insbesondere kann die genannte stickstoffhaltige Verbindung auch als technische Produktmischung eingesetzt werden. Geeignete technische Produktmischungen werden weiter unten genauer beschrieben.

Es versteht sich von selbst, dass der Fachmann zur Herstellung der unterschiedlichen Polyurethansysteme, insbesondere der unterschiedlichen Polyurethanschaumstofftypen, beispielsweise von Heiß-, Kalt-, Ester-Polyurethanweichschaumstoffen oder Polyurethanhartschaumstoffen, die hierfür jeweils notwendigen Substanzen, wie Isocyanate, Polyole, Stabilisatoren, Tenside, etc. entsprechend auswählt, um den jeweils gewünschten Polyurethan-Typ, insbesondere Polyurethanschaum-Typ, zu erhalten.

Bei der erfindungsgemäßen Herstellung von Polyurethansystemen, insbesondere von Polyurethanschaumstoffen, werden vorzugsweise zumindest eine erfindungsgemäße Verbindung der Formel (VI) und/oder eine entsprechende quaternisierte und/oder protonierte Verbindung, zumindest eine Polyolkomponente und zumindest eine Isocyanatkomponente, optional in Gegenwart von Wasser, physikalischen Treibmitteln, Flammschutzmitteln, zusätzlichen Katalysatoren und/oder weiteren Zusatzstoffen, miteinander reagiert.

Weitere Angaben zu den verwendeten Ausgangsstoffen, Katalysatoren sowie Hilfs- und Zusatzstoffen finden sich beispielsweise im Kunststoffhandbuch, Band 7, Polyurethane, Carl-Hanser-Verlag München, 1. Auflage 1966, 2. Auflage, 1983 und 3. Auflage, 1993. Die nachstehenden Verbindungen, Komponenten und Zusatzstoffe sind lediglich beispielhaft genannt und können durch andere dem Fachmann bekannte Stoffe ersetzt und/oder ergänzt werden.

Die erfindungsgemäß einzusetzenden Verbindungen der Formel (VI) und/oder eine entsprechend quaternisierte und/oder protonierte Verbindungen, in Gesamtmenge, werden hierbei vorzugsweise in einem Massenanteil von 0,01 bis 20,0 Teilen (pphp), bevorzugt 0,01 bis 5,00 Teilen und besonders bevorzugt 0,02 bis 3,00 Teilen bezogen auf 100 Teile (pphp) Polyolkomponente eingesetzt.

Es entspricht daher einer bevorzugten Ausführungsform der Erfindung, wenn bei der Herstellung des Polyurethansystems, insbesondere Polyurethanschaums, eine Zusammensetzung hergestellt wird, die zumindest eine stickstoffhaltige Verbindung nach der Formel (VI) und/oder eine entsprechende quaternisierte und/oder protonierte Verbindung, sowie weiterhin zumindest eine Polyolkomponente, zumindest eine Isocyanatkomponente sowie optional ein oder mehrere Treibmittel aufweist, und diese Zusammensetzung reagiert wird. Insbesondere kann die genannte stickstoffhaltige Verbindung auch als technische Produktmischung eingesetzt werden. Geeignete technische Produktmischungen werden weiter unten genauer beschrieben.

Als Isocyanatkomponenten werden vorzugsweise ein oder mehrere organische Polyisocyanate mit zwei oder mehr Isocyanat-Funktionen eingesetzt. Als Polyolkomponenten werden vorzugsweise ein oder mehrere Polyole mit zwei oder mehr gegenüber Isocyanat reaktiven Gruppen, eingesetzt.

Als Isocyanatkomponenten geeignete Isocyanate im Sinne dieser Erfindung sind alle Isocyanate, die mindestens zwei Isocyanat-Gruppen enthalten. Generell können alle an sich bekannten aliphatischen, cycloaliphatischen, arylaliphatischen und vorzugsweise aromatischen mehrfunktionalen Isocyanate verwendet werden. Bevorzugt werden Isocyanate in einem Bereich von 60 bis 350 mol%, besonders bevorzugt in einem Bereich von 60 bis 140 mol%, relativ zu der Summe der isocyanatverbrauchenden Komponenten eingesetzt.

Beispielhaft genannt werden können hier Alkylendiisocyanate mit 4 bis 12 Kohlenstoffatomen im Alkylenrest, wie 1,12-Dodecandiisocyanat, 2-Ethyltetramethylendiisocyanat-1,4, 2-Methylpentamethylendiisocyanat-1,5, Tetramethylendiisocyanat-1,4, und vorzugsweise Hexamethylendiisocyanat-1,6 (HMDI), cycloaliphatische Diisocyanate, wie Cyclohexan-1,3-und 1-4-diisocyanat sowie beliebige Gemische dieser Isomeren, 1-Isocyanato-3,35-trimethyl-5-isocyanatomethylcyclohexan (Isophorondiisocyanat oder kurz IPDI), 2,4- und 2,6-Hexahydrotoluylendiisocyanat sowie die entsprechenden Isomerengemische, und vorzugsweise aromatische Di- und Polyisocyanate, wie beispielsweise 2,4- und 2,6-Toluoldiisocyanat (TDI) und die entsprechenden Isomerengemische, Mischungen aus 2,4'- und 2,2'-Diphenylmethandiisocyanaten (MDI) und Polyphenylpolymethylenpolyisocyanate (Roh-MDI) und Mischungen aus Roh-MDI und Toluoldiisocyanaten (TDI). Die organischen Di- und Polyisocyanate können einzeln oder in Form ihrer Mischungen eingesetzt werden.

Es ist auch möglich, Isocyanate einzusetzen, die durch den Einbau von Urethan-, Uretdion-, Isocyanurat-, Allophanat- und anderen Gruppen modifiziert wurden, sogenannte modifizierte Isocyanate.

Besonders geeignete organische Polyisocyanate und daher besonders bevorzugt angewendet werden verschiedene Isomere des Toluoldiisocyanat (2,4- und 2,6-Toluoldiisocyanat (TDI), in reiner Form oder als Isomerengemische unterschiedlicher Zusammensetzung), 4,4'-Diphenylmethandiisocyanat (MDI), das so genannte "crude MDI" oder "polymere MDI" (enthält neben dem 4,4'- auch die 2,4'- und 2,2'-Isomeren des MDI und höherkernige Produkte) sowie das als "pure MDI" bezeichnete zweikernige Produkt aus überwiegend 2,4'- und 4,4'-Isomerengemischen bzw. deren Prepolymeren. Beispiele für besonders geeignete Isocyanate sind beispielsweise in EP 1712578, EP 1161474, WO 00/58383, US 2007/0072951, EP 1678232 und der WO 2005/085310 aufgeführt, auf die hier in vollem Umfang Bezug genommen wird.

Als Polyolkomponente geeignete Polyole im Sinne der vorliegenden Erfindung sind alle organischen Substanzen mit mehreren gegenüber Isocyanaten reaktiven Gruppen, vorzugsweise OH-Gruppen, sowie deren Zubereitungen. Bevorzugte Polyole sind alle zur Herstellung von Polyurethan-Systemen, insbesondere Polyurethan-Schaumstoffen; üblicherweise verwendeten Polyetherpolyole und/oder Polyesterpolyole und/oder hydroxylgruppenhaltigen aliphatischen Polycarbonate, insbesondere Polyetherpolycarbonatpolyole und/oder Füllkörperpolyole (Polymerpolyole) wie SAN-, PHD- und PIPA-Polyole, die sich dadurch auszeichnen, dass sie feste organische Füllstoffe bis zu einem Feststoffgehalt von 40 % oder mehr in disperser Verteilung enthalten, und/oder autokatalytische Polyole, die katalytisch aktive funktionelle Gruppen, insbesondere Amino-Gruppen, enthalten, und/oder Polyole natürlicher Herkunft, sogenannte "natural oil based polyols" (NOPs). Üblicherweise besitzen die Polyole eine Funktionalität von 1.8 bis 8 und zahlengemittelte Molekulargewichte im Bereich von 500 bis 15000. Üblicherweise kommen die Polyole mit OH-Zahlen im Bereich von 10 bis 1200 mgKOH/g zum Einsatz. Die zahlengemittelten Molekulargewichte werden üblicherweise durch Gelpermeationschromatographie (GPC), insbesondere mit Polypropylenglycol als Referenzsubstanz und Tetrahydrofuran (THF) als Elutionsmittel, bestimmt. Die OH-Zahlen können insbesondere nach der DIN-Norm DIN 53240:1971-12 bestimmt werden.

Polyetherpolyole können nach bekannten Verfahren hergestellt werden, beispielsweise durch anionische Polymerisation von Alkylenoxiden in Gegenwart von Alkalihydroxiden, Alkylialkoholaten oder Aminen als Katalysatoren und unter Zusatz mindestens eines Startermoleküls, dass bevorzugt 2 oder 3 reaktive Wasserstoffatome gebunden enthält oder durch kationische Polymerisation von Alkylenoxiden in Gegenwart von Lewis-Säuren wie beispielsweise Antimonpentachlorid oder Bortrifluorid-Etherat oder durch Doppelmetallcyanidkatalyse. Geeignete Alkylenoxide enthalten 2 bis 4 Kohlenstoffatome im Alkylenrest. Beispiele sind Tetrahydrofuran, 1,3-Propylenoxid, 1,2- bzw. 2,3-Butylenoxid; vorzugsweise werden Ethylenoxid und 1,2-Propylenoxid eingesetzt. Die Alkylenoxide können einzeln, kumulativ, blockweise, alternierend nacheinander oder als Mischungen verwendet werden. Als Startmoleküle kommen insbesondere Verbindungen mit mindestens 2, vorzugsweise 2 bis 8 Hydroxylgruppen oder mit mindestens zwei primären Aminogruppen im Molekül zum Einsatz. Als Startermoleküle eingesetzt werden können z.B. Wasser, 2-, 3- oder 4-wertige Alkohole wie Ethylenglykol, Propandiol-1,2 und -1,3, Diethylenglykol, Dipropylenglykol, Glycerin, Trimethylolpropan, Pentaerythrit, Rizinusöl usw., höhere polyfunktionelle Polyole, insbesondere Zuckerverbindungen wie beispielsweise Glucose, Sorbit, Mannit und Saccharose, mehrwertige Phenole, Resole, wie z.B. oligomere Kondensationsprodukte aus Phenol und Formaldehyd und Mannich-Kondensate aus Phenolen, Formaldehyd und Dialkanolaminen sowie Melamin, oder Amine wie Anilin, EDA, TDA, MDA und PMDA, besonders bevorzugt TDA und PMDA. Die Wahl des geeigneten Startermoleküls ist abhängig von dem jeweiligen Anwendungsgebiet des resultierenden Polyetherpolyols bei der Polyurethanherstellung (z.B. werden zur Herstellung von Polyurethanweichschäumen andere Polyole verwendet als bei der Herstellung von Polyurethanhartschaumstoffen).

Polyesterpolyole basieren auf Estern mehrwertiger aliphatischer oder aromatischer Carbonsäuren, bevorzugt mit 2 bis 12 Kohlenstoffatomen. Beispiele für aliphatische Carbonsäuren sind Bernsteinsäure, Glutarsäure, Adipinsäure, Korksäure, Azelainsäure, Sebacinsäure, Decandicarbonsäure, Maleinsäure und Fumarsäure. Beispiele für aromatische Carbonsäuren sind Phthalsäure, Isophthalsäure, Terephthalsäure und die isomeren Naphthalindicarbonsäuren. Die Polyesterpolyole werden durch Kondensation dieser mehrwertigen Carbonsäuren mit mehrwertigen Alkoholen, vorzugsweise von Diolen oder Triolen mit 2 bis 12, besonders bevorzugt mit 2 bis 6 Kohlenstoffatomen, bevorzugt Trimethylolpropan und Glycerin erhalten.

Polyetherpolycarbonatpolyole sind Polyole, welche Kohlenstoffdioxid als Carbonat gebunden enthalten. Da Kohlenstoffdioxid bei vielen Prozessen in der chemischen Industrie in großen Mengen als Nebenprodukt entsteht, ist die Verwendung von Kohlendioxid als Comonomer in Alkylenoxid-Polymerisationen aus kommerzieller Sicht von besonderem Interesse. Ein teilweiser Ersatz von Alkylenoxiden in Polyolen durch Kohlendioxid hat das Potential, die Kosten für die Herstellung von Polyolen deutlich zu senken. Außerdem ist die Verwendung von CO₂ als Comonomer ökologisch sehr vorteilhaft, da diese Reaktion die Umsetzung eines Treibhausgases zu einem Polymer darstellt. Die Herstellung von Polyetherpolycarbonatpolyolen durch Anlagerung von Alkylenoxiden und Kohlendioxid an H-funktionelle Startsubstanzen unter Verwendung von Katalysatoren ist seit langem bekannt. Verschiedene Katalysatorsysteme können hierbei zum Einsatz kommen: Die erste Generation stellten heterogene Zink- oder Aluminiumsalze dar, wie sie beispielsweise in US-A 3 900 424 oder US-A 3 953 383 beschrieben sind. Des Weiteren sind mono- und binukleare Metallkomplexe zur Copolymerisation von CO2 und Alkylenoxiden erfolgreich eingesetzt worden (WO 2010/028362, WO 2009/130470, WO 2013/022932 oder WO 2011/163133). Die wichtigste Klasse von Katalysatorsystemen für die Copolymerisation von Kohlenstoffdioxid und Alkylenoxiden stellen die Doppelmetallcyanidkatalysatoren, auch als DMC-Katalysatoren bezeichnet, dar (US-A 4500704, WO 2008/058913). Geeignete Alkylenoxide und H-funktionelle Startsubstanzen sind solche, die auch zur Herstellung von carbonatfreien Polyetherpolyolen - wie oben beschrieben - eingesetzt werden.

Polyole auf Basis nachwachsender Rohstoffe "Natural oil based polyols" (NOPs) zur Herstellung von Polyurethanschäumen sind mit Blick auf die langfristig begrenzte Verfügbarkeit fossiler Ressourcen, namentlich Öl, Kohle und Gas, und vor dem Hintergrund steigender Rohölpreise von zunehmendem Interesse und bereits vielfach in solchen Anwendungen beschrieben (WO 2005/033167; US 2006/0293400, WO 2006/094227, WO 2004/096882, US 2002/0103091, WO 2006/116456 und EP 1 678 232). Mittlerweile sind auf dem Markt eine Reihe dieser Polyole von verschiedenen Herstellern verfügbar (WO2004/020497, US2006/0229375, WO2009/058367). In Abhängigkeit vom Basis-Rohstoff (z.B. Sojabohnenöl, Palmöl oder Rizinusöl) und die daran angeschlossene Aufarbeitung ergeben sich Polyole mit unterschiedlichem Eigenschaftsbild. Hierbei können im Wesentlichen zwei Gruppen unterschieden werden: a) Polyole auf Basis nachwachsender Rohstoffe, die soweit modifiziert werden, dass sie zu 100 % zur Herstellung von Polyurethanen eingesetzt werden können (WO2004/020497, US2006/0229375); b) Polyole auf Basis nachwachsender Rohstoffe, die bedingt durch ihre Aufarbeitung und Eigenschaften nur zu einem gewissen Anteil das petrochemisch basierte Polyol ersetzen können (WO2009/058367).

Eine weitere Klasse von einsetzbaren Polyolen stellen die sogenannten Füllkörperpolyole (Polymerpolyole) dar. Diese zeichnen sich dadurch aus, dass sie feste organische Füllstoffe bis zu einem Feststoffgehalt von 40 % oder mehr in disperser Verteilung enthalten. Einsetzbar sind unter anderem SAN-, PHD- und PIPA-Polyole. SAN-Polyole sind hochreaktive Polyole, welche ein Copolymer auf der Basis von Styrol/Acrylnitril (SAN) dispergiert enthalten. PHD-Polyole sind hochreaktive Polyole, welche Polyharnstoff ebenfalls in dispergierter Form enthalten. PIPA-Polyole sind hochreaktive Polyole, welche ein Polyurethan, beispielsweise durch in situ-Reaktion eines Isocyanats mit einem Alkanolamin in einem konventionellen Polyol gebildet, in dispergierter Form enthalten.
Der Festkörperanteil, der je nach Anwendung bevorzugt zwischen 5 und 40 %, bezogen auf das Polyol liegt, ist für eine verbesserte Zellöffnung verantwortlich, so dass das Polyol insbesondere mit TDI kontrolliert verschäumbar wird und kein Schrumpfen der Schäume auftritt. Der Festkörper wirkt damit als wesentliche Prozesshilfe. Eine weitere Funktion besteht darin, über den Feststoffanteil die Härte zu kontrollieren, denn höhere Festkörperanteile bewirken eine größere Härte des Schaums. Die Formulierungen mit feststoffhaltigen Polyolen sind deutlich weniger eigenstabil und bedürfen daher neben der chemischen Stabilisierung durch die Vernetzungsreaktion eher auch zusätzlich einer physikalischen Stabilisierung. Je nach Feststoffgehalt der Polyole können diese z.B. alleine oder z.B. in Abmischung mit den oben genannten ungefüllten Polyolen eingesetzt werden.

Eine weitere Klasse von einsetzbaren Polyolen sind solche, die als Prepolymere durch Umsetzung von Polyol mit Isocyanat in einem Molverhältnis von 100 zu 1 bis 5 zu 1, bevorzugt 50 zu 1 bis 10 zu 1, erhalten werden. Solche Prepolymere werden vorzugsweise gelöst in Polymer angesetzt, wobei das Polyol bevorzugt dem zur Herstellung der Prepolymeren eingesetzten Polyol entspricht.

Eine weitere Klasse von einsetzbaren Polyolen stellen die sogenannten autokatalytischen Polyole, insbesondere autokatalytische Polyetherpolyole, dar. Solche Polyole basieren zum Beispiel auf Polyether-Blöcken, vorzugsweise auf Ethylenoxid- und/oder PropylenoxidBlöcken, und beinhalten zudem katalytisch aktive funktionelle Gruppen, wie zum Beispiel stickstoffhaltige funktionelle Gruppen, insbesondere Amino-Gruppen, vorzugsweise tertiäre Amin-Funktionen, Harnstoff-Gruppen und/oder Heterocyclen enthaltend Stickstoff-Atome. Durch die Verwendung solcher autokatalytsichen Polyole bei der Herstellung von Polyurethansystemen, insbesondere von Polyurethanschaumstoffen, bevorzugt von Polyurethanweichschaumstoffen, kann die benötigte Menge an ggf. zusätzlich verwendeten Katalysatoren je nach Anwendung gegebenfalls reduziert und/oder auf spezielle gewünschte Schaumeigenschaften angepasst werden. Geeignete Polyole sind zum Beispiel in WO0158976 (A1), WO2005063841 (A1), WO0222702 (A1), WO2006055396 (A1), WO03029320 (A1), WO0158976 (A1), US6924321 (B2), US6762274 (B2), EP2104696 (B1), WO2004060956 (A1) oder WO2013102053 (A1) beschrieben und können zum Beispiel unter den Handelsnamen Voractiv^{™} und/oder SpecFlex^{™} Activ bei der Firma Dow bezogen werden.

Je nach den geforderten Eigenschaften der resultierenden Schäume können entsprechende Polyole verwendet werden, wie sie beispielsweise beschrieben werden in: US 2007/0072951 A1, WO 2007/111828, US 2007/0238800, US 6359022 oder WO 96/12759. Weitere Polyole sind dem Fachmann bekannt und können zum Beispiel der EP-A-0380993 oder US-A-3346557 entnommen werden, auf die im vollen Umfang Bezug genommen wird.

Gemäß einer bevorzugten Ausführungsform der Erfindung, insbesondere zur Herstellung von Form- und hochelastischen Weichschaumstoffen, werden zwei- und/oder dreifunktionelle Polyetheralkohole eingesetzt, die primäre Hydroxylgruppen, bevorzugt über 50 %, besonders bevorzugt über 80 %, aufweisen, insbesondere solche mit einem Ethylenoxidblock am Kettenende. Je nach den geforderten Eigenschaften dieser erfindungsgemäß bevorzugten Ausführungsform, insbesondere zur Herstellung der oben genannten Schaumstoffe, werden neben den hier beschriebenen Polyetheralkoholen vorzugsweise weitere Polyetheralkohole eingesetzt, die primäre Hydroxylgruppen tragen und überwiegend auf Ethylenoxid basieren, insbesondere mit einem Anteil an Ethylenoxidblöcken von > 70 %, bevorzugt > 90 %. Alle im Sinne dieser bevorzugten Ausführungsform beschriebenen Polyetheralkohole besitzen vorzugsweise eine Funktionalität von 2 bis 8, besonders bevorzugt 2 bis 5, zahlengemittelte Molekulargewichte im Bereich von 2500 bis 15000, bevorzugt 4500 bis 12000 und üblicherweise OH-Zahlen im Bereich von 5 bis 80, vorzugsweise 20 bis 50 mgKOH/g.

Gemäß einer weiteren bevorzugten Ausführungsform der Erfindung, insbesondere zur Herstellung von Blockweichschaumstoffen, werden zwei- und/oder dreifunktionelle Polyetheralkohole eingesetzt, die sekundäre Hydroxylgruppen, bevorzugt über 50 %, besonders bevorzugt über 90 %, aufweisen, insbesondere solche mit einem Propylenoxidblock oder statistischen Propylen- und Ethylenoxidblock am Kettenende oder solche, die nur auf Propylenoxidblöcken basieren. Solche Polyetheralkohole besitzen vorzugsweise eine Funktionalität von 2 bis 8, besonders bevorzugt 2 bis 4, zahlengemittelte Molekulargewichte im Bereich von 500 bis 8000, vorzugsweise 800 bis 5000, besonders bevorzugt 2500 bis 4500 und üblicherweise OH-Zahlen im Bereich von 10 bis 100, vorzugsweise 20 bis 60 mgKOH/g.

Gemäß einer weiteren bevorzugten Ausführungsform der Erfindung, insbesondere zur Herstellung von Polyurethanschaumstoffen, vorzugsweise von Polyurethanweichschaumstoffen, bevorzugt zur Herstellung von Form- und hochelastischen Weichschaumstoffen, werden autokatalytische Polyole, wie oben beschrieben, eingesetzt.

Gemäß einer weiteren bevorzugten Ausführungsform der Erfindung, insbesondere zur Herstellung von Polyurethan-Polyesterweichschaumstoffen, werden Polyesteralkohole auf Basis von Diolen und/oder Triolen, bevorzugt Glycerin und/oder Trimethylolpropan, und aliphatischen Carbonsäuren, bevorzugt Adipinsäure, Korksäure, Azelainsäure und/oder Sebacinsäure, eingesetzt. Solche Polyesteralkohole besitzen vorzugsweise eine Funktionalität von 2 bis 4, besonders bevorzugt 2 bis 3, zahlengemittelte Molekulargewichte im Bereich von 200 - 4000, vorzugsweise 400 - 3000, besonders bevorzugt 600 - 2500 und üblicherweise OH-Zahlen im Bereich von 10 - 1000, vorzugsweise 20 - 500, besonders bevorzugt 30 - 300 mgKOH/g.

Gemäß einer weiteren bevorzugten Ausführungsform der Erfindung, insbesondere zur Herstellung von Polyisocyanurat (PIR)-Hartschaumstoffen, werden Polyesteralkohole auf Basis von Diolen und/oder Triolen, bevorzugt Monoethylenglycol, und aromatischen Carbonsäuren, bevorzugt Phthalsäure und/oder Terephthalsäure, eingesetzt. Solche Polyesteralkohole besitzen vorzugsweise eine Funktionalität von 2 bis 4, besonders bevorzugt 2 bis 3, zahlengemittelte Molekulargewichte im Bereich von 200 - 1500, vorzugsweise 300 - 1200, besonders bevorzugt 400 - 1000 und üblicherweise OH-Zahlen im Bereich von 100 - 500, vorzugsweise 150 - 300, besonders bevorzugt 180 - 250 mgKOH/g.

Gemäß einer weiteren bevorzugten Ausführungsform der Erfindung, insbesondere zur Herstellung von Polyurethan-Hartschaumstoffen, werden zwei- bis acht-funktionelle Polyetheralkohole eingesetzt, die sekundäre Hydroxylgruppen, bevorzugt über 50 %, besonders bevorzugt über 90 %, aufweisen, insbesondere solche mit einem Propylenoxidblock oder statistischen Propylen- und Ethylenoxidblock am Kettenende oder solche, die nur auf Propylenoxidblöcken basieren. Solche Polyetheralkohole besitzen vorzugsweise eine Funktionalität von 2 bis 8, besonders bevorzugt 3 bis 8, zahlengemittelte Molekulargewichte im Bereich von 500 bis 2000, vorzugsweise 800 bis 1200 und üblicherweise OH-Zahlen im Bereich von 100 bis 1200, bevorzugt 120 bis 700, besonders bevorzugt 200 bis 600 mgKOH/g. Je nach den geforderten Eigenschaften dieser erfindungsgemäß bevorzugten Schaumstoffe werden neben den hier beschriebenen Polyolen zusätzlich Polyetheralkohole, wie oben beschrieben mit größeren zahlengemittelten Molgewichten und niedrigeren OH-Zahlen, und/oder zusätzliche Polyesterpolyole, wie oben beschrieben basierend auf aromatischen Carbonsäuren, eingesetzt.

Gemäß einer weiteren bevorzugten Ausführungsform der Erfindung, insbesondere zur Herstellung von viskoelastischen Polyurethan-Schaumstoffen, werden vorzugsweise Gemische verschiedener, bevorzugt zweier oder dreier, mehrfunktioneller Polyesteralkohole und/oder Polyetheralkohole eingesetzt. Üblicherweise bestehen hier die eingesetzten Polyol-Kombinationen aus einem niedermolekularen "Crosslinker"-Polyol, beispielweise einem Hartschaum-Polyol, mit hoher Funktionalität (> 3) und/oder einem konventionellen hochmolekularen Blockweichschaum- oder HR-Polyol und/oder einem "Hypersoft"-Polyetherpolyol mit hohem Anteil an Ethylenoxidblöcken und mit zellöffnenden Eigenschaften.

Ein bevorzugtes Verhältnis von Isocyanat und Polyol, ausgedrückt als Index der Formulierung, d.h. als stöchiometrisches Verhältnis von Isocyanat-Gruppen zu gegenüber Isocyanat reaktiven Gruppen (z.B. OH-Gruppen, NH-Gruppen) multipliziert mit 100, liegt im Bereich von 10 bis 1000, bevorzugt 40 bis 350, besonders bevorzugt 70 bis 140. Ein Index von 100 steht für ein molares Verhältnis der reaktiven Gruppen von 1 zu 1.

Je nach Anwendung kann es erfindungsgemäß bevorzugt sein, dass neben den erfindungsgemäßen stickstoffhaltigen Verbindungen nach der Formel (VI), und/oder entsprechender protonierter und/oder quaternisierter Verbindungen, zusätzliche Katalysatoren eingesetzt werden und zwar einzeln während der Verschäumung oder als mit den erfindungsgemäßen stickstoffhaltigen Verbindungen nach der Formel (VI), und/oder entsprechenden protonierten und/oder quaternisierten Verbindungen vorgemischte Katalysatorkombination.

Der Ausdruck "zusätzliche Katalysatoren" umfasst im Sinne dieser Erfindung insbesondere den Einsatz von Verbindungen, die ungleich den erfindungsgemäßen stickstoffhaltigen Verbindungen nach der Formel (VI) und/oder entsprechender protonierter und/oder quaternisierter Verbindungen sind, und gleichzeitig in der Lage sind Isocyanat-Reaktionen, insbesondere die im Folgenden genannten Reaktionen, zu katalysieren und/oder bei der Herstellung von Polyisocyanat-Reaktionsprodukten, insbesondere bei der Herstellung von Polyurethansystemen, besonders bevorzugt bei der Herstellung von Polyurethanschaumstoffen als Katalysatoren, Co-Katalysatoren oder Aktivatoren eingesetzt werden.

Der Ausdruck "vorgemischte Katalysatorkombination", im Folgenden auch als Katalysatorkombination bezeichnet, umfasst im Sinne dieser Erfindung insbesondere fertige Mischungen von erfindungsgemäßen stickstoffhaltigen Verbindungen nach der Formel (VI), entsprechender protonierter und/oder quaternisierter Verbindungen, zusätzlicher Katalysatoren, sowie optional noch weiterer Inhalts- oder Zusatzstoffe wie zum Beispiel Wasser, organische Lösungsmittel, Säuren zur Blockierung der Amine, Emulgatoren, Tenside, Treibmittel, Antioxidantien, Flammschutzmittel, Stabilisatoren und/oder Siloxane, vorzugsweise Polyethersiloxane, die bereits vor der Verschäumung als solche vorliegen und während der Verschäumungsvorgangs nicht als Einzelkomponenten zugegeben werden müssen.

Als zusätzliche Katalysatoren können im Rahmen dieser Erfindung zum Beispiel jegliche Katalysatoren für die Reaktionen Isocyanat-Polyol (Urethan-Bildung) und/oder Isocyanat-Wasser (Amin- und Kohlenstoffdioxid-Bildung) und/oder die Isocyanat-Dimerisierung (Uretdion-Bildung) Isocyanat-Trimerisierung (Isocyanurat-Bildung), Isocyanat-Isocyanat mit CO₂-Abspaltung (Carbodiimid-Bildung) und/oder Isocyanat-Amin (Harnstoff-Bildung) und/oder "sekundäre" Vernetzungsreaktionen wie Isocyanat-Urethan (Allophanat-Bildung) und/oder Isocyanat-Harnstoff (Biuret-Bildung) und/oder Isocyanat-Carbodiimid (Uretonimin-Bildung) eingesetzt werden.

Geeignete zusätzliche Katalysatoren im Sinne der vorliegenden Erfindung sind beispielsweise Substanzen, die eine der vorgenannten Umsetzungen, insbesondere die Gelreaktion (Isocyanat-Polyol), die Treibreaktion (Isocyanat-Wasser) und/oder die Di- bzw. Trimerisierung des Isocyanats katalysieren. Solche Katalysatoren sind vorzugsweise stickstoffhaltige Verbindungen, insbesondere Amine und Ammonium-Salze, und/oder metallhaltige Verbindungen.

Geeignete stickstoffhaltige Verbindungen als zusätzliche Katalysatoren im Sinne der vorliegenden Erfindung sind alle stickstoffhaltigen Verbindungen nach dem Stand der Technik, ungleich den erfindungsgemäßen stickstoffhaltigen Verbindungen nach der Formel (VI), die eine der oben genannten Isocyanat-Reaktionen katalysieren und/oder zur Herstellung von Polyurethanen, insbesondere von Polyurethanschaumstoffen eingesetzt werden können.

Der Ausdruck ""stickstoffhaltige Verbindungen nach der Formel (VI)" umfasst im Sinne dieser Erfindung jeweils die entsprechenden protonierten und/oder quaternisierten Verbindungen sowie Mischungen dieser Verbindungen. Der Ausdruck "stickstoffhaltige Verbindungen nach der Formel (VI)" umfasst im Sinne dieser Erfindung jeweils die entsprechenden protonierten und/oder quaternisierten Verbindungen sowie Mischungen dieser Verbindungen. Der Ausdruck "zumindest eine stickstoffhaltige Verbindungen nach der Formel (VI)" umfasst im Sinne dieser Erfindung auch den gemeinsamen Einsatz solcher stickstoffhaltiger Verbindungen.

Beispiele für geeignete zusätzliche stickstoffhaltige Verbindungen als Katalysatoren im Sinne der vorliegenden Erfindung sind die Amine Triethylamin, N,N-Dimethylcyclohexylamin, N,N-Dicyclohexylmethylamin, N,N-Dimethylaminoethylamin, N,N,N',N'-Tetramethylethylen-1,2-diamin, N,N,N',N'-Tetramethylpropylen-1,3-diamin, N,N,N',N'-Tetramethyl-1,4-butandiamin, N,N,N',N'-Tetramethyl-1,6-hexandiamin, N,N,N',N",N"-Pentamethyldiethylentriamin, N,N,N'-Trimethylaminoethylethanolamin, N,N-Dimethylaminopropylamin, N,N-Diethylaminopropylamin, 1-(2-Aminoethyl)pyrrolidin, 1-(3-Aminopropyl)pyrrolidin, N,N-Dimethylaminopropyl-N',N'-dipropan-2-olamin, 2-[[3-(dimethylamino)propyl]methylamino]ethanol, 3-(2-Dimethylamino)ethoxy)propylamin, N,N-Bis[3-(dimethylamino)propyl]-amin, N,N,N',N",N"-Pentamethyldipropylentriamin, 1-[Bis[3-(dimethylamino)propyl]amino]-2-propanol, N,N-Bis[3-(dimethylamino)propyl]-N',N'-dimethylpropan-1,3-diamin, Triethylendiamin, 1,4-Diazabicyclo-[2.2.2]octane-2-methanol, N,N'-Dimethylpiperazin, 1,2-Dimethylimidazol, N-(2-Hydroxypropyl)imidazol, 1-Isobutyl-2-methylimidazol, N-(3-Aminopropyl)imidazol, N-Methylimidazol, N-Ethylmorpholin, N-Methylmorpholin, 2,2,4-Trimethyl-2-silamorpholin, N-Ethyl-2,2-dimethyl-2-silamorpholin, N-(2-Aminoethyl)morpholin, N-(2-Hydroxyethyl)morpholin, 2,2'-Dimorpholinodiethylether, N,N'-Dimethylpiperazin, N-(2-Hydroxyethyl)piperazin, N-(2-Aminoethyl)piperazin, N,N-Dimethylbenzylamin, N,N-Dimethylaminoethanol, N,N-Diethylaminoethanol, 1-(2-Hydroxyethyl)pyrrolidin, 3-Dimethylamino-1-propanol, 1-(3-Hydroxypropyl)-pyrrolidin, N,N-Dimethylaminoethoxyethanol, N,N-Diethylaminoethoxyethanol, Bis(2-Dimethylaminoethylether), N,N,N'-Trimethyl-N'-(2-hydroxyethyl)bis(2-aminoethyl)ether, N,N,N'-Trimethyl-N-3'-aminopropyl(bisaminoethyl)ether, Tris(dimethylaminopropyl)-hexahydro-1,3,5-triazin, 1,8-Diazabicyclo[5.4.0]undec-7-en, 1,5-Diazabicyclo[4.3.0]non-5-en, 1,5,7-triazabicyclo[4.4.0]dec-5-en, N-Methyl-1,5,7-triazabicyclo[4.4.0]dec-5-en, 1,4,6-triazabicyclo[3.3.0]oct-4-en, 1,1,3,3-Tetramethylguanidin, tert-Butyl-1,1,3,3-Tetramethylguanidin, Guanidin, 3-Dimethylaminopropylharnstoff, 1,3-Bis[3-(dimethylamino)propyl]harnstoff, Bis-N,N-(dimethylaminoethoxyethyl)isophorondicarbamat, 3-Dimethylamino-N,N-dimethylpropionamid und 2,4,6-Tris(dimethylaminomethyl)phenol. Geeignete zusätzliche stickstoffhaltige Katalysatoren, nach dem Stand der Technik, können zum Beispiel von der Firma Evonik unter dem Handelsnamen TEGOAMIN^{®} bezogen werden.

Geeignete metallhaltige Verbindungen als zusätzliche Katalysatoren im Sinne der vorliegenden Erfindung sind alle metallhaltigen Verbindungen nach dem Stand der Technik, die eine der oben genannten Isocyanat-Reaktionen katalysieren und/oder zur Herstellung von Polyurethanen, insbesondere von Polyurethanschaumstoffen neben den erfindungsgemäßen stickstoffhaltigen Verbindungen nach der Formel (VI) eingesetzt werden können. Sie können zum Beispiel ausgewählt werden aus der Gruppe der metallorganischen oder organometallischen Verbindungen, metallorganischen oder organometallischen Salze, organischen Metallsalze, anorganischen Metallsalze sowie aus der Gruppe der geladenen oder ungeladenen metallhaltigen Koordinationsverbindungen, insbesondere der Metall-Chelat-Komplexe.

Der Ausdruck "metallorganische oder organometallische Verbindungen" umfasst im Sinne dieser Erfindung insbesondere den Einsatz metallhaltiger Verbindungen, die über eine direkte Kohlenstoff-Metall-Bindung verfügen, hier auch als Metallorganyle (z.B. Zinnorganyle) oder organometallische bzw. Organometall-Verbindungen (z.B. Organozinn-Verbindungen) bezeichnet. Der Ausdruck "organometallische oder metallorganische Salze" umfasst im Sinne dieser Erfindung insbesondere den Einsatz von metallorganischen oder organometallischen Verbindungen mit Salzcharakter, das heißt Ionenverbindungen, bei denen entweder das Anion oder Kation von metallorganischer Natur ist (z.B. Organozinn-Oxide, Organozinn-Chloride oder Organozinn-Carboxylate). Der Ausdruck "organische Metallsalze" umfasst im Sinne dieser Erfindung insbesondere den Einsatz von metallhaltigen Verbindungen, die über keine direkte Kohlenstoff-Metall-Bindung verfügen und gleichzeitig Metallsalze sind, bei denen entweder das Anion oder das Kation eine organische Verbindung ist (z.B. Zinn(II)-Carboxylate). Der Ausdruck "anorganische Metallsalze" umfasst im Sinne dieser Erfindung insbesondere den Einsatz von metallhaltigen Verbindungen oder von Metallsalzen, bei denen weder Anion noch Kation eine organische Verbindung ist, z.B. Metall-Chloride (z.B. Zinn(II)-Chlorid), reine oder gemischte, also mehrere Metalle enthaltende, Metall-Oxide (z.B. Zinn-Oxide) und/oder Metall-Silicate oder -Alumosilicate. Der Ausdruck "Koordinationsverbindung" umfasst im Sinne dieser Erfindung insbesondere den Einsatz von metallhaltigen Verbindungen, die aus einem oder mehreren Zentralteilchen und einem oder mehreren Liganden aufgebaut sind, wobei die Zentralteilchen geladene oder ungeladene Metalle sind (z.B. Metall- bzw. Zinn-Amin-Komplexe). Der Ausdruck "Metall-Chelat-Komplexe" umfasst im Sinne dieser Erfindung insbesondere den Einsatz von metallhaltigen Koordinationsverbindungen, die Liganden mit mindestens zwei Koordinations- oder Bindungsstellen zum Metallzentrum aufweisen (z.B. Metall- bzw. Zinn-Polyamin- oder Metall- bzw. Zinn-Polyether-Komplexe).

Geeignete metallhaltige Verbindungen, insbesondere wie oben definiert, als zusätzliche Katalysatoren im Sinne der vorliegenden Erfindung können zum Beispiel ausgewählt werden aus allen metallhaltigen Verbindungen enthaltend Lithium, Natrium, Kalium, Magnesium, Calcium, Scandium, Yttrium, Titan, Zirconium, Vanadium, Niob, Chrom, Molybdän, Wolfram, Mangan, Cobalt, Nickel, Kupfer, Zink, Quecksilber, Aluminium, Gallium, Indium, Germanium, Zinn, Blei, und/oder Bismuth, insbesondere Natrium, Kalium, Magnesium, Calcium, Titan, Zirconium, Molybdän, Wolfram, Zink, Aluminium, Zinn und/oder Bismuth, besonders bevorzugt Zinn, Bismuth, Zink und/oder Kalium.

Geeignete anorganische Metallsalze, insbesondere wie oben definiert, als zusätzliche Katalysatoren im Sinne der vorliegenden Erfindung können zum Beispiel ausgewählt werden aus der Gruppe der Salze von anorganischen Säuren wie zum Beispiel Salzsäure, Kohlensäure, Schwefelsäure, Salpetersäure und Phosphorsäure und/oder von weiteren halogenhaltigen Säuren. Die resultierenden anorganische Metall-Salze, beispielsweise Metall-Chloride, Metall-Sulfate, Metall-Phosphate, bevorzugt Metall-Chloride wie Zinn(II)-Chlorid, können bei der Herstellung von Polyurethansystemen, insbesondere von Polyurethanschaumstoffen, in der Regel nur in Kombination mit anderen metallorganischen Salzen, organischen Metallsalzen oder stickstoffhaltigen Katalysatoren und nicht als einzige Katalysatoren, in reiner Form oder abgemischt in einem Lösungsmittel, eingesetzt werden.

Geeignete geladene oder ungeladene metallhaltige Koordinationsverbindungen, insbesondere der Metall-Chelat-Komplexe, insbesondere wie oben definiert, als zusätzliche Katalysatoren im Sinne der vorliegenden Erfindung, können zum Beispiel ausgewählt werden aus der Gruppe der ein- oder mehrkernigen Metall-Amin-, Metall-Polyamin, Metall-Polyether-, Metall-Polyester- und/oder Metall-Polyamin-Polyether-Komplexe. Solche Komplexe können entweder in situ während der Verschäumung und/oder der Verschäumung vorgelagert gebildet werden, oder als isolierte Komplexe, in reiner Form oder abgemischt in einem Lösungsmittel, eingesetzt werden. Als geeignete Komplexbildner, Liganden und/oder Chelat-Liganden kommen beispielsweise Acetylaceton, Benzoylaceton, Trifluoracetylaceton, Ethylacetoacetat, Salicylaldehyd, Salicyladehydimin und andere Schiff-Basen, Cyclopentanon-2-Carboxylat, Pyrrolidone wie zum Beispiel N-Methyl-2-pyrrollidon, N-Ethyl-2-pyrrolidon und Polyvinylpyrrolidone (verschiedener Molgewichtsverteilungen), Polyether verschiedener Molgewichte, cyclische Polyether wie zum Beispiel Kronenether und Diamine und Polyamine enthaltend primäre, sekundäre und/oder tertiäre Amine in Betracht.

Geeignete metallhaltige Koordinationsverbindungen sind zum Beispiel alle Metall-Acetylacetonate wie Nickel(II)-acetylacetonat, Zink(II)-acetylacetonat, Kupfer(II)-acetylacetonat, Molybdändioxo-acetylacetonat, alle Eisen-acetylacetonate, alle Cobaltacetylacetonate, alle Zirconium-acetylacetonate, alle Titan-acetylacetonate, alle Bismuthacetylacetonate und alle Zinn-acetylacetonate.

Geeignete metallorganischen Salze und organischen Metallsalze, insbesondere wie oben definiert, als zusätzliche Katalysatoren im Sinne der vorliegenden Erfindung können zum Beispiel ausgewählt werden aus der Gruppe der Salze von organischen Säuren.

Der Ausdruck "organische Säuren" umfasst im Sinne dieser Erfindung alle organisch chemischen, also Kohlenstoff enthaltenden, Verbindungen, die über eine funktionelle Gruppe verfügen, die mit Wasser und anderen protonierbaren Lösungsmitteln eine Gleichgewichtsreaktion im Sinne einer Säure-Base-Reaktion eingehen kann.

Geeignete organische Säuren können zum Beispiel ausgewählt sein aus der Gruppe von Carbonsäuren, d.h. organischen Verbindungen, die eine oder mehrere Carboxygruppen (*-COOH) tragen, so genannte Carboxylate, und/oder von Alkoholen, d.h. organische Verbindungen eine oder mehrere Hydroxygruppen (*-OH) tragen, so genannte Alkoholate, und/oder von Thiolen, d.h. organische Verbindungen, die eine oder mehrere Thiolgruppen (*-SH, bei Molekülen mit funktionellen Gruppe höherer Priorität auch als Mercapto-Gruppen bezeichnete) tragen, so genannte Thiolate (oder Mercaptide), und/oder von Mercaptoessigsäure-Estern als Spezialfall der Thiole, d.h. organische Verbindungen, die eine oder mehrere Mercaptoessigsäure-Ester-Gruppen tragen (*-O-CO-CH₂-CH₂-SH) tragen, so genannte Mercaptoacetate, und/oder von Schwefelsäureestern, d.h. organische Verbindungen, die eine oder mehrere Sulfat-Gruppen (*-O-SO₃H) tragen, so genannte Sulfate, und/oder von Sulfonsäuren, d.h. organische Verbindungen, die eine oder mehrere Sulfonsäure-Gruppen (*-SO₂-OH) tragen, so genannte Sulfonate, und/oder von Phoshporsäureestern (Alkylphosphate), d.h. organische Verbindungen, die ein- oder zweiwertige Alkyl-Ester der Orthophosphorsäure darstellen (*-O-PO(OH)₂ oder *-O-PO(OR)OH), so genannte Phosphate, und/oder von Phosphonsäuren, d.h. organische Verbindungen, die eine oder mehrere Phosphonsäure-Gruppen (*-PO(OH)₂) tragen, so genannte Phosphonate, und/oder Phosphorigsäureester, organische Verbindungen, die Alkyl-Ester der Phosphonsäure darstellen (*-P(OR)₂(OH) oder *-P(OR)(OH)₂), so genannte Phosphite.

Geeignete Carbonsäuren im Sinne der vorliegenden Erfindung sind beispielsweise alle linearen, verzweigten oder cyclischen, aliphatischen oder aromatischen, gesättigten oder ungesättigten, gegebenenfalls mit einem oder mehreren Heteroatomen, vorzugsweise mit Hydroxygruppen (*-OH), primären, sekundären oder tertiären Aminogruppen (*-NH₂, *-NHR, *-NR₂) oder Mercapto-Gruppen (*-SH), substituierten, oder durch ein oder mehrere Heteroatome unterbrochenen Mono-, Di- oder Poly-Carbonsäuren. Besonders geeignet im Sinne der vorliegenden Erfindung sind Carbonsäuren, an deren Carbobnyl-Kohlenstoffatom ein Wasserstoffatom oder ein linearer, verzweigter oder cyclischer, aliphatischer gesättigter oder ungesättigter, gegebenenfalls mit einem oder mehreren Heteroatomen, vorzugsweise mit Hydroxygruppen (*-OH), primären, sekundären oder tertiären Aminogruppen (*-NH₂, *-NHR, *-NR₂) oder Mercapto-Gruppen (*-SH), substituierter, oder durch ein oder mehrere Heteroatome unterbrochener Kohlenwasserstoffrest gebunden ist. Besonders geeignet im Sinne der vorliegenden Erfindung sind solche aliphatischen Carbonsäuren, die in 2-Position, also am Kohlenstoffatom neben der Carbonylfunktion, disubstituierte (tertiäre) oder trisubstituierte (quarternäre) Kohlenstoffe, bzw. entsprechende Kohlenwasserstoffreste, besitzen. Bevorzugt im Sinne der vorliegenden Erfindung sind solche aliphatischen Carbonsäuren, die in 2-Position eine oder zwei Methyl-, Ethyl, n-Propyl-, iso-Propyl, n-Butyl und/oder iso-Butyl-Verzweigung(en) aufweisen. Besonders bevorzugt im Sinne der vorliegenden Erfindung sind solche aliphatischen Carbonsäuren, insbesondere Monocarbonsäuren, die neben der beschriebenen Verzweigung in 2-Position eine gesättigte oder ungesättigte, lineare oder verzweigte Alkylkette aufweisen und optional mit einem oder mehreren Heteroatomen, vorzugsweise mit Hydroxygruppen (*-OH), primären, sekundären oder tertiären Aminogruppen (*-NH₂, *-NHR, *-NR₂) oder Mercapto-Gruppen (*-SH), substituiert sind. Insbesondere können geeignete Carbonsäuren ausgewählt werden aus der Gruppe der Neosäuren bzw. Koch-Säuren.

Beispiele für geeignete ein-, zwei- und mehrwertige, gesättigte und ungesättigte substituierte und nicht substituierte Carbonsäuren, Fettsäuren und Neosäuren bzw. Koch-Säuren, sind Carbonsäuren wie Ameisensäure, Essigsäure, Propionsäure, Acrylsäure, Buttersäure, Isobuttersäure, 2,2-Dimethylbuttersäure, Valeriansäure, Isovaleriansäure, 2-Methylvaleriansäure, 2,2-Dimethylvaleriansäure (iso-Heptansäure), Pivalinsäure, Capronsäure, 2-Ethylhexansäure (iso-Oktansäure), Önanthsäure, Caprylsäure, Pelargonsäure, iso-Nonansäure, 3,5,5-Trimethylhexansäure, 2,5,5-Trimethylhexansäure, 4,5,5-Trimethylhexansäure, 2,2,4,4-Tetramethylpentansäure, 6,6-Dimethylheptansäure, Caprinsäure, Neodecansäure, 7,7-Dimethyloctansäure, 2,2-Dimethyloctansäure, 2,4-Dimethyl-2-isopropylpentansäure, 2,2,3,5-Tetramethylhexansäure, 2,2-Diethylhexansäure, 2,5-Dimethyl-2-ethylhexansäure, Undecansäure, Laurinsäure, Tridecansäure, Neotridecansäure, Myristinsäure, Pentadecansäure, Palmitinsäure, Margarinsäure, Stearinsäure, Ölsäure, Linolsäure, Alpha-Linolensäure Phytansäure, Icosensäure, Erucasäure, Rizinolsäure, Vernolsäure, Arachinsäure, Arachidonsäure, Oxalsäure, Glycolsäure, Glyoxalsäure, Malonsäure, Milchsäure, Zitronensäure, Bernsteinsäure, Fumarsäure, Maleinsäure, Äpfelsäure, Weinsäure, Glutarsäure, Adipinsäure, Sorbinsäure, Zimtsäure, Salicylsäure, Benzoesäure, Terephthalsäure, Phthalsäure, iso-Phthalsäure, Nicotinsäure, Carbaminsäure, Pyrrolidin-2-Carbonsäure und Cyclohexancarbonsäure.

Geeignete Alkohole sind alle linearen, verzweigten oder cyclischen, aliphatischen oder aromatischen, gesättigten oder ungesättigten, gegebenenfalls mit einem oder mehreren Heteroatomen, vorzugsweise mit primären, sekundären oder tertiären Amino-Gruppen (*-NH₂, *-NHR, *-NR₂) oder Mercapto-Gruppen (*-SH), substituierte, oder durch ein oder mehrere Heteroatome unterbrochene einwertige Alkohole, zweiwertige Alkohole (Diole) und/oder mehrwertige Alkohole (Polyole). Geeignet hierfür sind zum Beispiel Methanol, Ethanol, Propanol, iso-Propanol, Butanol, tert-Butanol, Neopentylalkohol, Phenole und/oder Nonylphenol.

Geeignete Thiole, Mercaptoessigsäure-Ester, Schwefelsäureester, Sulfonsäuren, Phoshporsäureester (Alkylphosphate), Phosphonsäuren und/oder Posphorigsäureester sind beispielsweise alle linearen, verzweigten oder cyclischen, aliphatischen oder aromatischen, gesättigten oder ungesättigten, gegebenenfalls mit einem oder mehreren Heteroatomen substituierte, oder durch ein oder mehrere Heteroatome unterbrochene organische Verbindungen, enthaltend eine oder mehrere entsprechende funktionelle Gruppen, wie oben definiert. Geeignete hierfür sind zum Beispiel Dialkylphosphite, Methansulfonsäure, Trifluormethansulfonsäure, p-Toluolsulfonsäure, Dodecylbenzylsulfonsäure, Taurin, Isooctylmercaptoacetat, 2-Ethylhexylmercaptoacetat, Ethantiol und/oder n-Laurylmercaptid.

Besonders geeignete metallorganische Salze und organische Metallsalze, wie oben definiert, als zusätzliche Katalysatoren im Sinne der vorliegenden Erfindung sind zum Beispiel Organozinn-, Zinn-, Zink-, Bismuth und Kalium-Salze, insbesondere entsprechende MetallCarboxylate, -Alkoholate, -Thiolate und -Mercaptoacetate, wie zum Beispiel Dibutylzinndiacetat, Dimethylzinndilaurat, Dibutylzinndilaurat (DBTDL), Dioctylzinndilaurat (DOTDL), Dimethylzinndineodecanoat, Dibutylzinndineodecanoat, Dioctylzinndineodecanoat, Dibutylzinndioleat, Dibutylzinn-bis-n-laurylmercaptid, Dimethylzinn-bis-n-laurylmercaptid, Monomethylzinn-tris-2-ethylhexylmercaptoacetat, Dimethylzinn-bis-2-ethylhexylmercaptoacetat, Dibutylzinn-bis-2-ethylhexylmercaptoacetat, Dioctylzinn-bis-isooctylmercaptoacetat, Zinn(II)-acetat, Zinn(II)-2-ethylhexanoat (Zinn(II)-octoat), Zinn(II)-isononanoat (Zinn(II)-3,5,5-trimethylhexanoat), Zinn(II)-neodecanoat, Zinn(II)-ricinoleat, Zink(II)-acetat, Zink(II)-2-ethylhexanoat (Zink(II)-octoat), Zink(II)-isononanoat (Zink(II)-3,5,5-trimethylhexanoat), Zink(II)-neodecanoat, Zink(II)-ricinoleat, Bismuthacetat, Bismuth-2-ethylhexanoat, Bismuthoctoat, Bismuthisononanoat, Bismuthneodecanoat, Kaliumformiat, Kaliumacetat, Kalium-2-ethylhexanoat (Kaliumoctoat), Kalium-isononanoat, Kalium-neodecanoat und/oder Kaliumricinoleat.

Bei der erfindungsgemäßen Herstellung von Polyurethanen, kann es je nach Art der Anwendung, insbesondere bei der Herstellung von Polyurethanschaumstoffen, bevorzugt sein, die Verwendung von metallorganischen Salzen wie zum Beispiel von Dibutylzinndilaurat auszuschließen.

Geeignete zusätzliche metallhaltige Katalysatoren werden in der Regel vorzugsweise so ausgewählt, dass sie keinen störenden Eigengeruch aufweisen, toxikologisch im Wesentlichen unbedenklich sind und dass die resultierenden Polyurethansysteme, insbesondere Polyurethanschäume möglichst geringe Katalysator-bedingte Emissionen aufweisen.

Neben zusätzlichen Aminen und metallhaltigen Verbindungen können auch Ammoniumsalze als zusätzliche Katalysatoren eingesetzt werden. Geeignet sind zum Beispiel Ammoniumformiat und/oder Ammoniumacetat.

Geeignete zusätzliche Katalysatoren sind beispielsweise in DE 102007046860, EP 1985642, EP 1985644, EP 1977825, US 2008/0234402, EP 0656382 B1 und US 2007/0282026 A1 und den darin zitierten Patentschriften genannt.

Geeignete Einsatzmengen an zusätzlichen Katalysatoren richten sich nach dem Typ des Katalysators und liegen vorzugsweise im Bereich von 0,01 bis 10,0 pphp, besonders bevorzugt im Bereich von 0,02 bis 5,00 pphp (= Gewichtsteile bezogen auf 100 Gewichtsteile Polyol) bzw. 0,10 bis 10,0 pphp für Kaliumsalze.

Je nach Anwendung kann es erfindungsgemäß bevorzugt sein, wenn im Falle der Verwendung von zusätzlichen Katalysatoren und/oder von vorgemischten Katalysatorkombinationen, wie oben definiert, aus der Summe aller eingesetzten stickstoffhaltigen Verbindungen, also die Summe der erfindungsgemäßen stickstoffhaltigen Verbindungen nach Formel (VI) und der zusätzlichen stickstoffhaltigen Katalysatoren nach dem Stand der Technik, gegenüber der Summe der metallhaltigen Katalysatoren, insbesondere Kalium-, Zink- und/oder Zinn-Katalysatoren, ein molares Mengenverhältnis von 1:0,05 bis 0,05:1, vorzugsweise 1:0,07 bis 0,07:1 und besonders bevorzugt 1:0,1 bis 0,1:1 resultiert.

Es kann erfindungsgemäß bevorzugt sein, dass zusätzlichen Katalysatoren und/oder vorgemischte Katalysatorkombinationen, wie oben definiert, frei von Dimethylamin-tragenden stickstoffhaltigen Verbindungen sind. Frei von Dimethylamin-tragenden stickstoffhaltigen Verbindungen sind Katalysatorkombinationen im Sinne dieser Erfindung vorzugsweise dann, wenn weniger als 75 Gew-%, insbesondere weniger als 50 Gew-%, bevorzugt weniger als 30 Gew-%, besonders bevorzugt weniger als 10 Gew-% der Katalysatoren in der Katalysatormischung Dimethylamin-tragende, stickstoffhaltige Verbindungen beinhalten. Insbesondere bevorzugt sind Katalysatorkombinationen, die gar keine, also 0 Gew-%, Dimethylamin-tragenden, stickstoffhaltigen Verbindungen enthalten.

Um eine Reaktion der Komponenten untereinander zu vermeiden, insbesondere Reaktion von erfindungsgemäß verwendeten stickstoffhaltigen Verbindungen nach Formel (VI) und/oder zusätzlichen stickstoffhaltigen Katalysatoren mit zusätzlichen metallhaltigen Katalysatoren, insbesondere Kalium-, Zink- und/oder Zinn-Katalysatoren, kann es bevorzugt sein, diese Komponenten getrennt voneinander zu lagern und dann der Isocyanat- und Polyol-Reaktionsmischung gleichzeitig oder nacheinander zuzuführen.

Es entspricht einer bevorzugten Ausführungsform der Erfindung, wenn im Rahmen der erfindungsgemäßen Verwendung zumindest eine stickstoffhaltige Verbindung nach Formel (VI) und/oder eine entsprechende quaternisierte und/oder protonierte Verbindung in Kombination mit
a) einer oder mehreren zusätzlichen (also nicht erfindungsgemäßen) stickstoffhaltigen Verbindungen als zusätzliche Katalysatoren, vorzugsweise wie oben definiert und beispielhaft beschrieben,
b) einem oder mehreren zusätzlichen metallhaltigen Katalysatoren, insbesondere einer oder mehrerer Zinn-, Zink-, Bismuth- und/oder Kaliumverbindungen, vorzugsweise wie oben definiert und beispielhaft beschrieben,
c) einer oder mehrerer Säuren zur Blockierung der enthaltenden Amine, vorzugsweise wie oben beschrieben,
d) Wasser
e) einem oder mehreren organischen Lösungsmitteln, vorzugsweise wie im Folgenden definiert und beispielhaft beschrieben,
f) einem oder mehreren chemischen oder physikalischen Treibmitteln, vorzugsweise wie im Folgenden beschrieben,
g) einem oder mehreren Stabilisatoren gegen oxidativen Abbau, beispielsweise Antioxidantien, vorzugsweise wie im Folgenden beschrieben,
h) einem oder mehreren Flammschutzmitteln, vorzugsweise wie im Folgenden beschrieben, und/oder
i) einem oder mehreren Schaumstabilisatoren auf der Basis von Siloxanen und/oder Polydialkylsiloxan-Polyoxyalkylen-copolymeren, vorzugsweise wie im Folgenden definiert und beschrieben, und/oder
j) einem oder mehreren weiteren Zusatzstoffen, zum Beispiel ausgewählt aus der Gruppe der Tenside Biozide, Farbstoffe, Pigmente, Füllstoffe, Antistatik-Additive, Vernetzer, Kettenverlängerer, Zellöffner, und/oder Duftstoffe
eingesetzt wird, wobei vorteilhafterweise vor der Herstellung des Polyurethans, insbesondere des Polyurethanschaumstoffs, zuerst eine Zusammensetzung hergestellt wird, beispielsweise im Sinne einer Vordosierung der Einzelkomponenten im Mischkopf oder als vorgemischte Katalysatorkombination, wie oben definiert, beinhaltend die vorgenannte Kombination. Insbesondere kann die genannte stickstoffhaltige Verbindung nach Formel (VI) auch als technische Produktmischung eingesetzt werden. Geeignete technische Produktmischungen sind in der Beschreibung erläutert.

Im Sinne der vorgenannten bevorzugten Ausführungsform sind besonders bevorzugte Kombinationen im Rahmen der vorliegenden Erfindung solche Zusammensetzungen, in denen zumindest eine stickstoffhaltige Verbindung nach Formel (VI) und/oder eine entsprechende quaternisierte und/oder protonierte Verbindung verwendet wird in Kombination mit a), mit b), mit c), mit d), mit e), mit f), mit a), b), c), d) e) und f), mit a) und b), mit a) und c), mit a), b) und c), mit a), b) und d), mit a), b) und e), mit a), b), d) und e), mit a), b), d), e) und f), mit a), b), e) und f), mit a), c) und d), mit a), c) und e), mit a), c), d) und e), mit a), b) und e), mit a), b), c) und e), mit a), b), c), d) und e), mit c) und d), mit c) und e), mit c), d) und e), mit c), e) und f), mit c), d), e) und f), mit c), d), e) und f), mit b) und c), mit b), c) und d), mit b), c) und e), mit b), c), d) und e), mit b), c), e) und f), mit b), c), d), e), f), mit b) und d), mit b) und e), mit b), d) und e), mit b), d) und f), mit b), e) und f), oder mit b), d), e) und f).

Die erfindungsgemäßen Verbindungen der Formel (VI), die entsprechenden protonierten und/oder quaternisierten Verbindungen können als Reinsubstanz oder in Abmischung, zum Beispiel mit geeigneten Lösungsmitteln und/oder weiteren Zusatzstoffen einzeln während der Verschäumung oder als vorgemischte Katalysatorkombination, wie oben definiert, eingesetzt werden.

Als Lösungsmittel kommen alle nach dem Stand der Technik geeigneten Substanzen in Frage. Je nach Anwendung können aprotisch-unpolare, aprotisch-polare und protische Lösungsmittel eingesetzt werden. Geeignete aprotisch-unpolare Lösungsmittel können beispielsweise ausgewählt werden aus folgenden Substanzklassen, bzw. Substanzklassen enthaltend folgende funktionelle Gruppen: aromatische Kohlenwasserstoffe, aliphatische Kohlenwasserstoffe (Alkane (Paraffine) und Olefine), Carbonsäurester und Polyester, (Poly-)Ether und/oder halogenierte Kohlenwasserstoffe niedriger Polarität. Geeignete aprotisch-polare Lösungsmittel können beispielsweise ausgewählt werden aus folgenden Substanzklassen, bzw. Substanzklassen enthaltend folgende funktionelle Gruppen: Ketone, Lactone, Lactame, Nitrile, Carbonsäureamide, Sulfoxide und/oder Sulfone. Geeignete protische Lösungsmittel können beispielsweise ausgewählt werden aus folgenden Substanzklassen, bzw. Substanzklassen enthaltend folgende funktionelle Gruppen: Alkohole, Polyole, (Poly-)Alkylenglykole, Amine, Carbonsäuren, insbesondere Fettsäuren und/oder primäre und sekundäre Amide.
Bevorzugte Lösungsmittel sind beispielsweise Mineralöle, Hexan, Pentan, Heptan, Dekan oder Mischungen von gesättigten Kohlenwasserstoffen, wie z. B. Kaydol Produkte der Fa. Sonneborn, Glycolether wie Ethylenglycoldimethylether (Monoglyme), Bis(2-methoxyethyl)ether (Diglyme), Triethylenglycoldimethylether (Triglyme), Tetraethylenglycoldimethylether (Tetraglyme), Polyester- und Polyether-Polyole, Polyole basierend auf nachwachsenden Rohstoffen (NOPs), endverkappte Polyether, vorzugsweise Dialkyl-Polyether, die als Alkylreste Butyl-/Methyl-, Methyl-/Methyl- oder Butyl-/Butyl-Reste aufweisen, bevorzugt solche, die aus Diol-gestarteten Polyethern erhältlich sind, Glycole, Glycerin, Carbonsäureester, vorzugsweise Fettsäureester, beispielsweise Ethylacetat und Isopropylmyristat, Polycarbonate, Phthalate, vorzugsweise Dibutylphthalat (DBP), Dioctylphthalat (DNOP), Diethylhexylphthalat (DEHP), Diisononylphthalat (DINP), Dimethylphthalat (DMP), Diethylphthalat (DEP), Cyclohexanoate, vorzugsweise Diisononylcyclohexanoat (DINCH).

Besonders bevorzugte Lösungsmittel sind Verbindungen, die in der Verschäumung problemlos verarbeitet werden können und die Eigenschaften des Schaums nicht negativ beeinflussen. So sind zum Beispiel Isocyanat-reaktive Verbindungen geeignet, da diese mit in die Polymermatrix einreagieren und keine Emissionen im Schaum generieren. Beispiele sind OH-funktionelle Verbindungen wie (Poly-)Alkylenglykole, vorzugsweise Monoethylenglycol (MEG oder EG), Diethylenglycol (DEG), Triethylenglycol (TEG), 1-2-Propylenglycol (PG), Dipropylenglycol (DPG), Trimethylenglycol (1,3-Propandiol PDO), Tetramethylenglycol (Butandiol BDO), Butyldiglycol (BDG), Neopentylglycol, 2-Methyl-1,3-propandiol (Ortegol CXT) und höhere Homologe davon wie zum Beispiel Polyethylenglykol (PEG) mit mittleren Molekülmassen zwischen 200 und 3000. Weitere besonders bevorzugte OH-funktionelle Verbindungen sind Polyether mit mittleren Molekülmassen von 200 bis 4500, insbesondere 400 bis 2000, hierin bevorzugt Wasser-, Allyl-, Butyl- oder Nonyl-gestartete Polyether, insbesondere solche, die auf Propylenoxid- (PO) und/oder Ethylenoxidblöcken (EO) basieren.

Werden im Rahmen einer bevorzugten Ausführungsform die zumindest eine stickstoffhaltige Verbindung nach der Formel (VI), in Kombination mit zumindest einem Lösungsmittel eingesetzt wird, so beträgt das Massenverhältnis von insgesamt eingesetztem Katalysator, umfassend alle katalytisch aktiven Verbindungen gleich und ungleich der Formel (VI), zu Lösungsmittel vorzugsweise von 100 zu 1 bis 1 zu 4, bevorzugt von 50 zu 1 bis 1 zu 3 und besonders bevorzugt von 25 zu 1 bis 1 zu 2.

Als Zusatzstoffe können alle nach dem Stand der Technik bekannten Substanzen verwendet werden, die bei der Herstellung von Polyurethanen, insbesondere von Polyurethanschaumstoffen, Verwendung finden, wie zum Beispiel Treibmittel, vorzugsweise Wasser zur Bildung von CO₂ und, falls nötig, weitere physikalische Treibmittel, Vernetzer und Kettenverlängerer, Stabilisatoren gegen oxidativen Abbau (so genannte Antioxidantien), Flammschutzmittel, Tenside, Biozide, zellverfeinernde Additive, Zellöffner, feste Füllstoffe, Antistatik-Additive, Nukleierungsmittel, Verdicker, Farbstoffe, Pigmente, Farbpasten, Duftstoffe, Emulgatoren, Puffersubstanzen und/oder zusätzliche katalytisch aktive Substanzen, insbesondere wie oben definiert.

Sollen Polyurethanschäume als Polyurethansysteme hergestellt werden, kann es vorteilhaft sein, Wasser als Treibmittel einzusetzen. Vorzugsweise wird so viel Wasser eingesetzt, dass die Wassermenge 0,10 bis 25,0 pphp beträgt (pphp = Gewichtsteile bezogen auf 100 Gewichtsteile Polyol).

Es können auch geeignete physikalische Treibmittel eingesetzt werden. Dies sind beispielsweise verflüssigtes CO₂, und leichtflüchtige Flüssigkeiten, beispielsweise Kohlenwasserstoffe mit 3, 4 oder 5 Kohlenstoff-Atomen, bevorzugt cyclo-, iso- und n-Pentan, Fluorkohlenwasserstoffe, bevorzugt HFC 245fa, HFC 134a und HFC 365mfc, Fluorchlorkohlenwasserstoffe, bevorzugt HCFC 141b, Hydrofluoroolefine (HFO) oder Hydrohaloolefine wie z.B. 1234ze, 1233zd(E) oder 1336mzz, Sauerstoff-haltige Verbindungen wie Methylformiat, Aceton und Dimethoxymethan, oder Chlorkohlenwasserstoffe, bevorzugt Dichlormethan und 1,2-Dichlorethan.

Neben Wasser und den physikalischen Treibmitteln, können auch andere chemische Treibmittel eingesetzt werden, die mit Isocyanaten unter Gasentwicklung reagieren, wie beispielsweise Ameisensäure.

Als Vernetzer und Kettenverlängerer werden niedermolekulare, gegenüber Isocyanaten reaktive, mehrfunktionelle Verbindungen bezeichnet. Geeignet sind zum Beispiel Hydroxyl- oder Amin-terminierte Substanzen wie Glycerin, Neopentylglycol, 2-Methyl-1,3-propandiol, Triethanolamin (TEOA), Diethanolamin (DEOA) und Trimethylolpropan. Die Einsatzkonzentration liegt üblicherweise zwischen 0,1 und 5 Teilen, bezogen auf 100 Teile Polyol, kann aber je nach Formulierung auch davon abweichen. Bei der Verwendung von crude MDI bei der Formverschäumung übernimmt dies ebenfalls eine vernetzende Funktion. Der Gehalt an niedermolekularen Vernetzern kann daher bei steigender Menge an crude MDI entsprechend reduziert werden.

Geeignete Stabilisatoren gegen oxidativen Abbau, so genannte Antioxidantien, sind vorzugsweise alle gängigen Radikalfänger, Peroxidfänger, UV-Absorber, Lichtstabilisatoren, Komplexbildner für Metallionenverunreinigungen (Metalldeaktivatoren). Bevorzugt einsetzbar sind Verbindungen folgender Substanzklassen, bzw. Substanzklassen enthaltend folgende funktionelle Gruppen, wobei als Substituenten an den jeweiligen Grundkörpern insbesondere diejenigen bevorzugt sind, die gegenüber Isocyanat reaktive Gruppen besitzen: 2-(2'-Hydroxyphenyl)benzotriazole, 2-Hydroxybenzophenone, Benzoesäuren und Benzoate, Phenole, insbesondere enthaltend tert-Butyl- und oder Methylsubstituenten am Aromaten, Benzofuranone, Diarylamine, Triazine, 2,2,6,6-Tetramethylpiperidine, Hydroxylamine, Alkyl- und Arylphosphite, Sulfide, Zinkcarboxylate, Diketone. Als Phenole können beispielsweise Ester basierend auf 3-(4-Hydroxyphenyl)propionsäure wie Triethyleneglycol-bis-[3-(3-tert-butyl-4-hydroxy-5-methylphenyl)propionat], Octadecyl-3-(3,5-di-tert-butyl-4-hydroxyphenyl)-propionat, oder Methylendiphenole wie 4,4'-Butyliden-bis-(6-tert-butyl-3-methylphenol) ein-gesetzt werden. Als 2-(2'-Hydroxyphenyl)benzotriazole sind beispielsweise 2-(2'Hydroxy-5-methylphenyl)benzotriazol oder 2-(2'Hydroxy-3',5'-di-tert-butylphenyl)benzotriazol bevorzugt. Als 2-Hydroxybenzophenone sind beispielsweise 2-Hydoxy-4-n-octoxybenzophenon, 2,2',4,4'-tetrahydroxybenzophenon oder 2,4-dihydroxybenzophenon bevorzugt. Als Benzoate sind beispielsweise Hexadecyl-3,5-di-tert-butyl-4-hydroxybenzoat oder Tannine bevorzugt.

Geeignete Flammschutzmittel im Sinne dieser Erfindung sind alle Substanzen, die nach dem Stand der Technik als dafür geeignet betrachten werden. Bevorzugte Flammschutzmittel sind beispielsweise flüssige organische Phosphor-Verbindungen, wie halogenfreie organische Phosphate, z.B. Triethylphosphat (TEP), halogenierte Phosphate, z.B. Tris(1-chlor-2-propyl)phosphat (TCPP) und Tris(2-chlorethyl)phosphat (TCEP) und organische Phosphonate, z.B. Dimethylmethanphosphonat (DMMP), Dimethylpropanphosphonat (DMPP), oder Feststoffe wie Ammoniumpolyphosphat (APP) und roter Phosphor. Des Weiteren sind als Flammschutzmittel halogenierte Verbindungen, beispielsweise halogenierte Polyole, sowie Feststoffe wie Blähgraphit und Melamin geeignet.

Tenside, die insbesondere bei der Herstellung von Polyurethanschaumstoffen eingesetzt werden, können zum Beispiel ausgewählt sein aus der Gruppe umfassend anionische Tenside, kationische Tenside, nichtionische Tenside und/oder amphotere Tenside. Als Tenside können erfindungsgemäß auch polymere Emulgatoren wie Polyalkylpolyoxyalkylpolyacrylate, Polyvinylpyrrolidone oder Polyvinylacetate verwendet werden.

Als Biozide können zum Beispiel handelsübliche Produkte verwendet werden, wie Chlorophen, Benzisothiazolin, Hexahydro-1,3,5-tris(hydroxyethyl-s-triazin), Chloromethylisothiazolin, Methylisothiazolin oder 1,6-Dihydroxy-2,5-dioxohexan, die unter den Handelsnamen BIT 10, Nipacide BCP, Acticide MBS, Nipacide BK, Nipacide Cl, Nipacide FC bekannt sind.

Zur Beeinflussung der Schaumeigenschaften von Polyurethanschäumen können bei deren Herstellung insbesondere Siloxane bzw. organomodifizierte Siloxane eingesetzt werden, wobei die im Stand der Technik genannten Substanzen verwendet werden können. Vorzugsweise werden solche Verbindungen eingesetzt, die für die jeweiligen Schaumtypen (Hartschäume, Heissweichschäume, viskoelastische Schäume, Esterschäume, Kaltweichschäume (HR-Schäume), halbharte Schäume) besonders geeignet sind. Geeignete (organomodifizierte) Siloxane sind beispielsweise in den folgenden Schriften beschrieben: EP 0839852, EP 1544235, DE 102004001408, EP 0839852, WO 2005/118668, US 20070072951, DE 2533074, EP 1537159 EP 533202, US 3933695, EP 0780414, DE 4239054, DE 4229402, EP 867465. Die Herstellung dieser Verbindungen kann wie im Stand der Technik beschrieben erfolgen. Geeignete Beispiele sind z. B. in US 4147847, EP 0493836 und US 4855379 beschrieben.

Als (Schaum-)stabilisatoren können alle aus dem Stand der Technik bekannten Stabilisatoren eingesetzt werden. Bevorzugt werden Schaumstabilisatoren auf der Basis von Polydialkylsiloxan-Polyoxyalkylen-copolymeren, wie sie allgemein bei Herstellung von Urethanschaumstoffen verwendet werden, eingesetzt. Diese Verbindungen sind vorzugsweise so aufgebaut, dass z.B. ein langkettiges Copolymerisat aus Ethylen- und Propylenoxid mit einem Polydimethylsiloxanrest verbunden ist. Die Verknüpfung zwischen dem Polydialkylsiloxan und dem Polyetherteil kann dabei über eine SiC-Verknüpfung oder eine Si-O-C-Bindung erfolgen. Strukturell kann der oder können die unterschiedlichen Polyether terminal oder seitenständig an das Polydialkylsiloxan gebunden sein. Der Alkylrest oder die verschiedenen Alkylreste können dabei aliphatisch, cycloaliphatisch oder aromatisch sein. Ganz besonders vorteilhaft sind dabei Methylgruppen. Das Polydialkylsiloxan kann dabei linear sein oder auch Verzweigungen enthalten. Geeignete Stabilisatoren, insbesondere Schaumstabilisatoren sind unter anderem in US 2834748, US2917480 sowie in US3629308 beschrieben. Geeignete Stabilisatoren können von der Evonik Industries AG unter dem Handelsnamen TEGOSTAB^{®} bezogen werden.

Geeignete Siloxane, die bei der erfindungsgemäßen Verwendung der stickstoffhaltigen Verbindungen nach der Formel (VI) und/oder der entsprechenden quaternisierten und/oder protonierten Verbindungen bei der Herstellung von Polyurethanschaumstoffen eingesetzt werden können, haben insbesondere die folgende Struktur: 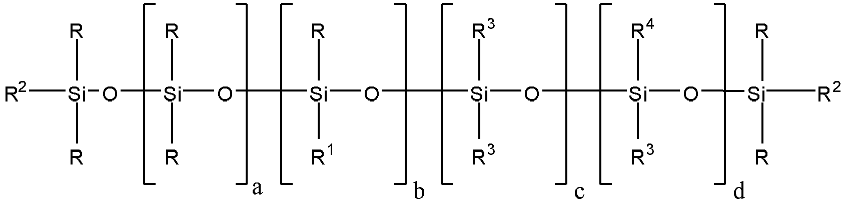 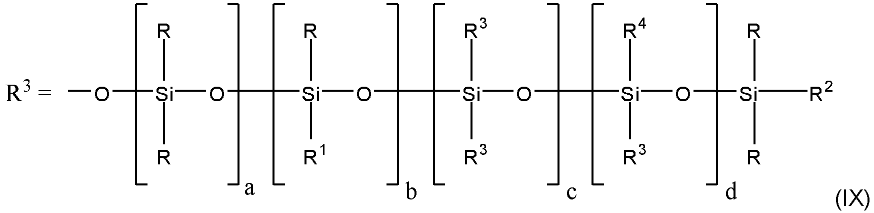 worin
- a: unabhängig voneinander 0 bis 500 ist, vorzugsweise 1 bis 300 und insbesondere 2 bis 150,
- b: unabhängig voneinander 0 bis 60 ist, vorzugsweise 1 bis 50 und insbesondere 1 bis 30,
- c: unabhängig voneinander 0 bis 10, vorzugsweise 0 oder > 0 bis 5, ist,
- d: unabhängig voneinander 0 bis 10, vorzugsweise 0 oder > 0 bis 5, ist,
mit der Maßgabe, dass pro Molekül der Formel (IX) die mittlere Anzahl Σd der T-Einheiten [SiR³R⁴O] und die mittlere Anzahl Σc der Q-Einheiten [SiR³R³O] pro Molekül jeweils nicht größer als 50, die mittlere Anzahl Σa der D-Einheiten [SiRRO] pro Molekül nicht größer als 2000 und die mittlere Anzahl Σb der R¹ tragenden Siloxy-Einheiten pro Molekül nicht größer als 100 ist,
- R: unabhängig voneinander mindestens ein Rest aus der Gruppe linearer, cyclischer oder verzweigter, aliphatischer oder aromatischer, gesättigter oder ungesättigter Kohlenwasserstoffreste mit 1 bis zu 20 C-Atomen, vorzugsweise jedoch ein Methylrest ist,
- R²: unabhängig voneinander R¹ oder R ist,
- R¹: ist ungleich R und unabhängig voneinander ein organischer Rest und/oder ein Polyetherrest, bevorzugt ist der R¹ ein Rest ausgewählt aus der Gruppe -CH₂-CH₂-CH₂-O-(CH₂-CH₂O-)ₓ-(CH₂-CH(R')O-)_{y}-R" -CH₂-CH₂-O-(CH₂-CH₂O-)ₓ-(CH₂-CH(R')O-)_{y}-R" -O-(C₂H₄O-)ₓ-(C₃H₅O-)_{y}-R' -CH₂-R^{IV} -CH₂-CH₂-(O)_{x'}-R^{IV} -CH₂-CH₂-CH₂-O-CH₂-CH(OH)-CH₂OH 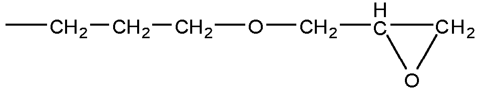 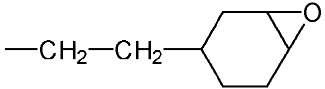
oder-CH₂-CH₂-CH₂-O-CH₂-C(CH₂OH)₂-CH₂-CH₃ ist,
worin
- x: 0 bis 100, vorzugsweise > 0, insbesondere 1 bis 50,
- x': 0 oder 1,
- y: 0 bis 100, vorzugsweise > 0, insbesondere 1 bis 50,
- z: 0 bis 100, vorzugsweise > 0, insbesondere 1 bis 10,
- R': unabhängig voneinander eine gegebenenfalls substituierte, beispielsweise mit Alkylresten, Arylresten oder Halogenalkyl- oder Halogenarylresten substituierte, Alkyl- oder Arylgruppe mit 1 bis 12 C-Atomen ist, wobei innerhalb eines Restes R¹ und/oder eines Moleküls der Formel (IX) untereinander verschiedene Substituenten R' vorliegen können, und
- R": unabhängig voneinander ein Wasserstoffrest oder eine Alkylgruppe mit 1 bis 4 C-Atomen, eine Gruppe -C(O)-R'" mit R'" = Alkylrest, eine Gruppe -CH₂-O-R', eine Alkylarylgruppe, wie z. B. eine Benzylgruppe, die Gruppe -C(O)NH-R' bedeutet,
- R^{IV}: ein linearer, cyclischer oder verzweigter, auch weiter substituierter, z. B. mit Halogenen substituierter, Kohlenwasserstoffrest mit 1 bis 50, vorzugsweise 9 bis 45, bevorzugt 13 bis 37 C-Atomen ist,
- R⁴: unabhängig voneinander R, R¹ und/oder ein mit Heteroatomen substituierter, funktionalisierter, organischer, gesättigter oder ungesättigter Rest ausgewählt aus der Gruppe der Alkyl-, Aryl-, Chloralkyl-, Chloraryl-, Fluoralkyl-, Cyanoalkyl-, Acryloxyaryl-, Acryloxyalkyl-, Methacryloxyalkyl-, Methacryloxypropyl- oder Vinyl-Rest sein kann,
mit der Maßgabe, dass mindestens ein Substituent aus R¹, R² und R⁴ nicht gleich R ist. Die verschiedenen Monomereinheiten der in den Formeln angegebenen Bausteine (Siloxanketten bzw. Polyoxyalkylenkette) können untereinander blockweise aufgebaut sein mit einer beliebigen Anzahl an Blöcken und einer beliebigen Sequenz oder einer statistischen Verteilung unterliegen. Die in den Formeln verwendeten Indices sind als statistische Mittelwerte zu betrachten.

Die Herstellung der Siloxane nach Formel (IX) kann nach den bekannten Methoden erfolgen, wie z.B. die edelmetallkatalysierte Hydro-silylierungsreaktion von Verbindungen, die eine Doppelbindung enthalten, mit entsprechenden Wasserstoffsiloxanen wie beispielsweise in EP 1520870, beschrieben. Die Schrift EP 1520870 wird hiermit als Referenz eingeführt und gilt als Teil des Offenbarungsgehaltes der vorliegenden Erfindung.

Als Verbindungen, die zumindest eine Doppelbindung pro Molekül aufweisen, können z. B. α -Olefine, Vinylpolyoxyalkylene und/oder Allylpolyoxyalkylene eingesetzt werden. Vorzugsweise werden Vinylpolyoxyalkylene und/oder Allylpolyoxyalkylene ein-gesetzt. Besonders bevorzugte Vinylpolyoxyalkylene sind z. B. Vinylpolyoxyalkylene mit einem Molgewicht im Bereich von 100 g/Mol bis 8.000 g/Mol, die aus den Monomeren Propylenoxid, Ethylenoxid, Butylenoxid und/oder Styroloxid blockweise oder statistisch verteilt aufgebaut sein können und die sowohl hydroxyfunktionell als auch durch eine Methyletherfunktion oder eine Acetoxyfunktion endverkappt sein können. Besonders bevor-zugte Allylpolyoxyalkylene sind z. B. Allylpolyoxyalkylene mit einem Molgewicht im Bereich von 100 g/Mol bis 5.000 g/Mol, die aus den Monomeren Propylenoxid, Ethylenoxid, Butylenoxid und/oder Styroloxid blockweise oder statistisch verteilt aufgebaut sein können und die sowohl hydroxyfunktionell als auch durch eine Methyletherfunktion oder eine Acetoxyfunktion end-verkappt sein können. Besonders bevorzugt werden als Verbindungen, die zumindest eine Doppelbindung pro Molekül aufweisen, die in den Beispielen genannten α -Olefine, Allylalkohol, 1-Hexenol, Vinylpolyoxyalkylene und/oder Allylpolyoxyalkylene sowie Allylglycidylether und Vinylcyclohexenoxid eingesetzt.

Bevorzugt werden im Rahmen der vorliegenden Erfindung (insbesondere im Rahmen der erfindungsgemäßen Verwendung) Siloxane der Formel (IX) eingesetzt worin a unabhängig voneinander 1 bis 300 ist, b unabhängig voneinander 1 bis 50 ist, c unabhängig voneinander 0 bis 4 ist, d unabhängig voneinander 0 bis 4 ist, mit der Maßgabe, dass pro Molekül der Formel (IX) die mittlere Anzahl ∑ d der T-Einheiten und die mittlere Anzahl ∑ c der Q-Einheiten pro Molekül jeweils nicht größer als 20, die mittlere Anzahl ∑ a der D-Einheiten pro Molekül nicht größer als 1500 und die mittlere Anzahl ∑ b der R¹ tragenden Siloxy-Einheiten pro Molekül nicht größer als 50 ist.

In einer besonders bevorzugten Ausführungsform der vorliegenden Erfindung (insbesondere im Rahmen der erfindungsgemäßen Verwendung) werden Siloxane der Formel (IX) eingesetzt worin R¹ unabhängig voneinander ein organischer Rest -CH₂-CH₂-CH₂-O-(CH₂-CH₂O-)ₓ-(CH₂-CH(R')O-)_{y}-R" -CH₂-CH₂-O-(CH₂-CH₂O-)ₓ-(CH₂-CH(R')O-)_{y}-R" -CH₂-R^{IV}
ist, worin x 0 bis 100, vorzugsweise > 0, insbesondere 1 bis 50 und y 0 bis 100, vorzugsweise > 0, insbesondere 1 bis 50 ist, R' unabhängig voneinander verschieden sein können und Methyl-, Ethyl- und/oder Phenyl-Reste darstellen. R" unabhängig voneinander ein Wasserstoffrest oder eine Alkylgruppe mit 1 bis 4 C-Atomen, eine Gruppe -C(O)-R'" mit R'" = Alkylrest, eine Gruppe -CH₂-O-R', eine Alkylarylgruppe, wie z. B. eine Benzylgruppe, die Gruppe -C(O)NH-R' bedeutet, R^{IV} ein linearer, cyclischer oder verzweigter, gegebenenfalls substituierter, z. B. mit Halogenen substituierter, Kohlenwasserstoffrest mit 1 bis 50, vorzugsweise 9 bis 45, bevorzugt 13 bis 37 C-Atomen ist.

In einer weiteren bevorzugten Ausführungsform der vorliegenden Erfindung (insbesondere im Rahmen der erfindungsgemäßen Verwendung), vorzugsweise zur Herstellung von Hartschaumstoffen, werden Siloxane der Formel (IX) eingesetzt, worin R¹ unabhängig voneinander ein organischer Rest ausgewählt aus der Gruppe umfassend -CH₂-CH₂-CH₂-O-(CH₂-CH₂O-)ₓ-(CH₂-CH(R')O-)_{y}-R" und/oder -CH₂-CH₂-O-(CH₂-CH₂O-)ₓ-(CH₂-CH(R')O-)_{y}-R" und /oder -CH₂-R^{IV} ist, worin x 0 bis 100, vorzugsweise > 0, insbesondere 1 bis 50, y 0 bis 100, vorzugsweise > 0, insbesondere 1 bis 50, R' Methyl ist und R" unabhängig voneinander ein Wasserstoff-rest oder eine Alkylgruppe mit 1 bis 4 C-Atomen, eine Gruppe C(O)-R'" mit R'" = Alkylrest, eine Gruppe -CH₂-O-R', eine Alkylarylgruppe, wie z. B. eine Benzylgruppe, die Gruppe C(O)NH-R' bedeutet, wobei der molare Anteil an Oxyethylen-Einheiten bezogen auf die Gesamtmenge Oxyalkyleneinheiten mind. 70% der Oxalkylen-Einheiten ausmacht, also x/(x+y) >0,7 ist. Unter dieser Voraussetzung ist bevorzugt, dass Außerdem die PolyoxyalkylenKette am Ende einen Wasserstoff trägt. Unter diesen Vorrausetzungen werden gemäß einer weiteren bevorzugten Ausführungsform der Erfindung (insbesondere im Rahmen der erfindungsgemäßen Verwendung) Siloxane der Formel (IX) verwendet in denen die in Rest R¹ enthaltenen Oxalkylen-Einheiten ausschließlich Oxyethylen-Einheiten sind und dabei der Rest R" kein Wasserstoff ist.

In einer weiteren bevorzugten Ausführungsform der vorliegenden Erfindung (insbesondere im Rahmen der erfindungsgemäßen Verwendung), vorzugsweise zur Herstellung von Blockweichschaumstoffen, werden Siloxane der Formel (IX) eingesetzt, worin R1 unabhängig voneinander ein organischer Rest ausgewählt aus der Gruppe umfassend -CH₂-CH₂-CH₂-O-(CH₂-CH₂O-)ₓ-(CH₂-CH(R')O-)_{y}-R" und/oder -CH₂-CH₂-O-(CH₂-CH₂O-)ₓ-(CH₂-CH(R')O-)_{y}-R" und /oder -CH₂-R^{IV} ist, worin x 0 bis 100, vorzugsweise > 0, insbesondere 1 bis 50, y 0 bis 100, vorzugsweise > 0, insbesondere 1 bis 50, R' Methyl ist und R" unabhängig voneinander ein Wasserstoff-rest oder eine Alkylgruppe mit 1 bis 4 C-Atomen, eine Gruppe C(O)-R'" mit R'" = Alkylrest, eine Gruppe -CH₂-O-R', eine Alkylarylgruppe, wie z. B. eine Benzylgruppe, die Gruppe C(O)NH-R' bedeutet, wobei der molare Anteil an Oxyethylen-Einheiten bezogen auf die Gesamtmenge Oxyalkyleneinheiten maximal 60% der Oxalkylen-Einheiten ausmacht, also x/(x+y) <0,6 ist.

In einer weiteren bevorzugten Ausführungsform der vorliegenden Erfindung (insbesondere im Rahmen der erfindungsgemäßen Verwendung) werden Siloxane der Formel (IX) verwendet in denen bei der Hydrosilylierung unter anderem Olefine eingesetzt werden wodurch R¹ mindestens zu 10 mol%, bevorzugt zu mindestens 20 mol%, besonders bevorzugt zu mindestens 40 mol% aus CH₂-R^{IV} besteht, wobei R^{IV} ein linearer oder verzweigter Kohlenwasserstoff mit 9 bis 17 Kohlenstoffatomen ist.

In einer weiteren bevorzugten Ausführungsform der vorliegenden Erfindung (insbesondere im Rahmen der erfindungsgemäßen Verwendung) werden Siloxane der Formel (IX) verwendet in denen die endständigen, oder auch alpha- und omega- genannten, Positionen am Siloxan mindestens teilweise mit Resten R¹ funktionalisiert sind. Es sind hierbei zumindest 10 mol-%, bevorzugt zumindest 30 mol-%, besonders bevorzugt zumindest 50 mol-% der endständigen Positionen mit Resten R¹ funktionalisiert.
In einer besonders bevorzugten Ausführungsform der Erfindung (insbesondere im Rahmen der erfindungsgemäßen Verwendung) werden Siloxane der Formel (IX) verwendet in denen im statistischen Mittel maximal 50%, bevorzugt maximal 45%, besonders bevorzugt maximal 40% des gesamten mittleren Molgewichts des Siloxans auf die aufsummierte Molmasse aller, gegebenenfalls unterschiedlichen, Reste R¹ im Siloxan entfällt.

In einer weiteren bevorzugten Ausführungsform der vorliegenden Erfindung (insbesondere im Rahmen der erfindungsgemäßen Verwendung) werden Siloxane der Formel (IX) verwendet in denen der Rest R für Methyl steht und die Strukturelemente mit dem Index a in größerer Anzahl vorliegen als die Strukturelemente mit dem Index b, in der Art, dass der Quotient a/b mindestens gleich sieben , vorzugsweise größer 10, besonders bevorzugt größer 12 ist.

In einer weiteren bevorzugten Ausführungsform der vorliegenden Erfindung (insbesondere im Rahmen der erfindungsgemäßen Verwendung) werden Siloxane der Formel (IX) verwendet in denen die in Rest R¹ enthaltenen Oxalkylen-Einheiten ausschließlich Oxyethylen-Einheiten sind und dabei der Rest R" kein Wasserstoff ist.

Die Siloxane können im Rahmen der vorliegenden Erfindung (insbesondere im Rahmen der erfindungsgemäßen Verwendung) auch als Teil von Zusammensetzungen mit verschiedenen Trägermedien eingesetzt werden. Als Trägermedien kommen beispielweise Glykole, wie beispielsweise Monoethylenglycol (MEG), Diethylenglycol (DEG), Propylenglycol (PG) oder Dipropylenglycol (DPG), Alkoxylate oder Öle synthetischer und/oder natürlicher Herkunft in Frage.

Bevorzugt wird der Zusammensetzung zur Herstellung von Polyurethansystemen, vorzugsweise von Polyurethanschaumstoffen, so viel der Siloxane nach der Formel (IX) zugegeben, dass der Massenanteil an Verbindungen der Formel (IX) am fertigen Polyurethansystem, vorzugsweise dem Polyurethanschaum von 0,01 bis 10 Gew.-%, bevorzugt von 0,1 bis 3 Gew.-% beträgt.

Die erfindungsgemäßen stickstoffhaltigen Verbindungen nach der Formel (VI), entsprechende quaternisierte und/oder protonierte Verbindungen, werden bevorzugt bei der Herstellung von Polyurethansystemen, insbesondere Polyurethanschäumen verwendet.

Es kann vorteilhaft sein, wenn bei der Herstellung des Polyurethansystems eine Zusammensetzung hergestellt und/oder eingesetzt wird, die zumindest eine erfindungsgemäße stickstoffhaltige Verbindung nach der Formel (VI), wie zuvor definiert, und/oder eine entsprechende quaternisierten und/oder protonierte Verbindung, zumindest eine Polyolkomponente, ggf. zumindest eine Isocyanatkomponente sowie optional ein oder mehrere Treibmittel aufweist, und diese Zusammensetzung reagiert wird. Besonders bevorzugt werden solche Zusammensetzungen eingesetzt, die die oben bei der Verwendung beschriebenen Stoffe bzw. Komponenten zur Herstellung von Polyurethanen, insbesondere von Polyurethanschäumen aufweisen.

Ein weiterer Gegenstand der Erfindung liegt in der Verwendung der zuvor beschriebenen stickstoffhaltigen Verbindung gemäß Formel (VI), und/oder einer entsprechenden quaternisierten und/oder protonierten Verbindung, zur Herstellung von emissionsarmen Polyurethanen, insbesondere von emissionsarmen Polyurethanschaumstoffen, nämlich vorteilhafterweise emissionsarm hinsichtlich Emissionen stickstoffhaltiger Verbindungen, wie vorher auch Amin-Emissionen genannt, vorteilhafterweise emissionsarm hinsichtlich Emissionen von Dimethylformamid (DMF), und/oder vorteilhafterweise emissionsarm hinsichtlich Aldehydemissionen, insbesondere Formaldehydemissionen. Bezüglich des Begriffs "emissionsarm" wird auf die vorangegangene Beschreibung und die dortigen Erläuterungen, insbesondere Testmethoden, verwiesen. Bezüglich bevorzugter Ausgestaltungen dieses Gegenstandes wird ebenfalls auf die vorangegangene Beschreibung verwiesen, insbesondere auf die genannten bevorzugten Ausführungsformen.

Ein weiterer Gegenstand der Erfindung liegt in der Verwendung der zuvor beschriebenen stickstoffhaltigen Verbindung gemäß Formel (VI), und/oder einer entsprechenden quaternisierten und/oder protonierten Verbindung, zur Herstellung von geruchsarmen Polyurethanen, vorzugsweise von geruchsarmen Polyurethanschaumstoffen, insbesondere von geruchsarmen Polyurethanweichschaumstoffen. Bezüglich des Begriffs "geruchsarm" wird auf die vorangegangene Beschreibung und die dortige Erläuterungen verwiesen. Bezüglich bevorzugter Ausgestaltungen dieses Gegenstandes wird ebenfalls auf die vorangegangene Beschreibung verwiesen, insbesondere auf die genannten bevorzugten Ausführungsformen.

Ein weiterer Gegenstand der Erfindung liegt in der Verwendung der zuvor beschriebenen stickstoffhaltigen Verbindung gemäß Formel (VI), und/oder einer entsprechenden quaternisierten und/oder protonierten Verbindung, zur Herstellung von alterungsbeständigen Polyurethansystemen, insbesondere Polyurethanschaumstoffen. Bezüglich des Begriffs "alterungsbeständig" wird auf die vorangegangene Beschreibung und die dortige Erläuterungen und Testmethoden verwiesen. Bezüglich bevorzugter Ausgestaltungen dieses Gegenstandes wird ebenfalls auf die vorangegangene Beschreibung verwiesen, insbesondere auf die genannten bevorzugten Ausführungsformen.

Ein weiterer Gegenstand der Erfindung liegt in der Verwendung der zuvor beschriebenen stickstoffhaltigen Verbindung gemäß Formel (VI), und/oder einer entsprechenden quaternisierten und/oder protonierten Verbindung, zur Herstellung von verfärbungsminimierten Polyurethansystemen, insbesondere von Polyurethanschaumstoffen, vorzugsweise von Polyurethanen für die Anwendung in der Automobilindustrie, insbesondere in Automobilinnenräumen, beispielsweise als Dachhimmel, Innenverkleidungen von Türen, ausgestanzte Sonnenblenden, Lenkräder und/oder Sitzsysteme. Verfärbungsminimiert bedeutet, dass die unter Verwendung von erfindungsgemäßen stickstoffhaltigen Katalysatoren bereitgestellten Polyurethansysteme insbesondere zu geringeren Verfärbungen von Kunststoffen, insbesondere Kunststoffbezügen, in Automobilinnenräumen führen als solche Polyurethansysteme, die unter Einsatz konventioneller Katalysatoren nach dem Stand der Technik, insbesondere von nicht erfindungsgemäßen Aminen, hergestellt werden, wie dies beispielsweise an Hand eines PVC-Verfärbungs-Tests gezeigt werden kann. Auch hier wird auf die vorangegangene Beschreibung und die dortigen Erläuterungen und Testmethoden verwiesen. Bezüglich bevorzugter Ausgestaltungen dieses Gegenstandes wird ebenfalls auf die vorangegangene Beschreibung verwiesen, insbesondere auf die genannten bevorzugten Ausführungsformen.

Ein weiterer Gegenstand der Erfindung liegt in der Verwendung der zuvor beschriebenen stickstoffhaltigen Verbindung gemäß Formel (VI), und/oder einer entsprechenden quaternisierten und/oder protonierten Verbindung, zur Herstellung von Polyurethansystemen mit breitem Verarbeitungsspiel, insbesondere von halbharten Polyurethanschäumen (offenzelligen Hartschäumen, insbesondere zur Anwendung als Dachhimmel in Automobilinnenräumen). "Breites Verarbeitungsspiel" heißt, dass vorteilhafterweise eine größere Variationsbreite der Einsatzkonzentration der erfindungsgemäßen stickstoffhaltigen Verbindungen ohne negative Beeinflussung der gewünschten Materialeigenschaften, beispielsweise der Offenzelligkeit des Schaums oder der Raumgewichtsverteilung über den Schaumblock, möglich ist gegenüber vergleichbaren oder für solche Anwendungen üblicherweise eingesetzten Amin-Katalysatoren nach dem Stand der Technik. Auch hier wird auf die vorangegangene Beschreibung und die dortigen Erläuterungen und Testmethoden verwiesen. Bezüglich bevorzugter Ausgestaltungen dieses Gegenstandes wird ebenfalls auf die vorangegangene Beschreibung verwiesen, insbesondere auf die genannten bevorzugten Ausführungsformen.

Ein weiterer Gegenstand der Erfindung ist Zusammensetzung enthaltend mindestens eine Polyolkomponente, wobei die Zusammensetzung zumindest eine stickstoffhaltige Verbindung der Formel (VI) wie zuvor definiert und beschrieben, und/oder die entsprechenden quaternisierten und/oder protonierten Verbindungen aufweist, wobei die Zusammensetzung vorzugsweise mindestens eine Isocyanatkomponente aufweist, und wobei die stickstoffhaltige Verbindung der Formel (VI) vorzugsweise als technische Produktmischung, wie zuvor beschrieben, enthalten ist, und wobei die Zusammensetzung vorzugsweise zusätzliche Amin-Katalysatoren ungleich Formel (VI) umfasst.

Das molare Verhältnis der Gesamtmenge der stickstoffhaltigen Katalysatoren, umfassend die stickstoffhaltigen Verbindungen der Formel (VI), gegenüber der Gesamtmenge der mit Isocyanaten reaktiven Gruppen der Polyolkomponente beträgt dabei vorzugsweise von 4 x 10-⁴ zu 1 bis 0,2 zu 1.

Es ist bevorzugt, dass die stickstoffhaltigen Verbindungen der Formel (VI), entsprechende quaternisierte und/oder protonierte Verbindungen, in Gesamtmenge in einem Massenanteil von 0,01 bis 20,0 Teilen (pphp), bevorzugt 0,01 bis 5,00 Teilen und besonders bevorzugt 0,02 bis 3,00 Teilen bezogen auf 100 Teile (pphp) Polyolkomponente eingesetzt werden.

Die erfindungsgemäße Zusammensetzung kann zusätzlich ein oder mehrere Treibmittel, wie sie oben beschrieben sind, aufweisen. Neben oder an Stelle von Treibmitteln kann die erfindungsgemäße Zusammensetzung weitere Zusatzstoffe/Hilfsmittel oder Additive aufweisen, die bei der Herstellung von Polyurethansystemen, bevorzugt Polyurethanschäumen eingesetzt werden. Eine Auswahl geeigneter Hilfsmittel/Zusatzstoffe/Additive, wie z. B. Schaumstabilisatoren oder Flammschutzmittel, wurde bereits oben bei der Herstellung der Polyurethansysteme, insbesondere der Polyurethanschaumstoffe beschrieben.

Die Verarbeitung der erfindungsgemäßen Zusammensetzungen zu Polyurethansystemen, insbesondere Polyurethanschaumstoffen, kann nach allen dem Fachmann geläufigen Verfahren erfolgen, beispielsweise im Handmischverfahren oder bevorzugt mit Hilfe von Verschäumungsmaschinen, insbesondere Niederdruck- oder Hochdruck-Verschäumungsmaschinen. Dabei können diskontinuierliche Verfahren, beispielsweise zur Produktion von Formschäumen, Kühlschränken, Automobilsitzen, und Paneelen, oder kontinuierliche Verfahren, beispielsweise bei Dämmplatten, Metallverbundelementen, Blockschäumen oder bei Sprühverfahren zum Einsatz kommen.

Es können alle dem Fachmann bekannten Verfahren zur Herstellung von Polyurethanschaumstoffen verwendet werden. So kann zum Beispiel der Schäumungsprozess sowohl in horizontaler als auch in vertikaler Richtung, in diskontinuierlichen oder kontinuierlichen Anlagen erfolgen. Ebenso können die erfindungsgemäß eingesetzten Zusammensetzungen für die CO₂-Technologie benutzt werden. Die Verwendung in Niederdruck- und Hochdruckmaschinen ist möglich, wobei die Zusammensetzungen sowohl direkt in die Mischkammer dosiert werden können oder auch schon vor der Mischkammer einer der danach in die Mischkammer gelangenden Komponenten zugemischt werden. Die Zumischung kann auch im Rohstofftank erfolgen.

Mittels der erfindungsgemäßen Verwendung von stickstoffhaltigen Verbindungen der Formel (VI), und/oder der entsprechenden quaternisierten und/oder protonierten Verbindungen, sind die nachfolgend beschriebenen erfindungsgemäßen Polyurethansysteme erhältlich.

Ein weiterer Gegenstand der vorliegenden Erfindung ist eine Zusammensetzung, geeignet zum Einsatz bei der Herstellung von Polyurethanen, insbesondere von Polyurethanschaumstoffen, enthaltend
(a) zumindest eine stickstoffhaltige Verbindung der Formel (VI), vorteilhafterweise in einer Gesamtmenge von ≥ 5 Gew.-%, bevorzugt 20-95 Gew.-%, insbesondere 30-70 Gew.-%,
(b) optional 1-(3-Aminopropyl)pyrrolidin, vorteilhafterweise in einer Menge ≥ 5 Gew.-%, bevorzugt 20-95 Gew.-%, insbesondere 30-70 Gew.-%,
(c) optional 1-(2-Aminoethyl)pyrrolidin, vorteilhafterweise in einer Menge ≥ 5 Gew.-%, bevorzugt 20-95 Gew.-%, insbesondere 30-70 Gew.-%,
(d) optional 1-(2-Hydroxyethyl)pyrrolidin, vorteilhafterweise in einer Menge ≥ 5 Gew.-%, bevorzugt 20-95 Gew.-%, insbesondere 30-70 Gew.-%,
(e) optional 1-(3-Hydroxypropyl)pyrrolidin, vorteilhafterweise in einer Menge ≥ 5 Gew.-%, bevorzugt 20-95 Gew.-%, insbesondere 30-70 Gew.-%,
(f) optional Trimethylendiamin, vorteilhafterweise in einer Menge ≥ 5 Gew.-%, insbesondere 20-95 Gew.-%, bevorzugt 30-70 Gew.-%,
(g) optional Ethylendiamin (EDA), vorteilhafterweise in einer Menge ≤ 95 Gew.-%, insbesondere 20-90 Gew.-%, bevorzugt 30-80 Gew.-%,
(h) optional 1,4-Butandiol, vorteilhafterweise in einer Menge ≤ 95 Gew.-%, insbesondere 20-90 Gew.-%, bevorzugt 30-80 Gew.-%,
(i) optional Monoethylenglycol (MEG), vorteilhafterweise in einer Menge ≤ 95 Gew.-%, insbesondere 20-90 Gew.-%, bevorzugt 30-80 Gew.-%,
(j) optional Diethylenglycol (DEG) vorteilhafterweise in einer Menge ≤ 95 Gew.-%, insbesondere 20-90 Gew.-%, bevorzugt 30-80 Gew.-%,
(k) optional Monoethanolamin (MEA) vorteilhafterweise in einer Menge ≤ 95 Gew.-%, insbesondere 20-90 Gew.-%, bevorzugt 30-80 Gew.-%,
(l) optional Propylenglycol (PG) vorteilhafterweise in einer Menge ≤ 95 Gew.-%, insbesondere 20-90 Gew.-%, bevorzugt 30-80 Gew.-%,
(m) optional Dipropylenglycol (DPG) vorteilhafterweise in einer Menge ≤ 95 Gew.-%, insbesondere 20-90 Gew.-%, bevorzugt 30-80 Gew.-%, und/oder
(n) optional Trimethylenglycol, Butyldiglycol, Neopentylglycol, 2-Methyl-1,3-propandiol, N,N-Dimethylcyclohexylamin, N,N-Dimethylaminopropylamin, Triethylendiamin, 2,2,4-Trimethyl-2-silamorpholin, N-Ethyl-2,2-dimethyl-2-silamorpholin, N-(2-Aminoethyl)morpholin, N-(2-Hydroxyethyl)morpholin, N,N-Dimethylaminoethanol, N,N-Diethylaminoethanol, Bis(2-Dimethylaminoethylether), N,N-Dimethylaminoethoxyethanol, N,N,N'-Trimethyl-N'-(2-hydroxyethyl)bis(2-aminoethyl)ether, Tris(dimethylaminopropyl)hexahydro-1,3,5-triazin, 1,8-Diazabicyclo[5.4.0]undec-7-en, 1,5-Diazabicyclo[4.3.0]non-5-en, 1,5,7-triazabicyclo[4.4.0]dec-5-en, N-Methyl-1,5,7-triazabicyclo[4.4.0]dec-5-en, 1,4,6-triazabicyclo[3.3.0]oct-4-en, 1,1,3,3-Tetramethylguanidin und/oder 2,4,6-Tris(dimethylaminomethyl)phenol, vorteilhafterweise in einer Gesamtmenge ≤ 95 Gew.-%, insbesondere 20-90 Gew.-%, bevorzugt 30-80 Gew.-%.

Im Sinne des vorgenannten Gegenstandes der vorliegenden Erfindung sind besonders bevorzugte Zusammensetzungen solche Zusammensetzungen, in denen zumindest eine stickstoffhaltige Verbindung nach Formel (VI) und/oder eine entsprechende quaternisierte und/oder protonierte Verbindung verwendet wird in Kombination mit b), mit c), mit d), mit e), mit f), mit g), mit h), mit i), mit j), mit k), mit I), mit m), mit n), mit b) und h), mit c) und h), mit d) und h), mit e) und h), mit n) und h), mit b) und i), mit c) und i), mit d) und i), mit e) und i), mit n) und i), mit b) und j), mit c) und j), mit d) und j), mit e) und j), mit n) und j), mit b) und I), mit c) und I), mit d) und I), mit e) und I), mit n) und I), mit b) und m), mit c) und m), mit d) und m), mit e) und m), mit n) und m), mit b) und d), mit b), d) und h), mit b), d) und i), mit b), d) und j), mit b), d) und I), mit b), d) und m), mit b) und n), mit c) und n), mit d) und n), mit e) und n), mit f) und n), mit g) und n), mit h) und n), mit i) und n), mit j) und n), mit k) und n), mit I) und n), mit m) und n), mit b), d) und n), mit b), d), h) und n), mit b), d), i) und n) mit b), d), j) und n), mit b), d), I) und n) oder mit b), d), m) und n).

Eine weitere Möglichkeit der vorliegenden Anmeldung ist daher ein Polyurethansystem, welches durch eine Verwendung, wie zuvor beschrieben, erhältlich ist.

Diese Polyurethansysteme sind vorzugsweise Polyurethanschäume, bevorzugt Polyurethanhartschäume, Polyurethanweichschäume, viskoelastische Schäume, hochelastische Schäume sogenannte "High Resilience Schäume" (HR), halbharte Polyurethanschäume, thermoverformbare Polyurethanschäume oder Integralschäume. Die Bezeichnung Polyurethan ist hierbei wiederum als Oberbegriff für ein aus Di- bzw. Polyisocyanaten und Polyolen oder anderen gegenüber Isocyanat reaktive Spezies, wie z.B. Aminen, hergestelltes Polymer zu verstehen, wobei die Urethan-Bindung nicht ausschließlicher oder überwiegender Bindungstyp sein muss. Auch Polyisocyanurate und Polyharnstoffe sind ausdrücklich mit eingeschlossen.

Das Polyurethansystem, insbesondere Polyurethanschaum, zeichnet sich vorzugsweise dadurch aus dass es ein Polyurethanhartschaum, ein Polyurethanweichschaum, ein viskoelastischer Schaum, ein High Resilience (HR) Schaum, ein halbharter Polyurethanschaum, ein thermoverformbarer Polyurethanschaum oder ein Integralschaum ist, wobei es vorzugsweise einen Massenanteil an stickstoffhaltigen Verbindungen der Formel (VI), und/oder der entsprechenden quaternisierten und/oder protonierten Verbindungen bzw. der durch Umsetzung dieser erhaltenen Reste am fertigen Polyurethanschaum von 0,005 bis 10 Gew.-%, bevorzugt von 0,05 bis 3 Gew.-%, besonders bevorzugt 0,1 bis 1 Gew.-%, aufweist.

In einer bevorzugten Ausführungsform handelt es sich bei den erfindungsgemäß hergestellbaren Polyurethanschaumstoffen um offenzellige Polyurethan-Schaumstoffe, insbesondere Weichschaumstoffe, besonders bevorzugt Heißweichschaumstoffe. Offenzellig heißt im Rahmen der vorliegenden Erfindung, dass ein Schaum eine gute Luftdurchlässigkeit (= Porosität) aufweist. Die Luftdurchlässigkeit des Schaums kann durch eine Staudruckmessung am Schaumstoff ermittelt werden. Die Staudruckmessung kann in Anlehnung an EN 29053 erfolgen. Wird der gemessene Staudruck in mm Wassersäule angegeben, so weisen offenzellige Polyurethan-Schäume, insbesondere PolyurethanWeichschäume einen Staudruck von kleiner 100 mm, bevorzugt ≤ 50 mm Wassersäule, bestimmt gemäß der in den Beispielen beschriebenen Messmethode, auf.

Eine bevorzugte Zusammensetzung zur Herstellung von Polyurethan- bzw. Polyisocyanurat-Schaum im Sinne der vorliegenden Erfindung weist ein Raumgewicht (RG) von vorzugsweise 5 bis 800, insbesondere 5 bis 300, bevorzugt 5 bis 150, besonders bevorzugt von 10 bis 90 kg/m³ auf und hat insbesondere die folgende Zusammensetzung:

| Komponente | Gewichtsanteil |
|---|---|
| Polyol | 100 |
| (Amin-)Katalysator | 0,05 bis 5 |
| Zinn-Katalysator | 0 bis 5, vorzugsweise 0,001 bis 2 |
| Kalium-Trimerisierungskatalysator | 0 bis 10 |
| Siloxan | 0,1 bis 15, vorzugsweise 0,2 bis 7 |
| Wasser | 0 bis < 25, vorzugsweise 0,1 bis 15 |
| Treibmittel | 0 bis 130 |
| Flammschutzmittel | 0 bis 70 |
| Füllstoffe | 0 bis 150 |
| weitere Additive | 0 bis 20 |
| Isocyanat-Index: | größer 15 |

Möglich ist die Verwendung von Polyurethansystemen, insbesondere von Polyurethanschäumen, wie zuvor beschrieben, als Kühlschrankisolierung, Dämmplatte, Sandwichelement, Rohrisolation, Sprühschaum, 1- & 1,5-Komponenten-Dosenschaum, Holzimitat, Modellschaum, Blumensteckschaum, Verpackungsschaum, Matratze, Möbelpolster, Möbelformschaum, Kopfkissen, "Rebonded Foam", Schwammschaum, Automobil-Sitzpolster, Kopfstütze, Instrumententafel, Automobil-Innenverkleidung, Automobil-Dachhimmel, Schallabsorptionsmaterial, Lenkrad, Schuhsohle, Teppichrückseitenschaum, Filterschaum, Dichtschaum, Dichtmittel und Kleber oder zur Herstellung entsprechender Produkte.

Beispiele, alle nachfolgenden Beispiele sind nicht erfindungsgemäß Herstellung stickstoffhaltiger Verbindungen

Das für die Synthese der Verbindungen benötigte 1-(3-Aminopropyl)pyrrolidin (CAS 23159-07-1) wurde, wie nach dem Stand der Technik bekannt, über die Michael-Addition von Acrylnitril an Pyrrolidin und nachfolgender Hydrierung des entstanden Nitrils sowie abschließender Feindestillation hergestellt.

### Synthesebeispiel 1: Herstellung von 1-Pyrrolidinpropannitril

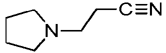

| Chemikalie | CAS | Lieferant |
|---|---|---|
| Acrylnitril, >99% | 107-13-1 | Sigma-Aldrich Chemie GmbH |
| Pyrrolidin, 99 % | 123-75-1 | ABCR |
| Dichlormethan | 75-09-2 | Sigma-Aldrich Chemie GmbH |
| Natriumhydroxid, ≥99 %, p.a. | 1310-73-2 | Karl Roth GmbH |
| Natriumchlorid, ≥99,8 %, extra fein | 7647-14-5 | Karl Roth GmbH |
| Methanol, ≥99.8% | 67-56-1 | Sigma-Aldrich Chemie GmbH |

In einem 6 L Fünfhalskolben ausgerüstet mit Tropftrichter, Thermometer, KPG-Rührwerk und Stickstoffzuleitung werden bei Raumtemperatur 2224 ml Wasser vorgelegt und das Reaktionsgefäß inertisiert. Es wurden dann 861,2 ml (737,18g, 10,37 mol) Pyrrolidin langsam hinzugegeben, wobei die Temperatur auf 35 °C anstieg. Die Reaktionsapparatur wurde erneut über den Tropftrichter inertisiert und dieser mit 625 ml (500 g, 9,42 mol) Acrylnitril befüllt. Mittels eines Kühlbades wurde die Amin-Lösung auf -5 °C abgekühlt und sodann mit der Zugabe des Acrylnitrils begonnen. Über 5 Stunden wurde die Zutropfgeschwindigkeit in der Form eingeregelt, dass die Reaktionstemperatur 5 °C nicht überstieg. Das Reaktionsgemisch wurde auf Raumtemperatur erwärmen gelassen und eine Stunde nachgerührt. Sodann wurde 2,1 L einer 33 Gew.-%igen wässrigen Natronlauge hinzugegeben, worauf sich eine milchartige Suspension bildete. Nach dem Abdekantieren in einen Scheidetrichter kam es zur Phasenseparation und die trübe, noch Wasser enthaltende, obere organische Phase wurde separiert. Zur besseren Phasentrennung wurden 200 ml Dichlormethan und 50 ml einer kaltgesättigten Kochsalzlösung hinzugegeben. Die obere, leicht opaque organische Phase wurde über Magnesiumsulfat getrocknet und das Lösungsmittel am Rotationsverdampfer eingeengt. Die folgende Feindestillation lieferte 1,25 kg eines klaren, leicht gelben Öls, dessen NMR spektroskopische Analyse in Einklang mit der Erwartung war. Eine GC-Messung bestätigte eine Reinheit von > 95 %.

### Synthesebeispiel 2: Herstellung von 1-(3-Aminopropyl)pyrrolidin

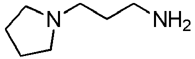

Die nachfolgende Hydrierung des zuvor hergestellten 1-Pyrrolidinpropannitrils erfolgte mittels Pd/Al₂O₃ (5 Gew.-%) wie von Krupka und Jiri et al. in - "Hydrogenation of 3-(dimethylamino)-propionitrile over palladium catalysts" (Czechoslovak Chemical Communications, 65 (11), 1805-1819; 2000) beschrieben. Das erhaltene krude Reaktionsgemisch wurde mit Celite^{®} Filterhilfe versetzt, abfiltriert und mit Methanol nachgewaschen. Das Lösungsmittel wurde eingeengt und das Rohprodukt einer Feindestillation unterzogen, wobei im Membranpumpenvakuum bei 24 mbar bei einer Kopftemperatur von 90 °C die Hauptfraktion überging. Ein alternatives elektrochemisches Verfahren ist in SU1421738 (A1) beschrieben.

### Synthesebeispiel 3: Herstellung einer Verbindung nach der (IIa)

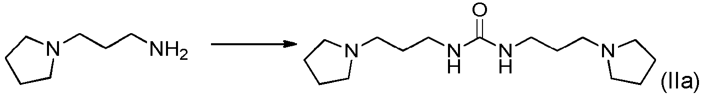

| Chemikalie | CAS | Lieferant |
|---|---|---|
| Harnstoff, p.a. | 57-13-6 | Sigma-Aldrich Chemie GmbH |
| 1-(3-Aminopropyl)pyrrolidin | 23159-07-1 | |

In einem Vierhalskolben, ausgestattet mit KPG-Rührer, Rückflusskühler, Temperaturmesssonde sowie Inertgaszuleitung wurden 19,82 g (0,33 mol) Harnstoff vorgelegt und mit 84,62 g (0,66 mol) 1-(3-Aminopropyl)pyrrolidin versehen. Nach einer Inertisierung der Reaktionsapparatur mittels Stickstoff wurde auf 130 °C erhitzt, wobei der Harnstoff in Lösung ging. Ein leichter Stickstoffstrom gewährleistete dabei die fortwährende Inertgasatmosphäre und das Fortschreiten der Reaktion konnte an einem kontinuierlichen Ammoniakabgang erkannt werden, der mittels Indikatorpapier am Gasauslass nachgewiesen werden konnte. Nach einer Gesamtreaktionszeit von 32 Stunden wurde an das bei 130 °C viskose, leicht gelbliche Produktgemisch ein Ölpumpenvakuum von > 1 mbar angelegt und so überschüssiges Edukt sowie sonstige flüchtige Bestandteile abdestilliert. Nach dem Abkühlen konnte das gewünschte Produkt nach der Formel (IIa) als ein weißes bis leicht beigefarbenes, kristallines Produkt gewonnen werden. Die 13C-NMR-Analysen entsprachen den Erwartungen und bestätigten die gewünschte Produktbildung.

### Synthesebeispiel 4: Herstellung einer Verbindung nach der Formel (IIb)

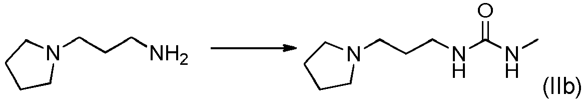

| Chemikalie | CAS | Lieferant |
|---|---|---|
| 1-Methylharnstoff | 598-50-5 | TCI Deutschland GmbH |
| 1-(3-Aminopropyl)pyrrolidin | 23159-07-1 | |

In einem 250 ml Vierhalskolben, ausgestattet mit KPG-Rührer, Rückflusskühler, Temperaturmesssonde sowie Inertgaszuleitung wurden 40,75 g (0,55 mol) 1-Methylharnstoff und 70,52 g (0,55 mol) des zuvor hergestellten 1-(3-Aminopropyl)pyrrolidins vorgelegt. Es wurde dann die Reaktionsapparatur mit Stickstoff inertisiert und auf eine Reaktionstemperatur von 110 °C aufgeheizt, wobei sich im Temperaturbereich um ∼ 90°C der Methylharnstoff schmolz und eine klare und farblose Lösung entstand. Es wurde ein kontinuierlicher Ammoniakabgang beobachtet, der mittels eines Indikatorpapiers am Gasauslass detektiert werden konnte. Nach einer Reaktionszeit von 17 Stunden wurde die Reaktionstemperatur auf 130 °C erhöht und diese für weitere 17 Stunden gehalten. Nachdem kein signifikanter Ammoniakabgang mehr detektiert werden konnte, wurde auf Raumtemperatur abkühlen gelassen. Es konnten so 101 g eines leicht gelben, hochviskosen Produktes nach der Formel (IIb) gewonnen werden, wobei die 13C-NMR-Analyse die Produktbildung bestätigte.

### Synthesebeispiel 5: Herstellung einer Verbindung nach der Formel (IIc)

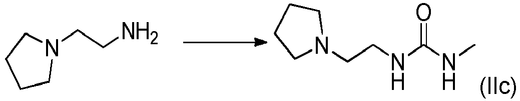

| Chemikalie | CAS | Lieferant |
|---|---|---|
| 1-Methylharnstoff | 598-50-5 | TCI Deutschland GmbH |
| 1-(2-Aminoethyl)pyrrolidin | 7154-73-6 | ABCR |

In einem 250 ml Vierhalskolben, ausgestattet mit KPG-Rührer, Rückflusskühler, Temperaturmesssonde sowie Inertgaszuleitung wurden 40,75 g (0,55 mol) 1-Methylharnstoff und 62,80 g (0,55 mol) 1-(2-Aminoethyl)pyrrolidin vorgelegt. Es wurde dann die Reaktionsapparatur mit Stickstoff inertisiert und auf eine Reaktionstemperatur von 100 °C aufgeheizt, wobei der Methylharnstoff schmolz und eine klare und farblose Lösung entstand. Es wurde ein kontinuierlicher Ammoniakabgang beobachtet, der mittels eines Indikatorpapiers am Gasauslass detektiert werden konnte. Nach einer Reaktionszeit von 32 Stunden konnte kein signifikanter Ammoniakabgang mehr detektiert werden und das Reaktionsgemisch wurde auf Raumtemperatur abkühlen gelassen. Es konnten so 92,2 g eines klaren, leicht bräunlichen und hochviskosen Produktes nach der Formel (IIc) gewonnen werden, wobei die 13C-NMR Analyse die Produktbildung bestätigte.

### Synthesebeispiel 6: Herstellung einer Verbindung nach der Formel (IIIa)

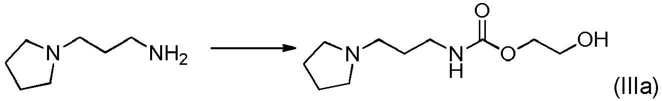

| Chemikalie | CAS | Lieferant |
|---|---|---|
| Ethylencarbonat, 98 % | 96-49-1 | Sigma-Aldrich Chemie GmbH |
| Aceton, 99,6% | 67-64-1 | Acros Organics |
| 1-(3-Aminopropyl)pyrrolidin | 23159-07-1 | |

In einem 250 ml Vierhalskolben, ausgestattet mit KPG-Rührer, Rückflusskühler, Temperaturmesssonde sowie Inertgaszuleitung wurden 51,29 g (0,4 mol) 1-(3-Aminopropyl)-pyrrolidin vorgelegt, die Reaktionsapparatur mit Stickstoff inertisiert und mit einem Kältebad auf 0 °C heruntergekühlt. In einem Tropftrichter wurden 35,22 g (0,4 mol) Ethylencarbonat gelöst in 35 ml VE-Wasser vorgelegt und sodann binnen 4 Stunden in der Form zugetropft, dass eine Reaktionstemperatur von 5 - 10 °C nicht überschritten wurde. Es wurde sodann der Reaktionsansatz auf Raumtemperatur erwärmen gelassen und für fünf weitere Stunden gerührt. Am Folgetag wurden ca. 100 ml Aceton dem Produkt hinzugegeben und das Reaktionsgemisch in einen Einhalsrundkolben überführt. Am Rotationsverdampfer wurden abschließend sukzessiv die Lösungsmittel sowie sämtliche flüchtigen Bestandteile bis zu einer finalen Badtemperatur von 60 °C und einem Ölpumpenvakuum von < 1 mbar entfernt. Es konnten so 84,8 g eines klaren, viskosen Produktes nach der Formel (IIIa) gewonnen werden. Die ¹³C-NMR-spektroskopische Analyse des Produktes war in Übereinstimmung mit den Erwartungen.

### Synthesebeispiel 7: Herstellung einer Verbindung nach Formel (IIIb)

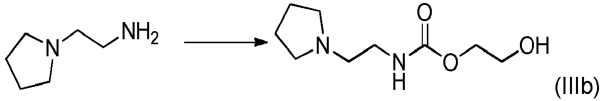

| Chemikalie | CAS | Lieferant |
|---|---|---|
| Ethylencarbonat, 98 % | 96-49-1 | Sigma-Aldrich Chemie GmbH |
| Aceton, 99,6% | 67-64-1 | Acros Organics |
| 1-(2-Aminoethyl)pyrrolidin | 7154-73-6 | ABCR |

In einem 250 ml Vierhalskolben, ausgestattet mit KPG-Rührer, Rückflusskühler, Temperaturmesssonde sowie Inertgaszuleitung wurden 45,08 g (0,4 mol) 1-(2-Aminoethyl)pyrrolidin vorgelegt, die Reaktionsapparatur mit Stickstoff inertisiert und mit einem Kältebad auf 0 °C heruntergekühlt. In einem Tropftrichter wurden 35,22 g (0,4 mol) Ethylencarbonat gelöst in 35 ml VE-Wasser vorgelegt und sodann binnen 4 Stunden in der Form zugetropft, dass eine Reaktionstemperatur von 5 - 10 °C nicht überschritten wurde. Es wurde sodann der Reaktionsansatz auf Raumtemperatur erwärmen gelassen und für fünf weitere Stunden gerührt. Am Folgetag wurden ca. 100 ml Aceton dem Produkt hinzugegeben und das Reaktionsgemisch in einen Einhalsrundkolben überführt. Am Rotationsverdampfer wurden abschließend sukzessiv die Lösungsmittel sowie sämtliche flüchtigen Bestandteile bis zu einer finalen Badtemperatur von 60 °C und einem Ölpumpenvakuums von < 1 mbar entfernt. Es konnten so 84,8 g eines klaren, viskosen Produktes nach der Formel (IIIb) gewonnen werden. Die ¹³C-NMR-spektroskopische Analyse des Produktes war in Übereinstimmung mit den Erwartungen.

### Synthesebeispiel 8: Herstellung einer Verbindung nach Formel (IIIc)

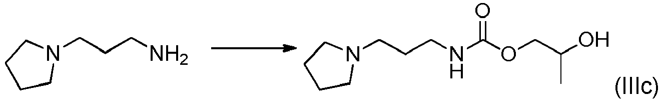

| Chemikalie | CAS | Lieferant |
|---|---|---|
| Propylencarbonat, 99 % | 108-32-7 | Sigma-Aldrich Chemie GmbH |
| 1-(3-Aminopropyl)pyrrolidin | 23159-07-1 | |

In einem 250 ml Vierhalskolben, ausgestattet mit KPG-Rührer, Rückflusskühler, Temperaturmesssonde sowie Inertgaszuleitung wurden 51,29 g (0,4 mol) 1-(3-Aminopropyl)-pyrrolidin vorgelegt, die Reaktionsapparatur mit Stickstoff inertisiert und mit einem Kältebad auf 0°C heruntergekühlt. Mittels eines Tropftrichters wurden 40,84 g (0,4 mol) Propylencarbonat binnen 4 Stunden in der Form zugetropft, dass eine Reaktionstemperatur von 5 - 10 °C nicht überschritten wurde. Es wurde sodann der Reaktionsansatz auf Raumtemperatur erwärmen gelassen und für fünf weitere Stunden gerührt. Am Folgetag wurden das Reaktionsgemisch in einen Einhalsrundkolben überführt und am Rotationsverdampfer abschließend sukzessiv sämtliche flüchtigen Bestandteile bis zu einer finalen Badtemperatur von 60 °C und einem Ölpumpenvakuum von < 1 mbar entfernt. Es konnten so 91 g eines klaren, viskosen Produktes nach Formel (IIIc) gewonnen werden. Die ¹³C-NMR-spektroskopische Analyse des Produktes war in Übereinstimmung mit den Erwartungen.

### Synthesebeispiel 9: Herstellung einer Verbindung nach Formel (IIId)

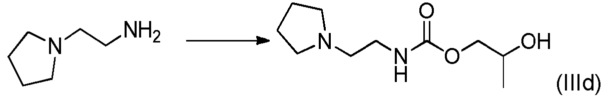

| Chemikalie | CAS | Lieferant |
|---|---|---|
| Propylencarbonat, 99 % | 108-32-7 | Sigma-Aldrich Chemie GmbH |
| 1-(2-Aminoethyl)pyrrolidin | 7154-73-6 | ABCR |

In einem 250 ml Vierhalskolben, ausgestattet mit KPG-Rührer, Rückflusskühler, Temperaturmesssonde sowie Inertgaszuleitung wurden 45,68 g (0,4 mol) 1-(2-Aminoethyl)pyrrolidin vorgelegt, die Reaktionsapparatur mit Stickstoff inertisiert und mit einem Kältebad auf 0 °C heruntergekühlt. Mittels eines Tropftrichters wurden 40,84 g (0,4 mol) Propylencarbonat binnen 4 Stunden in der Form zugetropft, dass eine Reaktionstemperatur von 5 - 10 °C nicht überschritten wurde. Es wurde sodann der Reaktionsansatz auf Raumtemperatur erwärmen gelassen und für fünf weitere Stunden gerührt. Am Folgetag wurden das Reaktionsgemisch in einen Einhalsrundkolben überführt und am Rotationsverdampfer abschließend sukzessiv sämtliche flüchtigen Bestandteile bis zu einer finalen Badtemperatur von 60°C und einem Ölpumpenvakuum von < 1 mbar entfernt. Es konnten so 85,6 g eines klaren, leicht gelblichen und viskosen Produktes nach der Formel (IIId) gewonnen werden. Die ¹³C-NMR-spektroskopische Analyse des Produktes war in Übereinstimmung mit den Erwartungen.

### Synthesebeispiel 10: Herstellung einer Verbindung nach Formel (IVa)

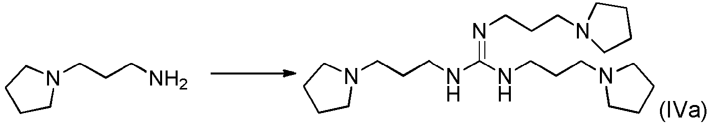

| Chemikalie | CAS | Lieferant |
|---|---|---|
| Guanidinhydrochlorid, >99% | 50-01-1 | Sigma-Aldrich Chemie GmbH |
| 1-(3-Aminopropyl)pyrrolidin | 23159-07-1 | |
| Natriummethylat | 124-41-4 | ABCR |
| Methanol | 67-56-1 | Sigma-Aldrich Chemie GmbH |

In einem 250 ml Vierhalskolben, ausgestattet mit Rückflusskühler, KPG-Rührer, Innenthermometer und Argoneinleitung wurden unter inerten Bedingungen 114,19 g (1 mol) 1-(3-Aminopropyl)pyrrolidin vorgelegt und mit 31,84 g (0,33 mol) Guanidinhydrochlorid versetzt. Die so hergestellte Reaktionsmischung wurde für auf 110°C aufgeheizt und mittels einer Membranpumpe ein leichtes Vakuum angelegt um den Ammoniakabgang zu erleichtern. Das Produkt verfärbte sich nach wenigen Minuten rot-braun. Nach einer Gesamtreaktionszeit von 35 Stunden wurde bei 95 - 100°C im Ölpumpenvakuum (< 1 mbar) sämtlichen flüchtigen Bestandteilen abgetrennt. Beim Abkühlen auf Raumtemperatur kristallisierte das rot-braune Produkt, zeigte jedoch eine gute Löslichkeit in laborüblichen Lösungsmitteln. Die 1H/13C-NMR Spektren bestätigten die Produktbildung und entsprach den Erwartungen.
Um die analoge freie Guanidinbase zu erhalten wurde ein Aliquot des obigen Produktes 40 Gew.-%ig in Methanol gelöst und mit einer stöchiometrischen Menge Natriummethylat versehen. Nach Rührung über Nacht bei Raumtemperatur zeigte sich eine Trübung und es wurde das Lösungsmittel am Rotationsverdampfer bei 80°C und einem Druck von 1 mbar entfernt, wobei es zum Ausfall von Natriumchlorid kam. Nach Filtration mittels einer Druckfilterpresse konnte die freie Guanidinbase nach der Formel (IVa) als ein braunes Öl erhalten werden.

### Synthesebeispiel 11: Herstellung einer Verbindung nach Formel (IVb)

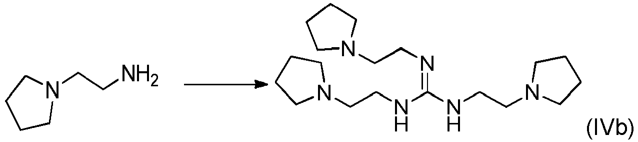

| Chemikalie | CAS | Lieferant |
|---|---|---|
| Guanidinhydrochlorid, >99% | 50-01-1 | Sigma-Aldrich Chemie GmbH |
| 1-(2-Aminoethyl)pyrrolidin | 7154-73-6 | ABCR |
| Natriummethylat | 124-41-4 | ABCR |
| Methanol | 67-56-1 | Sigma-Aldrich Chemie GmbH |

In einem 250 ml Vierhalskolben, ausgestattet mit Rückflusskühler, KPG-Rührer, Innenthermometer und Argoneinleitung wurden unter inerten Bedingungen 76,0 g (0,66 mol) 1-(2-Aminoethyl)pyrrolidin vorgelegt und mit 15,92 g (0,166 mol) Guanidinhydrochlorid versetzt. Die so hergestellte Reaktionsmischung wurde für auf 110 °C aufgeheizt und mittels einer Membranpumpe ein leichtes Vakuum angelegt um den Ammoniakabgang zu erleichtern. Das Produkt verfärbte sich nach wenigen Minuten rot-braun. Nach einer Gesamtreaktionszeit von 35 Stunden wurde bei 95 - 100 °C im Ölpumpenvakuum (< 1mbar) das überschüssige Äquivalent 1-(2-Aminoethyl)pyrrolidin sowie sämtlichen sonstigen flüchtigen Bestandteile abgetrennt. Beim Abkühlen auf Raumtemperatur kristallisierte das rot-braune Produkt, zeigte jedoch eine gute Löslichkeit in laborüblichen Lösungsmitteln. Die 1H/13C-NMR Spektren bestätigten die Produktbildung und entsprach den Erwartungen.
Um die analoge freie Guanidinbase zu erhalten wurde ein Aliquot des obigen Produktes 40 Gew.-%ig in Methanol gelöst und mit einer stöchiometrischen Menge Natriummethylat versehen. Nach Rührung über Nacht bei Raumtemperatur zeigte sich eine Trübung und es wurde das Lösungsmittel am Rotationsverdampfer bei 80 °C und einem Druck von 1 mbar entfernt, wobei es zum Ausfall von Natriumchlorid kam. Nach Filtration mittels einer Druckfilterpresse konnte die freie Guanidinbase nach der Formel (IVb) als ein braunes Öl erhalten werden.

### Synthesebeispiel 12: Herstellung einer Verbindung nach Formel (Va)

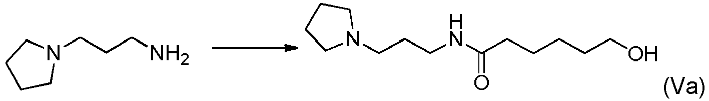

| Chemikalie | CAS | Lieferant |
|---|---|---|
| Caprolacton, 97% | 502-44-3 | Sigma-Aldrich Chemie GmbH |
| 1-(3-Aminopropyl)pyrrolidin | 23159-07-1 | |

In einem 1000 ml Vierhalskolben, ausgestattet mit Rückflusskühler, KPG-Rührer, Innenthermometer und Argoneinleitung werden unter inerten Bedingungen 256,43 g (2 mol) 1-(3-Aminopropyl)pyrrolidin und 228,28 g (2 mol) ε-Caprolacton vorgelegt. Die so hergestellte Reaktionsmischung wurde für vier Stunden auf 150 °C geheizt. Nach beendeter Reaktion wurde im Ölpumpenvakuum (5·10⁻² mbar) über eine kurze Zinke fraktioniert, wobei mit ~ 50 % Ausbeute eine Hauptfraktion von 241 g des Produktes gewonnen werden konnte. Die 1H/13C-NMR-Spektren der Hauptfraktion bestätigten die Produktbildung nach der Formel (Va) und entsprach den Erwartungen.

### Synthesebeispiel 13: Herstellung einer Verbindung nach der Formel (VI), am Beispiel der Verbindung nach Formel (VIa), erfindungsgemäß:

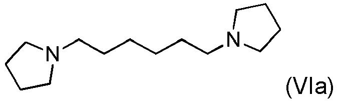

| Chemikalie | CAS | Lieferant |
|---|---|---|
| 1,6-Dichlorhexan, 98% | 2163-00-0 | Sigma-Aldrich Chemie GmbH |
| Triethylamin, 99% | 121-44-8 | ABCR |
| Toluol, 99,5%, ACS | 108-88-3 | Acros Organics |
| Natriumhydroxid, ≥99 %, p.a. | 1310-73-2 | Karl Roth GmbH |
| Diethylether, 99% | 60-29-7 | Sigma-Aldrich Chemie GmbH |

In einem 1 L Vierhalskolben, ausgestattet mit KPG-Rührer, Rückflusskühler, Temperaturmesssonde sowie Inertgaszuleitung wurden 242,9 g (2,4 mol) Triethylamin und 50 g Toluol vorgelegt. Es wurde dann die Reaktionsapparatur mit Stickstoff inertisiert und bei Raumtemperatur 170,7 g (2,4 mol) Pyrrolidin hinzugegeben woraufhin die Temperatur auf 35 °C stieg. Es wurde anschließend zum Rückfluss des Pyrrolidins bei 77 °C erhitzt und mittels eines Tropftrichters binnen 10 Minuten eine Menge von 93,4 g (0,6 mol) 1,6-Dichlorhexan hinzugegeben. Es wurde für weitere 16 Stunden bei 86 °C gerührt und anschließend eine Lösung von 48,2 g Natriumhydroxid in 250 ml Wasser hinzugegeben. Das nun zweiphasige Reaktionsgemisch wurde in einen Scheidetrichter überführt, die organische Phase abgetrennt und die wässrige Phase 2 x mit je 100 ml Diethylether extrahiert. Die vereinigten organischen Phasen wurden am Rotationsverdampfer bei 80 °C und 30 mbar eingeengt und das Rohprodukt einer fraktionierten Destillation unterzogen. Bei einer Kopftemperatur von 125 °C und 3 mbar Ölpumpenvakuum konnten 44,1 g einer klaren, farblosen Fraktion des Produktes nach der Formel (VIa) gewonnen werden, die nach GC-Analyse eine Reinheit von 97,3 % aufwies.

### Hartschaum - Verschäumungsbeispiele

### Beispiel 1: Herstellung von Polyurethanhartschaumstoffen, beispielsweise zur Anwendung bei der Isolation von Kühlmöbeln

Für die anwendungstechnische Ausprüfung der erfindungsgemäßen stickstoffhaltigen Verbindungen wurde die in Tabelle 1 angegebene Schaumformulierung verwendet.

**Tabelle 1: Formulierung 1 für Hartschaum-Anwendungen.**

| Formulierung 1 | Massenteile (pphp) |
|---|---|
| Polyol 1¹) | 100 Teile |
| Wasser | 2,60 Teile |
| Cyclopentan | 13,1 Teile |
| Amin | 0,80 oder 1,50 Teile (siehe Tabelle 2) |
| TEGOSTAB^{®} B 8460²⁾ | 1,50 Teile |
| Desmodur^{®} 44V20L³⁾ | 198,5 Teile |
| ¹⁾Polyol 1: Sorbitol/Glycerin-basiertes Polyetherpolyol mit einer OH-Zahl von 471 mgKOH/g. | |
| ²⁾Polyethermodifiziertes Polysiloxan. | |
| ³⁾polymeres MDI der Firma Bayer, 200 mPa·s, 31,5 % NCO, Funktionalität 2,7. | |

Die Durchführung der Verschäumungen erfolgte im Handmischverfahren. Es wurden die Formulierungen, wie in Tabelle 1 angegeben, mit verschiedenen stickstoffhaltigen Katalysatoren (Aminen) verwendet. Dazu wurden Polyol 1, konventioneller bzw. erfindungsgemäßer stickstoffhaltiger Katalysator (Amin), Wasser, Schaumstabilisator und Treibmittel in einen Becher eingewogen und mit einem Tellerrührer von 6 cm Durchmesser 30 Sekunden bei 1000 U/min vermischt. Durch erneutes Abwiegen wurde die beim Mischvorgang verdunstete Treibmittelmenge bestimmt und wieder ergänzt. Jetzt wurde das Isocyanat (MDI) zugegeben, die Reaktionsmischung wurde mit dem beschriebenen Rührer 5 s bei 3000 U/min verrührt und sofort in eine mit Papier ausgekleidete Kiste (27 cm x 14 cm Grundfläche und 14 cm Höhe) überführt. Zur Beurteilung der katalytischen Eigenschaften wurden die folgenden charakteristischen Parameter bestimmt: Creme-Zeit, Gel-Zeit (Fadenziehzeit), Steigzeit und Klebfrei-Zeit.

Die Ergebnisse der Beurteilung der katalytischen Eigenschaften der stickstoffhaltigen Verbindungen sind in Tabelle 2 (FORMEL (VIa) ist erfindungsgemäß) zusammengestellt. Als Vergleichskatalysatoren nach dem Stand der Technik wurden N,N-Dimethylcyclohexylamin (DMCHA), Dimethylaminoethoxyethanol (DMEE) und Pentamethyldiethylentriamin (PMDETA) eingesetzt.

**Tabelle 2: Ergebnisse der Verschäumungen nach Formulierung 1 (Tabelle 1).**

| Amin | Cremezeit [s]³⁾ | Gelzeit [s]³⁾ | Steigzeit [s]³⁾ | Klebfreizeit [s]³⁾ |
|---|---|---|---|---|
| DMCHA ¹⁾ | 38 | 137 | 298 | 310 |
| DMEE¹⁾ | 30 | 150 | 272 | 308 |
| PMDETA²⁾ | 15 | 128 | 199 | 224 |
| FORMEL (IIa)¹⁾ | 100 | 375 | 440 | 478 |
| FORMEL (IIb)¹⁾ | 55 | 225 | 315 | 408 |
| FORMEL (IIc)¹⁾ | 50 | 210 | 315 | 405 |
| FORMEL (IIIa)¹⁾ | 65 | 275 | 350 | 431 |
| FORMEL (IIIb)¹⁾ | 90 | 325 | 417 | 444 |
| FORMEL (IIIc)¹⁾ | 80 | 280 | 345 | 428 |
| FORMEL (IIId)¹⁾ | 85 | 315 | 405 | 445 |
| FORMEL (IVa)¹⁾ | 103 | 390 | 435 | 475 |
| FORMEL (IVb)¹⁾ | 105 | 397 | 438 | 460 |
| FORMEL (Va)¹⁾ | 79 | 277 | 347 | 431 |
| FORMEL (VIa)¹⁾⁴⁾ | 35 | 120 | 155 | 205 |

| | | | | |
|---|---|---|---|---|
| ¹⁾1,50 Teile Katalysator verwendet. ²⁾1,50 Teile Katalysator verwendet. ³⁾Zeit-Angaben in Sekunden [s]. ⁴⁾erfindungsgemäß | | | | |

Wie der Tabelle 2 entnommen werden kann, zeigen die stickstoffhaltigen Verbindungen nach der Formel (II), (III), (IV), (V) und erfindungsgemäß (VI) eine moderate bis sehr gute katalytische Aktivität und Selektivität im Hartschaum, teilweise vergleichbar mit DMCHA, teilweise sogar besser als DMEE. Im Falle der erfindungsgemäßen Verbindung nach Formel (VIa) handelt es sich sogar um einen sehr selektiven Treib-Katalysator, der hinsichtlich der Aktivität viel stärker als DMEE ist und ein ähnliches Selektivitätsprofil wie PMDETA aufweist.

### Weichschaum - Anwendungstechnische Tests

Physikalische Eigenschaften der Polyurethanweichschaumstoffe:
Die hergestellten Polyurethanweichschäume wurden anhand folgender physikalischer Eigenschaften beurteilt:
a) Rücksacken des Schaumstoffes nach dem Ende der Steigphase (=Rückfall): Der Rückfall, bzw. das Nachsteigen ergibt sich aus der Differenz der Schaumhöhe nach direktem Abblasen und nach 3 Minuten. nach Abblasen des Schaums. Die Schaumhöhe wird dabei durch eine an einem Zentimetermaß befestigte Nadel auf dem Maximum in der Mitte der Schaumkuppe gemessen. Ein negativer Wert beschreibt hierbei das Rücksacken des Schaumes nach dem Abblasen, ein positiver Wert beschreibt entsprechend das Nachsteigen des Schaumes.
b) Schaumhöhe: Die Endhöhe des Schaums wird dadurch bestimmt, dass der Rückfall bzw. das Nachsteigen von bzw. zu der Schaumhöhe nach dem Abblasen subtrahiert bzw. addiert wird. Die Schaumhöhe wird in Zentimeter (cm) angegeben.
c) Raumgewicht (RG): Die Bestimmung erfolgt, wie in ASTM D 3574 - 11 unter Test A beschrieben, durch Messung der Core Density. Das Raumgewicht wird in kg/m³ angegeben.
d) Porosität: Die Luftdurchlässigkeit des Schaums wurde durch eine Staudruckmessung am Schaumstoff ermittelt.
   Der gemessene Staudruck wird in mm Wassersäule angegeben, wobei niedrigere Staudruckwerte einen offeneren Schaum charakterisieren. Die Werte wurden im Bereich von 0 bis 300 mm gemessen.
   Die Messung des Staudrucks erfolgte mittels einer Apparatur umfassend eine Stickstoffquelle, Reduzierventil mit Manometer, Durchflussregelschraube, Waschflasche, Durchflussmessgerät, T-Stück, Auflagedüse und einem skaliertem Glasrohr, in welches Wasser gefüllt ist. Die Auflagedüse weist eine Kantenlänge von 100 x 100 mm, ein Gewicht von 800 g, eine lichte Weite der Austrittsöffnung von 5 mm, eine lichte Weite des unteren Auflageringes von 20 mm und einen Außendurchmesser des unteren Auflageringes von 30 mm auf.
   Die Messung erfolgt durch Einstellung des Stickstoffvordrucks per Reduzierventil auf 1 bar und Einregeln der Durchflussmenge auf 480 l/h. Die Wassermenge wird im skalierten Glasrohr so eingestellt, dass keine Druckdifferenz aufgebaut und ablesbar ist. Für die Vermessung des Prüfkörpers mit einer Dimension von 250 x 250 x 50 mm wird die Auflagedüse an den Ecken des Prüfkörpers kantenkongruent aufgelegt sowie einmal an der (geschätzten) Mitte des Prüfkörpers (jeweils auf der Seite mit der größten Oberfläche) aufgelegt. Abgelesen wird, wenn sich ein konstanter Staudruck eingestellt hat.
   Die Auswertung erfolgt durch Mittelwertbildung über die fünf erhaltenen Messwerte.
e) Stauchhärte CLD, 40 % nach DIN EN ISO 3386. Die Angabe der Messwerte erfolgt in Kilopascal (kPa).

Messung der Schaum-Emissionen (VOC- und Fog-Wert) in Anlehnung an die Prüfvorschrift VDA 278 in der Version vom Oktober 2011:
Das Verfahren dient zur Ermittlung von Emissionen aus nichtmetallischen Materialein, die für Formteile in Kraftfahrzeugen zum Einsatz kommen. Die Emission von leichtflüchtigen organischen Verbindungen (VOC-Wert, 30 Minuten bei 90 °C) sowie dem Anteil kondensierbarer Substanzen (Fog-Wert, 60 Minuten bei 120 °C), insbesondere der Katalyse-bedingten Emissionen, die Emissionen der einzelnen Bestandteile erfindungsgemäßer Katalysatorkombinationen oder ihrer Zer- oder Umsetzungsprodukte, wurde in Anlehnung an die Prüfvorschrift VDA 278 in der Version vom Oktober 2011 bestimmt. Im Folgenden wird die Durchführung der entsprechenden Thermodesorption mit anschließender Gaschromatographie/Massenspektrometrie-Kopplung (GC/MS) beschrieben.
a) Messtechnik: Die Thermodesorption wurde mit einem Thermodesorber "TDS2" mit Probenwechsler der Fa. Gerstel, Mülheim, in Verbindung mit einem Agilent 7890/5975 GC/MSD-System durchgeführt.
b) Die Messbedingungen für VOC-Messungen sind in Tabellen 3 und 4 angegeben.

**Tabelle 3: Messparameter Thermodesorption für den VOC-Analysenlauf.**

| Thermodesorption | Gerstel TDS2 |
|---|---|
| Desorptionstem peratu r | 90 °C |
| Desorptionsdauer | 30 min |
| Fluss | 65 ml/min |
| Transferline | 280°C |
| Kryofokussierung | KAS 4 |
| Liner | Glasverdampferrohr mit silanisierter Glaswolle |
| Temperatur | -150°C |

**Tabelle 4: Messparameter Gaschromatographie/Massenspektrometrie für den VOC-Analysenlauf.**

| | |
|---|---|
| GC | Kapillar-GC Agilent 7890 |
| Injektor | PTV Split 1:50 |
| Temperaturprogramm | -150°C; 1 min; 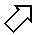 10°C/s; 280°C |
| Säule | Agilent 19091B-115, Ultra 2, 50 m * 0,32 mm dF 0,5µm |
| Fluss | 1,3 ml/min const. Flow |
| Temperaturprogramm | 50°C; 2 min; 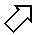 3°C/min; 92°C; 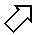 5°C/min; 160°C; 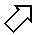 10°C/min; 280°C, 20 min |
| Detektor | Agilent MSD 5975 |
| Modus | Scan 29-350 amu 2,3 scans/sec |
| Auswertung | Auswertung des Totalionenstrom-Chromatrogramms durch Berechnung als Toluoläquivalent |

c) Kalibrierung: Zur Kalibrierung wurde 2 µl eines Gemisches aus Toluol und Hexadekan in Methanol (je 0,125 mg/ml) auf ein gereinigtes, mit Tenax^{®} TA (mesh 35/60) gefülltes Adsorptionsröhrchen gegeben und vermessen (Desorption 5 min; 280 °C).
d) Bei Tenax^{®} TA handelt es sich um ein poröses Polymerharz basierend auf 2.6-Diphenylenoxid, erhältlich beispielsweise bei der Firma Scientific Instrument Services, 1027 Old York Rd., Ringoes, NJ 08551.
e) Probenvorbereitung für die VOC-Messung: 15 mg Schaumstoff wurden in drei Teilproben in einem Thermodesorptionsröhrchen platziert. Dabei wurde darauf geachtet, dass der Schaum nicht komprimiert wird.
f) Probenvorbereitung für die Fog-Messung: Es wurde die gleiche Schaumprobe verwendet, wie für die VOC-Messung. Hinsichtlich der Messanordnung wurde immer zuerst die VOC-Messung und im Anschluss die Fog-Messung durchgeführt, wobei mittels eines Autosampler-Systems ein jeweils konstanter Abstand zwischen den entsprechenden VOC- und Fog-Messungen gewährleistet wurde.
g) Die Messbedingungen für Fog-Messungen sind in Tabelle 5 und 6 wiedergegeben.

**Tabelle 5: Messparameter Thermodesorption für den Fog-Analysenlauf.**

| Thermodesorption | Gerstel TDS2 |
|---|---|
| Desorptionstem peratu r | 120 °C |
| Desorptionsdauer | 60 min |
| Fluss | 65 ml/min |
| Transferline | 280°C |
| Kryofokussierung | KAS 4 |
| Liner | Glasverdampferrohr mit silanisierter Glaswolle |
| Temperatur | -150°C |

**Tabelle 6: Messparameter Gaschromatographie/Massenspektrometrie für den Fog-Analysenlauf.**

| | |
|---|---|
| GC | Kapillar-GC Agilent 7890 |
| Injektor | PTV Split 1:50 |
| Temperaturprogramm | -150°C; 1 min; 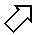 10°C/s; 280°C |
| Säule | Agilent 19091 B-115, Ultra 2, 50 m * 0,32 mm dF 0,5µm |
| Fluss | 1,3 ml/min const. Flow |
| Temperaturprogramm | 50°C; 2 min; 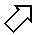 25°C/min; 160°C; 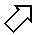 10°C/min; 280°C; 20 min |
| Detektor | Agilent MSD 5975 |
| Modus | Scan 29-450 amu 2,3 scans/sec |
| Auswertung | Auswertung des Totalionenstrom-Chromatrogramms durch Berechnung als Hexadekanäquivalent |

h) Kalibrierung: Zur Kalibrierung wurde 2 µl eines Gemisches aus Toluol und Hexadekan in Methanol (je 0,125 mg/ml) auf ein gereinigtes, mit Tenax^{®} TA (mesh 35/60) gefülltes Adsorptionsröhrchen gegeben und vermessen (Desorption 5 min; 280 °C).

Bestimmung der Raumtemperatur-Emission nach dem so genannten Prüfkammertest (PK): Von den erhaltenen Schäumen wurde die Emission, insbesondere die Katalyse-bedingten Emissionen, die Emissionen der einzelnen Bestanteile erfindungsgemäßer Katalysatorkombinationen oder ihrer Zer- oder Umsetzungsprodukte, bei Raumtemperatur in Anlehnung an die DIN-Vorschrift DIN EN ISO 16000-9:2008-04 bestimmt. Die Probenentnahme erfolgte nach 24 Stunden. Hierzu wurden 2 Liter der Prüfkammeratmosphäre mit einer Flussrate von 100 ml/min über ein mit Tenax^{®} TA (mesh35/60) gefülltes Adsorptionsröhrchen gegeben. Im Folgenden wird die Durchführung der Thermodesorption mit anschließender Gaschromatographie/Massenspektrometrie-Kopplung (GC/MS) beschrieben.
a) Messtechnik: Die Thermodesorption wurde mit einem Thermodesorber "TDS2" mit Probenwechsler der Fa. Gerstel, Mülheim, in Verbindung mit einem Agilent 7890/5975 GC/MSD-System durchgeführt.
b) Die Messbedingungen sind in Tabelle 7 und 8 angegeben.

**Tabelle 7: Messparameter Thermodesorption für PK-Messung.**

| Thermodesorption | Gerstel TDS2 |
|---|---|
| Desorptionstemperatur | 280°C |
| Desorptionsdauer | 5 min |
| Fluss | 65 ml/min |
| Transferline | 280°C |
| Kryofokussierung | KAS 4 |
| Liner | Glasverdampferrohr mit silanisierter Glaswolle |
| Temperatur | -150°C |

**Tabelle 8: Messparameter Gaschromatographie/Massenspektrometrie für PK-Messung.**

| | |
|---|---|
| GC | Kapillar-GC Agilent 7890 |
| Temperaturprogramm | -150°C; 1 min; 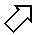 10°C/s; 280°C |
| Säule | Agilent 19091B-115, Ultra 2, 50 m * 0,32 mm dF 0,5µm |
| Fluss | 1,3 ml/min const. Flow |
| Temperaturprogramm | 50°C; 2 min; 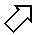 3°C/min; 92°C; 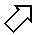 5°C/min; 160°C; 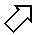 10°C/min; 280°C, 20 min |
| Detektor | Agilent MSD 5975 |
| Auswertung | Auswertung des Totalionenstrom-Chromatrogramms durch Berechnung als Toluoläquivalent |

c) Zur Kalibrierung wurde 2 µl eines Gemisches aus Toluol und Hexadekan in Methanol (je 0,125 mg/ml) auf ein gereinigtes, mit Tenax^{®} TA (mesh35/60) gefülltes Adsorptionsröhrchen gegeben und vermessen (Desorption 5 min; 280°C).

### Weichschaum - Verschäumungsbeispiele

### Beispiel 2: Herstellung von Polyurethanweichschaumstoffen (Weichblockschaum)

Für die anwendungstechnische Ausprüfung der erfindungsgemäßen stickstoffhaltigen Verbindungen wurden die in Tabelle 9 angegeben Schaumformulierung verwendet.

**Tabelle 9: Formulierung 2 für Weichblockschaum-Anwendungen.**

| Formulierung 2 | Massenteile (pphp) |
|---|---|
| Polyol 1¹⁾ | 100 Teile |
| Wasser | 3,00 Teile |
| Zinn-Katalysator²⁾ | 0,20 Teile |
| Amin | 0,20 Teile |
| TEGOSTAB^{®} BF 2370³⁾ | 0,80 Teile |
| Desmodur^{®} T 80⁴⁾ | 38,1 Teile |
| ¹⁾Polyol 1: Glycerin-basiertes Polyetherpolyol mit einer OH-Zahl von 48 mgKOH/g. | |
| ²⁾KOSMOS^{®} 29, erhältlich bei der Firma Evonik Industries: Zinn(II)-Salz der 2-Ethylhexansäure. | |
| ³⁾Polyethermodifiziertes Polysiloxan. | |
| ⁴⁾Toluylendiisocyanat T 80 (80 % 2,4-Isomer, 20 % 2,6-Isomer) der Firma Bayer, 3 mPa·s, 48 % NCO, Funktionalität 2. | |

Bei der Verschäumung wurden 500 g Polyol eingesetzt; die anderen Formulierungsbestandteile wurden entsprechend umgerechnet. Dabei bedeutete beispielsweise 1,00 Teil einer Komponente 1,00 g einer Substanz je 100 g Polyol.

Die Durchführung der Verschäumungen erfolgte im Handmischverfahren. Es wurden die Formulierungen, wie in Tabelle 9 angegeben, mit verschiedenen stickstoffhaltigen Katalysatoren (Aminen) verwendet. Dazu wurden Polyol, konventioneller bzw. erfindungsgemäßer stickstoffhaltiger Katalysator (Amin), Zinn-Katalysator, Wasser und Schaumstabilisator in einen Becher eingewogen und 60 Sekunden bei 1000 U/min vermischt. Nach Zugabe des Isocyanats (TDI) wurde die Reaktionsmischung 7 s bei 2500 U/min verrührt und sofort in eine mit Papier ausgekleidete Kiste (27 cm x 27 cm Grundfläche und 27 cm Höhe) überführt. Zur Beurteilung der katalytischen Eigenschaften wurden die folgenden charakteristischen Parameter bestimmt: Creme-Zeit, Steigzeit, Steighöhe, Stärke des Abblasens (=Blow Off) und Rücksacken des Schaumstoffes nach dem Ende der Steigphase (=Rückfall).

Aus den resultierenden Schaumblöcken wurden definierte Schaumkörper ausgeschnitten, welche weiter analysiert wurden. Folgende physikalischen Eigenschaften wurden an Hand der Probenkörper bestimmt: Raumgewicht (RG), Porosität (= Luftdurchlässigkeit) und Stauchhärte CLD (40%).

Die Ergebnisse der Beurteilung der katalytischen Eigenschaften der stickstoffhaltigen Verbindungen sowie der physikalischen Eigenschaften der resultieren Blockweichschaumstoffe sind in Tabelle 10 zusammengestellt. Als Vergleichskatalysatoren nach dem Stand der Technik wurden Triethylendiamin, 33% Gew.-ige Lösung in Dipropylenglykol (TEGOAMIN^{®} 33, erhältlich bei der Firma Evonik Industries), N,N-Dimethylethanolamin (TEGOAMIN^{®} DMEA, erhältlich bei der Firma Evonik Industries), 1,1'-{[3-(Dimethylamino)propyl]imino}bis-2-propanol (TEGOAMIN^{®} ZE 1, erhältlich bei der Firma Evonik Industries), Bis(2-Dimethyl-aminoethylether), 70 Gew.- %ige Lösung in Dipropylenglykol (TEGOAMIN^{®} BDE, erhältlich bei der Firma Evonik Industries) und N,N,N'-Trimethyl-N'-(2-hydroxyethyl)bis(2-aminoethyl)-ether (Jeffcat^{®} ZF-10, erhältlich bei der Firma Huntsman) eingesetzt. Es wurden jeweils 0,20 pphp (= Gewichtsteile bezogen auf 100 Gewichtsteile Polyol) Amin eingesetzt.

**Tabelle 10: Ergebnisse der Verschäumungen nach Formulierung 2 (Tabelle 9).**

| Amin | Steigzeit [s] | Rückfall [cm] | Höhe [cm] | RG [kg/m³] | Porosität [mm]¹⁾ | CLD 40 % [kPa] |
|---|---|---|---|---|---|---|
| TEGOAMIN^{®} 33 | 118 | 0,1 | 28,7 | 31,8 | 24 | 4,4 |
| TEGOAMIN^{®} ZE 1 | 143 | 0,2 | 28,2 | 31,9 | 24 | 4,3 |
| TEGOAMIN^{®} DMEA | 140 | 0,1 | 28,0 | 31,2 | 14 | 3,7 |
| TEGOAMIN^{®} BDE | 91 | 0,8 | 28,8 | 30,8 | 8 | 3,3 |
| Jeffcat^{®} ZF-10 | 108 | 0,7 | 28,9 | 30,7 | 9 | 3,4 |
| FORMEL (IIa) | 124 | 0,1 | 28,5 | 31,5 | 22 | 4,1 |
| FORMEL (IIb) | 127 | 0,2 | 28,6 | 31,3 | 23 | 4,0 |
| FORMEL (IIc) | 131 | 0,1 | 28,4 | 31,0 | 27 | 3,9 |
| FORMEL (IIIa) | 140 | 0,2 | 28,4 | 31,7 | 23 | 3,9 |
| FORMEL (IIIb) | 140 | 0,1 | 28,7 | 31,5 | 21 | 4,0 |
| FORMEL (IVa) | 138 | 0,2 | 28,5 | 31,2 | 28 | 4,3 |
| FORMEL (Va) | 143 | 0,1 | 27,6 | 31,3 | 26 | 3,9 |
| FORMEL (VIa) | 118 | 0,5 | 28,8 | 30,9 | 12 | 3,6 |

| | | | | | | |
|---|---|---|---|---|---|---|
| ¹⁾ = (Staudruck in mm Wassersäule). | | | | | | |

Wie der Tabelle 10 entnommen werden kann, zeigen die stickstoffhaltigen Verbindungen nach der Formel (II), (III), (IV), (V) und nach Formel (VIa) eine moderate bis gute katalytische Aktivität im Weichschaum. Hinsichtlich ihres katalytischen Profils und ihrer Selektivität sind alle untersuchten Verbindungen nach der Formel (II), (III), (IV) und (V) als leicht gel-selektive Katalysatoren einzustufen und liegen diesbezüglich in einem Bereich ähnlich dem TEGOAMIN ZE 1 oder dem TEGOAMIN^{®} DMEA. Die Verbindungen nach der Formel (IIa), (IIb), (IIc) und (VIa) liegen hinsichtlich ihres Steigprofils sogar im Bereich von TEGAOMIN^{®} 33. Insbesondere die Verbindung nach der Formel (VIa) besitzt eine ausgezeichnete katalytische Aktivität, der vergleichsweise große Rückfall und der etwas niedrigere CLD-Wert sind jedoch ein Indiz dafür, dass es sich bei Struktur (VIa) um einen Treib-selektiveren Katalysator handelt als TEGOAMIN^{®} 33.

### Beispiel 3: Emissionen von Polyurethanblockweichschaumstoffen

Um den Einfluss der erfindungsgemäßen stickstoffhaltigen Verbindungen auf die Schaum-emissionen zu untersuchen, wurden für die anwendungstechnische Ausprüfung von Blockweichschaumstoffen die in Tabelle 11 angegebene Schaumformulierung verwendet, die ein emissionsarmes Polyol und einen emissionsarmen Zinn-Katalysator enthält.

**Tabelle 11: Formulierung 3, Schaum-Emissionen in Weichblockschaum-Anwendungen.**

| Formulierung 3 | Massenteile (pphp) |
|---|---|
| Polyol 1¹⁾ | 100 Teile |
| Wasser | 3,00 Teile |
| Zinn-Katalysator²⁾ | 0,60 Teile |
| Amin | 0,15 Teile |
| TEGOSTAB^{®} BF 2370³⁾ | 0,80 Teile |
| Desmodur^{®} T 80⁴⁾ | 41,6 Teile |
| ¹⁾Polyol 1: Emissionsarmes Glycerin-basiertes Polyetherpolyol mit einer OH-Zahl von 56 mgKOH/g. | |
| ²⁾KOSMOS^{®} EF, erhältlich bei der Firma Evonik Industries: Zinn(II)-Salz der Rizinolsäure. | |
| ³⁾Polyethermodifiziertes Polysiloxan. | |
| ⁴⁾Toluylendiisocyanat T 80 (80 % 2,4-Isomer, 20 % 2,6-Isomer) der Firma Bayer, 3 mPa·s, 48 % NCO, Funktionalität 2. | |

Bei der Verschäumung wurden 500 g Polyol eingesetzt; die anderen Formulierungsbestandteile wurden entsprechend umgerechnet. Dabei bedeutete beispielsweise 1,00 Teil einer Komponente 1,00 g einer Substanz je 100 g Polyol.

Die Durchführung der Verschäumungen erfolgte im Handmischverfahren. Es wurden die in Formulierungen, wie in Tabelle 11 angegeben, mit verschiedenen stickstoffhaltigen Katalysatoren (Aminen) verwendet. Dazu wurden emissionsarmes Polyol, konventioneller bzw. erfindungsgemäßer stickstoffhaltiger Katalysator (Amin), emissionsarmer Zinn-Katalysator, Wasser und Schaumstabilisator in einen Becher eingewogen und 60 Sekunden bei 1000 U/min vermischt. Nach Zugabe des Isocyanats (TDI) wurde die Reaktionsmischung 7 s bei 2500 U/min verrührt und sofort in eine mit Papier ausgekleidete Kiste (27 cm x 27 cm Grundfläche und 27 cm Höhe) überführt und der resultierende Schaum nach dem Abblasen mit Polyethylen-Folie luftdicht verschlossen. Nach einer Aushärtungsphase von 24 Stunden wurde aus dem resultierenden Schaumblock ein definierter Schaumwürfel (7 cm x 7 cm x 7 cm) herausgeschnitten, welcher vollständig mit Aluminiumfolie umschlossen und weiterhin mit Polyethylen-Folie versiegelt wurde.

Das Emissionsverhalten der zuvor beschriebenen Schäume wurde im Anschluss bei Raumtemperatur nach dem Prüfkammertest in Anlehnung an die DIN-Vorschrift DIN EN ISO 16000-9:2008-04, wie oben beschrieben, untersucht. Die Ergebnisse sind in Tabelle 12 wiedergegeben.

**Tabelle 12: Emissionen der Blockweichschaumstoffe nach Formulierung 3 (Tabelle 11). Gehalt an flüchtigen organischen Verbindungen nach dem Prüfkammer-Test (PK)**

| Amin | PK_{ges}¹⁾ [µg/m³] | PK_{Amin}¹⁾ [µg/m³] |
|---|---|---|
| TEGOAMIN^{®} 33 | 92 | 64 |
| TEGOAMIN^{®} ZE 1 | < 20 | < 10 |
| TEGOAMIN^{®} DMEA | 28 | < 10 |
| TEGOAMIN^{®} BDE | 340 | 293 |
| Jeffcat^{®} ZF-10 | < 20 | < 10 |
| FORMEL (IIa) | < 20 | < 10 |
| FORMEL (IIb) | < 20 | < 10 |
| FORMEL (IIc) | < 20 | < 10 |
| FORMEL (IIIa) | < 20 | < 10 |
| FORMEL (IIIb) | < 20 | < 10 |
| FORMEL (IVa) | < 20 | < 10 |
| FORMEL (Va) | < 20 | < 10 |
| FORMEL (VIa) | < 20 | < 10 |

| | | |
|---|---|---|
| ¹⁾ PK_{ges} = Gesamtemission; PK_{Amin} = Amin-Emissionen aller flüchtigen organischen Verbindungen im Prüfkammertest. | | |

Tabelle 12 zeigt, dass die Amin-Emissionen nach dem Prüfkammertest bei Verwendung der stickstoffhaltigen Verbindungen nach der Formel (II), (III), (IV), (V) und (VIa) im Vergleich zu konventionellen Katalysatoren, wie TEGOAMIN^{®} 33, deutlich reduziert werden können, was eine Voraussetzung bei der Anwendung zur Herstellung von Blockweichschaumstoffen sein kann.

### Beispiel 4: Herstellung von HR-Schäumen (Block/Molded)

Für die anwendungstechnische Ausprüfung der erfindungsgemäßen stickstoffhaltigen Verbindungen wurde die in Tabelle 13 angegebene Schaumformulierung verwendet.

**Tabelle 13: Formulierung 4 für Kaltweichschaum-Anwendungen (HR-Block/Molded).**

| Formulierung 4 | Massenteile (pphp) |
|---|---|
| Polyol 1¹⁾ | 70,0 Teile |
| Polyol 2²⁾ | 30,0 Teile |
| Wasser | 3,70 Teile |
| Glycerin | 0,50 Teile |
| Diethanolamin (DEOA) | 1,00 Teile |
| Amin | 0,25 Teile |
| TEGOSTAB^{®} B 8716 LF2³⁾ | 1,00 Teile |
| Desmodur^{®} T 80⁴⁾ | 44,0 Teile |
| ¹⁾Polyol 1: Sorbitol/Glycerin-basiertes Polyetherpolyol, mit einer OH-Zahl von 32 mgKOH/g. | |
| ²⁾Polyol 2: Glycerin-basiertes Polyetherpolyol, enthaltend 43% Feststoff (SAN), mit einer OH-Zahl von 20 mgKOH/g. | |
| ³⁾Zubereitung von organomodifizierten Polysiloxanen. | |
| ⁴⁾Toluylendiisocyanat T 80 (80 % 2,4-Isomer, 20 % 2,6-Isomer) der Firma Bayer, 3 mPa·s, 48 % NCO, Funktionalität 2. | |

Hier wurden die gleichen Verschäumungsmethoden wie beim klassischen Polyurethanweichschaum in den Beispielen 2 und 3 angewendet.

Bei der Verschäumung wurden 500 g Polyol eingesetzt; die anderen Formulierungsbestandteile wurden entsprechend umgerechnet. Dabei bedeutete beispielsweise 1,00 Teil einer Komponente 1,00 g einer Substanz je 100 g Polyol.

Zur Verschäumung wurden Polyol, Wasser, Amin, und Silikonstabilisator unter Rühren gut vermischt. Nach Zugabe des Isocyanats wurde mit einem Rührer 4 s bei 3000 U/min. gerührt und das Gemisch in einem mit Papier ausgekleideten Holzkasten (27 cm × 27 cm Grundfläche und 27 cm Höhe) gegossen. Es entstand ein Schaumstoff, der den nachfolgend beschriebenen anwendungstechnischen Tests unterzogen wurde.

Die Ergebnisse der Beurteilung der katalytischen Eigenschaften der stickstoffhaltigen Verbindungen sowie der physikalischen Eigenschaften der resultieren Schaumstoffe sind in Tabelle 14 zusammengestellt. Als Vergleichskatalysatoren nach dem Stand der Technik wurden Triethylendiamin, 33% Gew.-ige Lösung in Dipropylenglykol (TEGOAMIN^{®} 33, erhältlich bei der Firma Evonik Industries), 1,1'-{[3-(Dimethylamino)propyl]imino}bis-2-propanol (TEGOAMIN^{®} ZE 1, erhältlich bei der Firma Evonik Industries), N,N-Dimethylethanolamin (TEGOAMIN^{®} DMEA, erhältlich bei der Firma Evonik Industries) eingesetzt. Es wurden jeweils 0,25 pphp (= Gewichtsteile bezogen auf 100 Gewichtsteile Polyol) Amin eingesetzt.

**Tabelle 14: Ergebnisse der Verschäumungen nach Formulierung 4 (Tabelle 13).**

| Amin | Gelzeit [s] | Steigzeit [s] | Höhe [cm] | Rückfall [cm] | Zellenzahl¹⁾ [cm⁻¹] |
|---|---|---|---|---|---|
| TEGOAMIN^{®} 33 | 85 | 151 | 31,2 | 0,4 | 10,5 |
| TEGOAMIN^{®} ZE 1 | 131 | 215 | 30,4 | -0,1 | 9,0 |
| TEGOAMIN^{®} DMEA | 148 | 265 | 26,6 | 0,0 | Kollaps |
| FORMEL (IIa) | 137 | 235 | 29,4 | 0,5 | 9,0 |
| FORMEL (IIIa) | 141 | 243 | 29,0 | 0,0 | 9,0 |
| FORMEL (IIIb) | 155 | 273 | 26,5 | 0,0 | 9,0 |
| FORMEL (IVa) | 153 | 263 | 26,8 | 0,1 | 9,0 |
| FORMEL (Va) | 151 | 275 | 27,1 | 0,1 | 9,0 |
| FORMEL (VIa) | 78 | 157 | 32,1 | 0,6 | 10,0 |

| | | | | | |
|---|---|---|---|---|---|
| ¹⁾ Zellenzahl = Anzahl der Zellen pro cm [cm⁻¹]. | | | | | |

Wie der Tabelle 14 entnommen werden kann, zeigen die stickstoffhaltigen Verbindungen nach der Formel (II), (III), (IV), (V) oder (VIa) in dieser Kaltschaum-Formulierung eine moderate katalytische Aktivität und Selektivität, teilweise vergleichbar mit TEGOAMIN^{®} ZE 1 und TEGOAMIN^{®} DMEA. Die Verbindung nach der Formel (VIa) hat wiederum eine hohe Aktivität im Bereich von TEGOAMIN 33, jedoch kann erneut eine leichte Selektivität hin zur Treib-Reaktion beobachtet werden.

## Patentansprüche

1. Verwendung zumindest einer stickstoffhaltigen Verbindung und/oder einer entsprechenden quaternisierten und/oder protonierten Verbindung, oder Mischungen der stickstoffhaltigen Verbindung mit entsprechenden quaternisierten und/oder protonierten Verbindungen bei der Herstellung von Polyurethanen, insbesondere von Polyurethanschaumstoffen, **dadurch gekennzeichnet, dass** diese stickstoffhaltige Verbindung 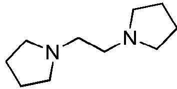 ist.

2. Verwendung nach Anspruch 1, **dadurch gekennzeichnet, dass** die stickstoffhaltige Verbindung 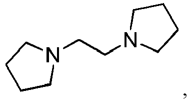 eine entsprechend quaternisierte und/oder protonierte Verbindung, oder Mischungen der stickstoffhaltigen Verbindung mit entsprechenden quaternisierten und/oder protonierten Verbindungen als Katalysator bei der Herstellung von Polyurethanen, insbesondere Polyurethanschaumstoffen eingesetzt wird.

3. Verwendung nach einem der vorigen Ansprüche, **dadurch gekennzeichnet, dass** bei der Herstellung des Polyurethans, insbesondere Polyurethanschaums, eine Zusammensetzung hergestellt wird, die zumindest eine stickstoffhaltige Verbindung 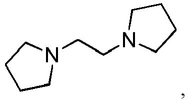 und/oder eine entsprechende quaternisierte und/oder protonierte Verbindung, zumindest eine Polyolkomponente, zumindest eine Isocyanatkomponente sowie optional ein oder mehrere Treibmittel aufweist, und diese Zusammensetzung reagiert wird.

4. Verwendung nach einem der vorigen Ansprüche, **dadurch gekennzeichnet, dass** die zumindest eine stickstoffhaltige Verbindung 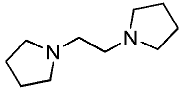 in Kombination mit zumindest einem Lösungsmittel eingesetzt wird.

5. Zusammensetzung enthaltend mindestens eine Polyolkomponente, **dadurch gekennzeichnet, dass** die Zusammensetzung zumindest eine stickstoffhaltige Verbindung wie in den vorigen Ansprüchen definiert, und/oder die entsprechenden quaternisierten und/oder protonierten Verbindungen aufweist, wobei die Zusammensetzung vorzugsweise mindestens eine Isocyanatkomponente aufweist.

6. Zusammensetzung nach Anspruch 5, **dadurch gekennzeichnet, dass** das molare Verhältnis der Gesamtmenge der stickstoffhaltigen Katalysatoren, umfassend die stickstoffhaltige Verbindung 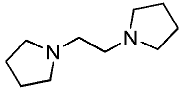 gegenüber der Gesamtmenge der mit Isocyanaten reaktiven Gruppen der Polyolkomponente von 4 x 10⁻⁴ zu 1 bis 0,2 zu 1 beträgt.

7. Zusammensetzungen nach Anspruch 5 oder 6, **dadurch gekennzeichnet, dass** die stickstoffhaltigen Verbindung 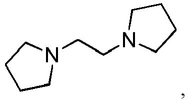 entsprechende quaternisierte und/oder protonierte Verbindungen, als Gesamtmenge in einem Massenanteil von 0,01 bis 20,0 Teilen (pphp), bevorzugt 0,01 bis 5,00 Teilen und besonders bevorzugt 0,02 bis 3,00 Teilen bezogen auf 100 Teile (pphp) Polyolkomponente eingesetzt werden.

## Claims

1. Use of at least one nitrogen compound and/or a corresponding quaternized and/or protonated compound, or mixtures of the nitrogen compound with corresponding quaternized and/or protonated compounds, in the production of polyurethanes, especially of polyurethane foams, **characterized in that** this nitrogen compound is

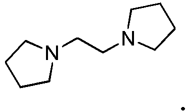

2. Use according to Claim 1, **characterized in that** the nitrogen compound 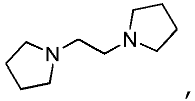 a correspondingly quaternized and/or protonated compound, or mixtures of the nitrogen compound with corresponding quaternized and/or protonated compounds, is used as catalyst in the production of polyurethanes, especially polyurethane foams.

3. Use according to either of the preceding claims, **characterized in that**, in the production of the polyurethane, especially polyurethane foam, a composition including at least one nitrogen compound 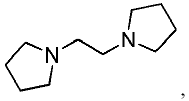 and/or a corresponding quaternized and/or protonated compound, at least one polyol component, at least one isocyanate component and optionally one or more blowing agents is produced, and this composition is reacted.

4. Use according to any of the preceding claims, **characterized in that** the at least one nitrogen compound 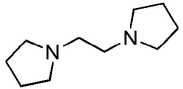 is used in combination with at least one solvent.

5. Composition comprising at least one polyol component, **characterized in that** the composition includes at least one nitrogen compound as defined in the preceding claims, and/or the corresponding quaternized and/or protonated compounds, wherein the composition preferably includes at least one isocyanate component.

6. Composition according to Claim 5, **characterized in that** the molar ratio of the total amount of the nitrogen-containing catalysts, comprising the nitrogen compounds 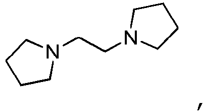 relative to the total amount of the groups reactive with isocyanates in the polyol component is from 4 × 10⁻⁴:1 to 0.2:1.

7. Compositions according to Claim 5 or 6, **characterized in that** the nitrogen compound 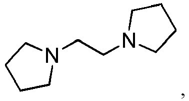 and corresponding quaternized and/or protonated compounds are used in an amount totalling a proportion by mass of 0.01 to 20.0 parts (pphp), preferably 0.01 to 5.00 parts and more preferably 0.02 to 3.00 parts, based on 100 parts (pphp) of polyol component.

## Revendications

1. Utilisation d'au moins un composé azoté et/ou d'un composé quaternisé et/ou protoné correspondant ou de mélanges du composé azoté avec des composés quaternisés et/ou protonés correspondants lors de la préparation de polyuréthanes, en particulier de mousses de polyuréthane, **caractérisée en ce que** ce composé azoté est

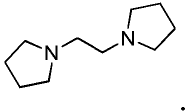

2. Utilisation selon la revendication 1, **caractérisée en ce que** le composé azoté 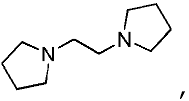 un composé quaternisé et/ou protoné correspondant ou des mélanges du composé azoté avec des composés quaternisés et/ou protonés correspondants est/sont utilisé(s) comme catalyseur lors de la préparation de polyuréthanes, en particulier de mousses de polyuréthane.

3. Utilisation selon l'une des revendications précédentes, **caractérisée en ce que** lors de la préparation du polyuréthane, en particulier de la mousse de polyuréthane, une composition est préparée qui présente au moins un composé azoté 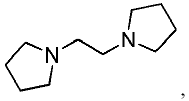 et/ou un composé quaternisé et/ou protoné correspondant, au moins un composant polyol, au moins un composé isocyanate ainsi qu'éventuellement un ou plusieurs agents gonflants, et cette composition est mise à réagir.

4. Utilisation selon l'une des revendications précédentes, **caractérisée en ce que** ledit au moins un composé azoté 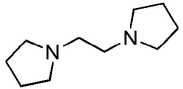 est utilisé en combinaison avec au moins un solvant.

5. Composition contenant au moins un composé polyol, **caractérisée en ce que** la composition présente au moins un composé azoté tel que défini dans les revendications précédentes et/ou les composés quaternisés et/ou protonés correspondants, la composition présentant de préférence au moins un composant isocyanate.

6. Composition selon la revendication 5, **caractérisée en ce que** le rapport molaire de la quantité totale des catalyseurs azotés, comprenant le composé azoté 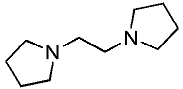 à la quantité totale des groupes réactifs vis-à-vis des isocyanates du composant polyol est de 4 × 10⁻⁴:1 à 0,2:1.

7. Compositions selon la revendication 5 ou 6, **caractérisées en ce que** le composé azoté 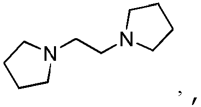 des composés quaternisés et/ou protonés correspondants, sont utilisés en tant quantité totale en une proportion massique de 0,01 à 20,0 parties (pphp), de préférence de 0,01 à 5,00 parties et de manière particulièrement préférée de 0,02 à 3,00 parties, par rapport à 100 parties (pphp) de composant polyol.
